# EUROPÄISCHE PATENTSCHRIFT

(11) **EP 1 942 198 B1**
(45) Veröffentlichungstag und Bekanntmachung des Hinweises auf die Patenterteilung: **02.11.2011**
(21) Anmeldenummer: 07123761.4
(22) Anmeldetag: 16.07.2005
(51) Int. Cl.: C12Q 1/68, C07K 14/34

(54) **PEF-TS EXPRESSIONSEINHEITEN BEI CORYNEBACTERIUM GLUTAMICUM**
P-ET-TS expression units in CORYNEBACTERIUM GLUTAMICUM
Unités d'expression P-ET-TS chez CORYNEBACTERIUM GLUTAMICUM

(30) Priorität: 20.07.2004 DE 102004035065
(43) Veröffentlichungstag der Anmeldung: 09.07.2008
(62) Teilanmeldung aus: 05759718.9
(73) Patentinhaber: BASF SE, 67056 Ludwigshafen (DE)
(72) Erfinder: Zelder, Oskar, 67346 Speyer (DE); Klopprogge, Corinna, 68239 Mannheim (DE); Kröger, Burkhard, 67117 Limburgerhof (DE); Schröder, Hartwig, 69226 Nußloch (DE); Haefner, Stefan, 67346 Speyer (DE)

(56) Entgegenhaltungen:
- EP-A- 1 108 790
- WO-A-01/00804
- WO-A-02/40679
- WO-A-2005/059093
- WO-A-2005/059144
- PARK SOO-DONG ET AL: "ISOLATION AND CHARACTERIZATION OF TRANSCRIPTIONAL ELEMENTS FROM CORYNEBACTERIUM GLUTAMICUM" JOURNAL OF MICROBIOLOGY AND BIOTECHNOLOGY, KOREAN SOCIETY FOR APPLIED MICROBIOLOGY, SEOUL, KO, Bd. 14, Nr. 4, August 2004 (2004-08), Seiten 789-795, XP009056160 ISSN: 1017-7825
- PÁTEK M ET AL: "Promoters of Corynebacterium glutamicum" JOURNAL OF BIOTECHNOLOGY, ELSEVIER SCIENCE PUBLISHERS B.V., AMSTERDAM, NL, Bd. 104, Nr. 1-3, 4. September 2003 (2003-09-04), Seiten 311-323, XP002323247 ISSN: 0168-1656
- PATEK M ET AL: "PROMOTERS FROM CORYNEBACTERIUM GLUTAMICUM: CLONING, MOLECULAR ANALYSIS AND SEARCH FOR A CONSENSUS MOTIF" MICROBIOLOGY, SOCIETY FOR GENERAL MICROBIOLOGY, READING, GB, Bd. 142, Nr. 5, 1996, Seiten 1297-1309, XP008006983 ISSN: 1350-0872
- DATABASE NCBI XP002488841 Database accession no. BX927154

## Beschreibung

Die vorliegende Erfindung betrifft ein Verfahren zur Veränderung oder Verursachung der Transkriptionsrate von Genen, und, genetisch veränderte Mikroorganismen mit veränderter oder verursachter Trankriptionsrate.

Verschiedene biosynthetische Produkte, wie beispielsweise Feinchemikalien, wie unter anderem Aminosäuren, Vitamine aber auch Proteine werden über natürliche Stoffwechselprozesse in Zellen hergestellt und werden in vielen Industriezweigen verwendet, einschließlich der Nahrungsmittel-, Futtermittel-, Kosmetik-, Feed-, Food- und pharmazeutischen Industrie. Diese Substanzen, die zusammen als Feinchemikalien/Proteine bezeichnet werden, umfassen unter anderem organische Säuren, sowohl proteinogene als auch nicht-proteinogene Aminosäuren, Nukleotide und Nukleoside, Lipide und Fettsäuren, Diole, Kohlenhydrate, aromatische Verbindungen, Vitamine und Cofaktoren, sowie Proteine und Enzyme. Ihre Produktion erfolgt am zweckmäßigsten im Großmaßstab mittels Anzucht von Bakterien, die entwickelt wurden, um große Mengen der jeweils gewünschten Substanz zu produzieren und sezernieren. Für diesen Zweck besonders geeignete Organismen sind coryneforme Bakterien, grampositive nicht-pathogene Bakterien.

Es ist bekannt, dass Aminosäuren durch Fermentation von Stämmen coryneformer Bakterien, insbesondere Corynebacterium glutamicum, hergestellt werden. Wegen der großen Bedeutung wird ständig an der Verbesserung der Herstellverfahren gearbeitet. Verfahrensverbesserungen können fermentationstechnische Maßnahmen, wie zum Beispiel Rührung und Versorgung mit Sauerstoff, oder die Zusammensetzung der Nährmedien, wie zum Beispiel die Zuckerkonzentration während der Fermentation, oder die Aufarbeitung zum Produkt , beispielsweise durch lonenaustauschchromatographie aber auch Sprühtrocknung, oder die intrinsischen Leistungseigenschaften des Mikroorganismus selbst betreffen.

Seit einigen Jahren werden ebenfalls Methoden der rekombinanten DNA-Technik zur Stammverbesserung von Feinchemikalien/Proteine produzierender Stämme von Corynebacterium eingesetzt, indem man einzelne Gene amplifiziert und die Auswirkung auf die Produktion von Feinchemikalien/Proteine untersucht.

Andere Wege, um ein Verfahren für die Herstellung Feinchemikalien, Aminosäuren oder Proteine zu entwickeln, oder die Produktivität eines bereits existierenden Verfahrens für die Herstellung Feinchemikalien, Aminosäuren oder Proteine zu erhöhen bzw. zu verbessern sind die Expression eines oder mehrerer Gene zu erhöhen bzw. zu verändern und oder die Translation einer mRNA durch geeignete Polynukleotidsequenzen zu beeinflussen. Beeinflussung kann in diesem Zusammenhang die Erhöhung, Verringerung, oder auch andere Parameter der Expression von Genen wie zeitliche Expressionsmuster umfassen.

Dem Fachmann sind unterschiedliche Bestandteile von bakteriellen Regulationssequenzen bekannt. Man unterscheidet die Bindungstellen von Regulatoren, auch Operatoren genannt, die Bindungstellen von RNA-Polymerase-Holoenzymen, auch -35 und - 10 Regionen genannt, und die Bindungsstelle von Ribosomaler 16S-RNA, auch Ribosomale Bindungsstelle oder auch Shine-Dalgarno-Sequenz genannt.

Als Sequenz einer Ribosomalen Bindungsstelle, auch Shine-Dalgarno-Sequenz genannt, im Sinne dieser Erfindung werden Polynukleotidsequenzen verstanden, die sich bis zu 20 Basen stromauf des Initiationskodon der Translation befinden.

In der Literatur (E. coli und S. typhimurium, Neidhardt F.C. 1995 ASM Press) wird beschrieben, dass sowohl die Zusammensetzung der Polynukletidsequenz der Shine-Delgarno-Sequenz, die Sequenzabfolge der Basen, aber auch der Abstand einer in der Shine-Delgarno-Sequenz enthaltenen Polynukletidsequenz zum einen wesentlichen Einfluss auf die Initiationstionsrate der Translation hat.

Nukleinsäuresequenzen mit Promotoraktivität können die Bildung von mRNA auf unterschiedliche Weise beeinflussen. Promotoren, deren Aktivität unabhängig von der physiologischen Wachstumsphase des Organismus sind, nennt man konstitutiv. Wiederum andere Promotoren reagieren auf externe chemische, wie physikalische Stimuli wie Sauerstoff, Metabolite, Hitze, pH, etc.. Wiederum andere zeigen in unterschiedlichen Wachstumsphasen eine starke Abhängigkeit ihrer Aktivität. Beispielsweise sind in der Literatur Promotoren beschrieben, die während der exponentiellen Wachstumsphase von Mikroorganismen eine besonders ausgeprägte Aktivität zeigen, oder aber auch genau in der stationären Phase des mikrobiellen Wachstums. Beide Charakteristika von Promotoren können für eine Produktion von Feinchemikalien und Proteine je nach Stoffwechselweg einen günstigen Einfluss auf die Produktivität haben.

Zum Beispiel kann man Promotoren die während des Wachstums die Expresssion eines Gens ausschalten, diese aber nach einem optimalen Wachstum anschalten dazu nutzen, ein Gen zu regulieren, das die Produktion eines Metaboliten kontrolliert. Der veränderte Stamm weist dann die gleichen Wachstumsparameter wie der Ausgangsstamm auf, produziert aber mehr Produkt pro Zelle. Diese Art der Modifizierung kann sowohl den Titer (g Produkt/Liter) als auch die C-Ausbeute (g ProduktJg-C-Quelle) erhöhen.

In Corynebacterium Spezies konnten bereits solche Nukleotidsequenzen isoliert werden, die für eine Erhöhung bzw. eine Abschwächung der Genexpression genutzt werden können. Diese regulierten Promotoren können die Rate, mit der ein Gen transkribiert wird, abhängig von den internen und/oder externen Bedingungen der Zelle erhöhen oder erniedrigen. Zum Teil kann die Anwesenheit eines bestimmten Faktors, bekannt als Inducer, die Rate der Transkrition vom Promotor stimulieren. Inducer können direkt oder aber indirekt die Transkription vom Promotor beeinflussen. Eine andere Klasse von Faktoren, bekannt als Suppressoren ist in der Lage, die Transkription vom Promotor zu reduzieren oder aber zu inhibieren. Wie auch die Inducer, können auch die Suppressoren direkt oder indirekt wirken. Es sind jedoch auch Promotoren bekannt, die über die Temperatur reguliert werden. So kann der Level der Transkription solcher Promotoren zum Beispiel durch eine Erhöhung der Wachstumstemperatur über die normale Wachstumstemperatur der Zelle erhöht oder aber abgeschwächt werden.

Eine geringe Anzahl von Promotoren aus C. glutamicum wurden bis zum heutigen Tag beschrieben. Der Promotor des Malatsynthase-Gens aus C. glutamicum wurde im DE 4440118 beschrieben. Dieser Promotor wurde einem für ein Protein kodierendes Strukturgen vorgeschaltet. Nach Transformation eines solchen Konstrukts in ein coryneformes Bakterium wird die Expression des dem Promotor nachgeschalteten Strukturgen reguliert. Die Expression des Strukturgens wird induziert sobald dem Medium ein ensprechender Induktor zugesetzt wird.

Reinscheid et al., Microbiology 145:503 (1999) haben eine trankriptionelle Fusion zwischen dem pta-ack Promotor aus C. glutamicum und einem Reportergen (Chloramphenicol Acetyltransferase) beschrieben. Zellen von C. glutamicum, die eine solche transkriptionelle Fusion enthalten, wiesen eine erhöhte Expression des Reportergenes bei Wachstum auf Acetat haltigem Medium auf. Im Vergleich dazu zeigten transformierte Zellen, die auf Glucose wuchsen, keine erhöhte Expression dieses Reportergens.

In Pa'tek et al., Microbiology 142:1297 (1996) wurden einige DNA Sequenzen aus C. glutamicum beschrieben, die die Expression eines Reportergens in C. glutamicum Zellen verstärken können, beschrieben. Diese Sequenzen wurden miteinander verglichen, um Consensus-Sequenzen für C. glutamicum Promotoren zu definieren.

Weitere DNA-Sequenzen aus C. glutamicum, die zur Regulation der Genexpression genutzt werden können, sind im Patent WO 02/40679 beschrieben worden. Diese isolierten Polynukleotide stellen Expressionseinheiten aus Corynebakterium glutamicum dar, die entweder zur Erhöhung oder aber zur Verringerung einer Genexpression genutzt werden können. Weiterhin sind in diesem Patent rekombinante Plasmide beschrieben, auf denen die Expressionseinheiten aus Corynebakterium glutamicum mit heterologen Genen assoziiert sind. Die hier beschrieben Methode , Fusion von einem Promotor aus Corynebakterium glutamicum mit einem heterlogen Gen, kann unter anderem zur Regulation der Gene der Aminosäurebiosynthese eingesetzt werden.

Der Erfindung lag die Aufgabe zugrunde weitere Verfahren zur Veränderung oder Verursachung der Transkriptionsrate von Genen im Mikroorganismus im Vergleich zum Wildtyp zur Verfügung zustellen.

Demgemäß wurde ein Verfahren zur Veränderung oder Verursachung der Transkriptionsrate von Genen in Mikroorganismen im Vergleich zum Wildtyp durch Regulation der Transkription von Genen im Mikroorganismus durch Nukleinsäuren mit Promotoraktivität, enthaltend
A) die Nukleinsäuresequenz SEQ.ID.NO. 1 oder
B) eine von dieser Sequenz durch Substitution, Insertion oder Deletion von Nukleotiden abgeleitete Sequenz, die eine Identität von mindestens 90 % auf Nukleinsäureebene mit der Sequenz SEQ.ID.NO. 1 aufweist oder
C) eine Nukleinsäuresequenz, die mit der Nukleinsäuresequenz SEQ.ID.NO. 1 unter stringenten Bedingungen hybridisiert,
wobei die Gene in Bezug auf die Nukleinsäuren mit Promotoraktivität heterolog sind, gefunden.

Unter "Transkription" wird erfindungsgemäß der Prozess verstanden, durch den ausgehend von einer DNA-Matrize ein komplementäres RNA-Molekül hergestellt wird. An diesem Prozess sind Proteine wie die RNA-Polymerase sogenannte Sigma-Faktoren und transkriptionelle Regulatorproteine beteiligt. Die synthetisierte RNA dient dann als Matrize im Prozess der Translation, der dann zum biosynthetisch aktiven Protein führt.

Die Bildungsrate, mit der ein biosynthetsich aktives Protein hergestellt wird, ist ein Produkt aus der Rate der Transkription und der Translation. Beide Raten können erfindungsgemäß beeinflusst werden und damit die Rate der Bildung von Produkten in einem Mikroorganismus beeinflussen.

Unter einem "Promotor" oder einer "Nukleinsäure mit Promotoraktivität" wird erfindungsgemäß eine Nukleinsäure verstanden, die in funktioneller Verknüpfung mit einer zu trankripierenden Nukleinsäure, die Transkription dieser Nukleinsäure reguliert.

Unter einer "funktionellen Verknüpfung" versteht man in diesem Zusammenhang beispielsweise die sequentielle Anordnung einer der Nukleinsäuren mit Promotoraktivität gemäß Anspruch 1 und einer zu transkribierenden Nukleinsäuresequenz und ggf. weiterer regulativer Elemente wie zum Beispiel Nukleinsäureseuqenzen, die die Transkription von Nukleinsäuren gewährleisten, sowie zum Beipsiel einen Terminator derart, dass jedes der regulativen Elemente seine Funktion bei der Transkription der der Nukleinsäuresequenz erfüllen kann. Dazu ist nicht unbedingt eine direkte Verknüpfung im chemischen Sinne erforderlich. Genetische Kontrollsequenzen, wie zum Beispiel Enhancer-Sequenzen, können ihre Funktion auch von weiter entfernten Positionen oder gar von anderen DNA-Molekülen aus auf die Zielsequenz ausüben. Bevorzugt sind Anordnungen, in denen die zu trankribierende Nukleinsäuresequenz hinter (d.h. am 3'-Ende) der erfindungsgemäßen Promotorsequenz positioniert wird, so dass beide Sequenzen kovalent miteinander verbunden sind. Bevorzugt ist dabei der Abstand zwischen der Promotorsequenz und der transgen zu exprimierende Nukleinsäuresequenz geringer als 200 Basenpaare, besonders bevorzugt kleiner als 100 Basenpaare, ganz besonders bevorzugt kleiner als 50 Basenpaare.

Unter "Promotoraktivität" wird erfindungsgemäß die in einer bestimmten Zeit durch den Promotor gebildete Menge RNA, also die Trankriptionsrate verstanden.

Unter "spezifischer Promotoraktivität" wird erfindungsgemäß die in einer bestimmten Zeit durch den Promotor gebildete Menge RNA pro Promotor verstanden.

Unter dem Begriff "Wildtyp" wird erfindungsgemäß der entsprechende Ausgangsmikroorganismus verstanden.

Je nach Zusammenhang kann unter dem Begriff "Mikroorganismus" der Ausgangsmikroorganismus (Wildtyp) oder ein erfindungsgemäßer, genetisch veränderter Mikroorganismus oder beides verstanden werden.

Vorzugsweise und insbesondere in Fällen, in denen der Mikroorganismus oder der Wildtyp nicht eindeutig zugeordnet werden kann, wird unter "Wildtyp" für die Veränderung oder Verursachung der Promotoraktivität oder Trankriptionsrate, für die Veränderung oder Verursachung der Expressionsaktivität oder Expressionsrate und für die Erhöhung des Gehalts an biosynthetischen Produkten jeweils ein Referenzorganismus verstanden.

In einer bevorzugten Ausführungsform ist dieser Referenzorganismus Corynebakterium glutamicum ATCC 13032.

In einer bevorzugten Ausführungsform werden Ausgangsmikroorganismen verwendet die bereits in der Lage sind, die gewünschte Feinchemikalie herzustellen. Besonders bevorzugt sind dabei unter den besonders bevorzugten Mikroorganismen der Bakterien der Gattung Corynebacterien und den besonders bevorzugten Feinchemikalien L-Lysin, L-Methionin und L-Threonin, diejenigen Ausgangsmikroorganismen die bereits in der Lage sind, L-Lysin, L-Methionin und/oder L-Threonin herzustellen. Dies sind besonderes bevorzugt Corynebakterien bei denen beispielsweise das Gen kodierend für eine Aspartokinase (ask-Gen) dereguliert ist oder die feed-back-Inhibierung aufgehoben oder reduziert ist. Beispielsweise weisen solche Bakterien im ask-Gen eine Mutation auf, die zu einer Reduzierung oder Aufhebung der feed-back-Inhibierung führen, wie beispielsweise die Mutation T3111.

Bei einer "verursachten Promotoraktivität" oder Transkriptionsrate im Bezug auf ein Gen im Vergleich zum Wildtyp wird somit im Vergleich zum Wildtyp die Bildung einer RNA verursacht, die im Wildtyp so nicht vorhanden war.

Bei einer veränderten Promotoraktivität oder Transkriptionsrate im Bezug auf ein Gen im Vergleich zum Wildtyp wird somit im Vergleich zum Wildtyp in einer bestimmten Zeit die gebildete Menge der RNA verändert.

Unter "verändert" wird in diesem Zusammenhang bevorzugt erhöht oder erniedrigt verstanden.

Dies kann beispielsweise durch die erhöhte Promotoraktivität oder Transkriptionsrate beispielsweise durch Regulation der Transkription von Genen im Mikroorganismus durch Nukleinsäuren mit Promotoraktivität gemäß Anspruch 1 erreicht werden, wobei die Gene in Bezug auf die Nukleinsäuren mit Promotoraktivität heterolog sind.

Vorzusgweise wird die Regulation der Transkription von Genen im Mikroorganismus durch Nukleinsäuren mit Promotoraktivität gemäß Anspruch 1 dadurch erreicht, dass man eine oder mehrere Nukleinsäuren mit Promotoraktivität gemäß Anspruch 1, gegebenenfalls mit veränderter spezifischer Promotoraktivität, in das Genom des Mikroorganismus einbringt, so dass die Transkription eines oder mehrerer endogenen Gene unter der Kontrolle der eingebrachten Nukleinsäure mit Promotoraktivität gemäß Anspruch 1 gebenenfalls mit veränderter spezifischer Promotoraktivität, erfolgt oder
ein oder mehrere Gene in das Genom des Mikrorganismus einbringt, so dass die Transkription eines oder mehrerer der eingebrachten Gene unter der Kontrolle der endogenen Nukleinsäuren mit Promotoraktivität gemäß Anspruch 1, gegebenenfalls mit veränderter spezifischer Promotoraktivität, erfolgt oder
ein oder mehrere Nukleinsäurekonstrukte, enthaltend eine Nukleinsäure mit Promotoraktivität gemäß Anspruch 1, gegebenenfalls mit veränderter spezifischer Promotoraktivität, und funktionell verknüpft eine oder mehrere, zu transkribierende Nukleinsäuren, in den Mikroorganismus einbringt. Die Nukleinsäuresequenz SEQ. ID. NO. 1 stellt die Promotorsequenz des Elongations-Faktors TS (P_{EF-TS}) aus Corynebakterium glutamicum dar. SEQ. ID.NO. 1 entspricht der Promotorsequenz des Wildtyps.

Weitere natürliche Beispiele für Promotoren gemäß Anspruch 1 lassen sich beispielsweise aus verschiedenen Organismen, deren genomische Sequenz bekannt ist, durch ldentitätsvergleiche der Nukleinsäuresequenzen aus Datenbanken mit der vorstehend beschriebenen Sequenzen SEQ ID NO: 1 leicht auffinden.

Künstliche Promotor-Sequenzen gemäß Anspruch 1 lassen sich ausgehend von der Sequenz SEQ ID NO: 1 durch künstliche Variation und Mutation, beispielsweise durch Substitution, Insertion oder Deletion von Nukleotiden leicht auffinden.

Unter dem Begriff "Substitution" ist in der Beschreibung der Austausch einer oder mehrerer Nukleotide durch ein oder mehrere Nukleotide zu verstehen. "Deletion" ist das Ersetzen eines Nukleotides durch eine direkte Bindung. Insertionen sind Einfügungen von Nukleotiden in die Nukleinsäuresequenz, wobei formal eine direkte Bindung durch ein oder mehrere Nukleotide ersetzt wird.

Unter Identität zwischen zwei Nukleinsäuren wird die Identität der Nukleotide über die jeweils gesamte Nukleinsäurelänge verstanden, insbesondere die Identität die durch Vergleich mit Hilfe der Vector NTI Suite 7.1 Software der Firma Informax (USA) unter Anwendung der Clustal Methode (Higgins DG, Sharp PM. Fast and sensitive multiple sequence alignments on a microcomputer. Comput Appl. Biosci. 1989 Apr;5(2):151-1) unter Einstellung folgender Parameter berechnet wird:
Multiple alignment parameter:
   Gap opening penalty 10
   Gap extension penalty 10
   Gap separation penalty range 8
   Gap separation penalty off
   % identity for alignment delay 40
   Residue specific gaps off
   Hydrophilic residue gap off
   Transition weighing 0
Pairwise alignment parameter:
   FAST algorithm on
   K-tuplesize 1
   Gap penalty 3
   Window size 5
   Number of best diagonals 5

Unter einer Nukleinsäuresequenz, die eine Identität von mindestens 90 % mit der Sequenz SEQ ID NO: 1 aufweist, wird dementsprechend eine Nukleinsäuresequenz verstanden, die bei einem Vergleich seiner Sequenz mit der Sequenz SEQ ID NO: 1, insbesondere nach obigen Programmlogarithmus mit obigem Parametersatz eine Identität von mindestens 90 % aufweist.

Besonders bevorzugte Promotoren weisen mit der Nukleinsäuresequenz SEQ. ID. NO. 1 eine Identität von 91%, bevorzugter 92%, 93%, 94%, 95%, 96%, 97%, 98%, besonders bevorzugt 99% auf.

Weitere natürliche Beispiele für Promotoren lassen sich weiterhin ausgehend von den vorstehend beschriebenen Nukleinsäuresequenzen, insbesondere ausgehend von der Sequenz SEQ ID NO: 1 aus verschiedenen Organismen, deren genomische Sequenz nicht bekannt ist, durch Hybridisierungstechniken in an sich bekannter Weise leicht auffinden.

Nukleinsäuren mit Promotoraktivität, enthaltend eine Nukleinsäuresequenz, die mit der Nukleinsäuresequenz SEQ. ID. No. 1 unter stringenten Bedingungen hybridisieren umfassen mindestens 10, bevorzugter mehr als12,15,30,50 oder besonders bevorzugt mehr als 150 Nukleotide.

Die Hybridisierung erfolgt erfinungsgemäß unter stringenten Bedingungen. Solche Hybridisierungsbedingungen sind beispielsweise bei Sambrook, J., Fritsch, E.F., Maniatis, T., in: Molecular Cloning (A Laboratory Manual), 2. Auflage, Cold Spring Harbor Laboratory Press, 1989, Seiten 9.31-9.57 oder in Current Protocols in Molecular Biology, John Wiley & Sons, N.Y. (1989), 6.3.1-6.3.6 beschrieben:

Unter stringenten Hybridisierungs-Bedingungen werden insbesondere verstanden: Die über Nacht Inkubation bei 42°C in einer Lösung bestehend aus 50 % Formamid, 5 x SSC (750 mM NaCl, 75 mM Tri-Natrium Citrat), 50 mM Natrium Phosphat (ph7,6), 5x Denhardt Lösung, 10% Dextransulfat und 20 g/ml denaturierte, gescheerte Lachsspermien-DNA, gefolgt von einem Waschen der Filter mit 0,1x SSC bei 65°C.

Die SEQ. ID. NO. 1 ist ohne Funktionszuordnung im Genbank-Eintrag AP005283 beschrieben worden.

Alle vorstehend erwähnten Nukleinsäuren mit Promotoraktivität sind weiterhin in an sich bekannter Weise durch chemische Synthese aus den Nukleotidbausteinen wie beispielsweise durch Fragmentkondensation einzelner überlappender, komplementärer Nukleinsäurebausteine der Doppelhelix herstellbar. Die chemische Synthese von Oligonukleotiden kann beispielsweise, in bekannter Weise, nach der Phosphoamiditmethode (Voet, Voet, 2. Auflage, Wiley Press New York, S. 896-897) erfolgen. Die Anlagerung synthetischer Oligonukleotide und Auffüllen von Lücken mithilfe des Klenow-Fragmentes der DNA-Polymerase und Ligationsreaktionen sowie allgemeine Klonierungsverfahren werden in Sambrook et al. (1989), Molecular cloning: A laboratory manual, Cold Spring Harbor Laboratory Press, beschrieben.

Unter einer Expressioneinheit wird eine Nukleinsäure mit Expressionsaktivität verstanden, also eine Nukleinsäure verstanden, die in funktioneller Verknüpfung mit einer zu exprimierenden Nukleinsäure oder Gens, die Expression, also die Transkription und die Translation dieser Nukleinsäure oder dieses Gens reguliert.

Unter einer "funktionellen Verknüpfung" versteht man in diesem Zusammenhang beispielsweise die sequentielle Anordnung einer Expressionseinheit und einer transgen zu exprimierenden Nukleinsäuresequenz und ggf. weiterer regulativer Elemente wie zum Beispiel einem Terminator derart, dass jedes der regulativen Elemente seine Funktion bei der transgenen Expression der Nukleinsäuresequenz erfüllen kann. Dazu ist nicht unbedingt eine direkte Verknüpfung im chemischen Sinne erforderlich. Genetische Kontrollsequenzen, wie zum Beispiel Enhancer-Sequenzen, können ihre Funktion auch von weiter entfernten Positionen oder gar von anderen DNA-Molekülen aus auf die Zielsequenz ausüben. Bevorzugt sind Anordnungen, in denen die transgen zu exprimierende Nukleinsäuresequenz hinter (d.h. am 3'-Ende) der Expressionseinheitssequenz positioniert wird, so dass beide Sequenzen kovalent miteinander verbunden sind. Bevorzugt ist dabei der Abstand zwischen der Expressionseinheitssequenz und der transgen zu exprimierende Nukleinsäuresequenz geringer als 200 Basenpaare, besonders bevorzugt kleiner als 100 Basenpaare, ganz besonders bevorzugt kleiner als 50 Basenpaare.

Unter "Expressionsaktivität" wird erfindungsgemäß die in einer bestimmten Zeit durch die Expressionseinheit gebildete Menge Protein, also die Expressionsrate verstanden.

Unter "spezifischer Expressionsaktivität" wird erfindungsgemäß die in einer bestimmten Zeit durch die Expressionseinheit gebildete Menge Protein pro Expressionseinheit verstanden.

Bei einer "verursachten Expressionsaktivität" oder Expressionsrate im Bezug auf ein Gen im Vergleich zum Wildtyp wird somit im Vergleich zum Wildtyp die Bildung eines Proteins verursacht, das im Wildtyp so nicht vorhanden war.

Bei einer "veränderten Expressionsaktivität" oder Expressionsrate im Bezug auf ein Gen im Vergleich zum Wildtyp wird somit im Vergleich zum Wildtyp in einer bestimmten Zeit die gebildete Menge des Proteins verändert.

Unter "verändert" wird in diesem Zusammenhang bevorzugt erhöht oder erniedrigt verstanden.

Dies kann beispielsweise durch Erhöhung oder Reduzierung der spezifischen Aktivität der endogenen Expressionseinheit, beispielsweise durch Mutation der Expressionseinheit oder durch Stimmulierung oder Hemmung der Expressionseinheit erfolgen. Weiterhin kann die erhöhte Expressionsaktivität oder Expressionsrate beispielsweise durch Regulation der Expression von Genen im Mikroorganismus durch Expressionseinheiten oder durch Expressionseinheiten mit Erhöhter spezifischer Expressionsaktivität erreicht werden, wobei die Gene im Bezug auf die Expressionseinheiten heterolog sind.

Vorzugsweise wird die Regulation der Expression von Genen im Mikroorganismus durch Expressionseinheiten oder durch Expressionseinheiten mit Erhöhter spezifischer Expressionsaktivitätdadurch erreicht, dass man
eine oder mehrere erfindungsgemäße Expressionseinheiten, gegebenenfalls mit veränderter spezifischer Expressionsaktivität, in das Genom des Mikroorganismus einbringt, so dass die Expression eines oder mehrerer endogenen Gene unter der Kontrolle der eingebrachten Expressionseinheiten, gegebenenfalls mit veränderter spezifischer Expressionsaktivität, erfolgt oder
ein oder mehrere Gene in das Genom des Mikrorganismus einbringt, so dass die Expression eines oder mehrerer der eingebrachten Gene unter der Kontrolle der endogenen, Expressionseinheiten, gegebenenfalls mit veränderter spezifischer Expressionsaktivität, erfolgt oder
ein oder mehrere Nukleinsäurekonstrukte, enthaltend eine Expressionseinheit, gegebenenfalls mit veränderter spezifischer Expressionsaktivität, und funktionell verknüpft eine oder mehrere, zu exprimierende Nukleinsäuren, in den Mikroorganismus einbringt.

Die Expressionseinheiten, enthalten eine vorstehend bechriebene Nukleinsäure mit Promotoraktivität gemäß Anspruch 1 und zusätzlich funktionell verknüpft eine Nukleinsäuresequenz, die die Translation von Ribonukleinsäuren gewährleistet.

Vorzugsweise enthält diese Nukleinsäuresequenz, die die Translation von Ribonukleinsäuren gewährleistet, die Nukleinsäuresequenz SEQ. ID. NO. 42 als ribosomale Bindungsstelle.

Die Expressionseinheit enthält:
E) die Nukleinsäuresequenz SEQ. ID. NO. 2 oder
F) eine von dieser Sequenz durch Substitution, Insertion oder Deletion von Nukleotiden abgeleitete Sequenz, die eine Identität von mindestens 90 % auf Nukleinsäureebene mit der Sequenz SEQ. ID. NO. 2 aufweist oder
G) eine Nukleinsäuresequenz, die mit der Nukleinsäuresequenz SEQ. ID. NO. 2 unter stringenten Bedingungen hybridisiert.

Die Nukleinsäuresequenz SEO. ID. NO. 2 stellt die Nukleinsäuresequenz der Expressionseinheit des Elongations-Faktors TS (P_{EF-TS}) aus Corynebakterium glutamicum dar. SEQ. ID.NO. 2 entspricht der Sequenz der Expressionseinheit des Wildtyps.

Weitere natürliche Beispiele für Expressionseinheiten lassen sich beispielsweise aus verschiedenen Organismen, deren genomische Sequenz bekannt ist, durch ldentitätsvergleiche der Nukleinsäuresequenzen aus Datenbanken mit der vorstehend beschriebenen Sequenzen SEQ ID NO: 2 leicht auffinden.

Künstliche Sequenzen der Expressionseinheiten lassen sich ausgehend von der Sequenz SEQ ID NO: 2 durch künstliche Variation und Mutation, beispielsweise durch Substitution, Insertion oder Deletion von Nukleotiden leicht auffinden.

Unter einer Nukleinsäuresequenz, die eine Identität von mindestens 90 % mit der Sequenz SEQ ID NO: 2 aufweist, wird dementsprechend eine Nukleinsäuresequenz verstanden, die bei einem Vergleich seiner Sequenz mit der Sequenz SEQ ID NO: 2, insbesondere nach obigen Programmlogarithmus mit obigem Parametersatz eine Identität von mindestens 90 % aufweist.

Weitere natürliche Beispiele für Expressionseinheiten lassen sich weiterhin ausgehend von den vorstehend beschriebenen Nukleinsäuresequenzen, insbesondere ausgehend von der Sequenz SEQ ID NO: 2 aus verschiedenen Organismen, deren genomische Sequenz nicht bekannt ist, durch Hybridisierungstechniken in an sich bekannter Weise leicht auffinden.

Unter "hybridisieren" versteht man die Fähigkeit eines Poly- oder Oligonukleotids unter stringenten Bedingungen an eine nahezu komplementäre Sequenz zu binden, während unter diesen Bedingungen unspezifische Bindungen zwischen nicht-komplementären Partnern unterbleiben. Dazu sollten die Sequenzen vorzugsweise zu 90-100%, komplementär sein. Die Eigenschaft komplementärer Sequenzen, spezifisch aneinander binden zu können, macht man sich beispielsweise in der Northem- oder Southem-Blot-Technik oder bei der Primerbindung in PCR oder RT-PCR zunutze.

Die Hybridisierung erfolgt unter stringenten Bedingungen. Solche Hybridisierungsbedingungen sind beispielsweise bei Sambrook, J., Fritsch, E.F., Maniatis, T., in: Molecular Cloning (A Laboratory Manual), 2. Auflage, Cold Spring Harbor Laboratory Press, 1989, Seiten 9.31-9.57 oder in Current Protocols in Molecular Biology, John Wiley & Sons, N.Y. (1989), 6.3.1-6.3.6 beschrieben:

Unter stringenten Hybridisierungs-Bedingungen werden insbesondere verstanden:

Die über Nacht Inkubation bei 42°C in einer Lösung bestehend aus 50 % Formamid, 5 x SSC (750 mM NaC1, 75 mM Tri-Natrium Citrat), 50 mM Natrium Phosphat (ph7,6), 5x Denhardt Lösung, 10% Dextransulfat und 20 g/ml denaturierte, gescheerte Lachsspermien-DNA, gefolgt von einem Waschen der Filter mit 0,1x SSC bei 65°C.

Die Nukleotidsequenzen gemäß Anspruch 1 ermöglichen ferner die Erzeugung von Sonden und Primern, die zur Identifizierung und/oder Klonierung von homologer Sequenzen in anderen Zelltypen und Mikroorganismen verwendbar sind. Solche Sonden bzw. Primer umfassen gewöhnlich einen Nukleotidsequenzbereich, der unter stringenten Bedingungen an mindestens etwa 12, vorzugsweise mindestens etwa 25, wie z.B. etwa 40, 50 oder 75 aufeinanderfolgende Nukleotide eines Sense-Stranges einer Nukleinsäuresequenz gemäß Anspruch 1 oder eines entsprechenden Antisense-Stranges hybridisiert.

Umfasst sind auch solche Nukleinsäuresequenzen, die sogenannte stumme Mutationen umfassen oder entsprechend der Codon-Nutzung eins speziellen Ursprungs- oder Wirtsorganismus, im Vergleich zu einer konkret genannten Sequenz verändert sind, ebenso wie natürlich vorkommende Varianten, wie z.B. Spleißvarianten oder Allelvarianten, davon.

Die Nukleinsäuresequenz SEQ. ID. NO. 2 kann als Expressionseinheit, d.h. zur Expression von Genen verwendet werden.

Die Expressionseinheit enthält eine Nukleinsäure mit Promotoraktivität gemäß Anspruch 1 zusätzlich funktionell verknüpft eine Nukleinsäuresequenz, die die Translation von Ribonukleinsäuren gewährleistet. Die Expressionseinheiten umfassen ein oder mehrere der folgenden genetischen Elemente: eine Minus 10 ("-10") Sequenz; eine Minus 35 ("-35") Sequenz; einen Transkriptionsstart, eine Enhancer Region; und eine Operator Region.

Vorzugsweise sind diese genetischen Elemente spezifisch für die Spezies Corynebakterien, speziell für Corynbacterium glutamicum.

Alle vorstehend erwähnten Expressionseinheiten sind weiterhin in an sich bekannter Weise durch chemische Synthese aus den Nukleotidbausteinen wie beispielsweise durch Fragmentkondensation einzelner überlappender, komplementärer Nukleinsäurebausteine der Doppelhelix herstellbar. Die chemische Synthese von Oligonukleotiden kann beispielsweise, in bekannter Weise, nach der Phosphoamiditmethode (Voet, Voet, 2. Auflage, Wiley Press New York, S. 896-897) erfolgen. Die Anlagerung synthetischer Oligonukleotide und Auffüllen von Lücken mithilfe des Klenow-Fragmentes der DNA-Polymerase und Ligationsreaktionen sowie allgemeine Klonierungsverfahren werden in Sambrook et al. (1989), Molecular cloning: A laboratory manual, Cold Spring Harbor Laboratory Press, beschrieben.

Für die Erfindungen in diesem Patent wurden Methoden und Techniken genutzt, die dem Fachmann, der in mikrobiologischen und rekombinanten DNA-Techniken geübt ist, bekannt sind. Methoden und Techniken für das Wachstum von Bakterienzellen, das Einschleusen von isolierten DNA-Molekülen in die Wirtszelle, und die Isolierung, Klonierung und Sequenzierung von isolierten Nukleinsäuremolekülen usw. sind Beispiele für solche Techniken und Methoden. Diese Methoden sind in vielen Standardliteraturstellen beschrieben: Davis et al., Basic Methods In Molecular Biology (1986); J. H. Miller, Experiments in Molecular Genetics, Cold Spring Harbor Laboratory Press, Cold Spring Harbor, New York (1972); J.H. Miller, A Short Course in Bacterial Genetics, Cold Spring Harbor Laboratory Press, Cold Spring Harbor, New York (1992); M. Singer and P. Berg, Genes & Genomes, University Science Books, Mill Valley, Carüfomia (1991); J. Sambrook, E.F. Fritsch and T. Maniatis, Molecular Cloning: A Laboratory Manual, 2nd ed., Cold Spring Harbor Laboratory Press, Cold Spring Harbor, New York (1989); P.B. Kaufmann et al., Handbook of Molcular and Cellular Methods in Biology and Medicine, CRC Press, Boca Raton, Florida (1995); Methods in Plant Molecular Biology and Biotechnology, B.R. Glick and J.E. Thompson, eds., CRC Press, Boca Raton, Florida (1993); and P.F. Smith-Keary, Molecular Genetics of Escherichia coli, The Guilford Press, New York, NY (1989).

Alle Nukleinsäuremoleküle der vorliegenden Erfindung liegen bevorzugt in Form eines isolierten Nukleinsäuremoleküls vor. Ein "isoliertes" Nukleinsäuremolekül wird von anderen Nukleinsäuremolekülen abgetrennt, die in der natürlichen Quelle der Nukleinsäure zugegen sind und kann überdies im wesentlichen frei von anderem zellulären Material oder Kulturmedium sein, wenn es durch rekombinante Techniken hergestellt wird, oder frei von chemischen Vorstufen oder anderen Chemikalien sein, wenn es chemisch synthetisiert wird.

Die Erfindung umfasst weiterhin die zu den konkret beschriebenen Nukleotidsequenzen komplementären Nukleinsäuremoleküle oder einen Abschnitt davon.

Die Promotoren und/oder Expressionseinheiten lassen sich beispielsweise besonders vorteilhaft in verbesserten Verfahren zur fermentativen Herstellung von biosynthetischen Produkten wie nachstehend beschrieben verwenden.

Die Promotoren und/oder Expressionseinheiten weisen insbesondere den Vorteil auf, dass sie in Mikroorganismen durch Stress induziert werden. Durch geeignete Steuerung des Fermentationsprozesses lässt sich diese Stress-Induktion gezieit für eine Erhöhung der Trankritptions/Expressionsrate gewünschter Gene steuern. Insbesondere bei der Produktion von L-Lysin wird diese Stressphase sehr früh erreicht, so das hier sehr früh eine erhöhte Transkriptions/Expressionsrate von gewünschten Genen erreicht werden kann.

Die Nukleinsäuren mit Promotoraktivität gemäß Anspruch 1 können zur Veränderung, also zur Erhöhung oder Reduzierung, oder zur Verursachung der Transkriptionsrate von Genen in Mikroorganismen im Vergleich zum Wildtyp verwendet werden.

Die Expressionseinheiten können zur Veränderung, also zur Erhöhung oder Reduzierung, oder zur Verursachung der Expressionsrate von Genen in Mikroorganismen im Vergleich zum Wildtyp verwendet werden.

Ferner können die Nukleinsäuren mit Promotoraktivität gemäß Anspruch 1 und die Expressionseinheiten zur Regulation und Verstärkung der Bildung von verschiedenen biosynthetischen Produkten, wie beispielsweise Feinchemikalien, Proteinen, inbesondere Aminosäuren, in Mikroorganismen, insbsondere in Corynebacterium species dienen.

Die Veränderung oder Verursachung der Transkriptionsrate von Genen in Mikroorganismen im Vergleich zum Wildtyp rfolgt dadurch, dass man die Transkription von Genen im Mikroorganismus durch Nukleinsäuren mit Promotoraktivität
A) die Nukleinsäuresequenz SEQ.ID.NO. 1 oder
B) eine von dieser Sequenz durch Substitution, Insertion oder Deletion von Nukleotiden abgeleitete Sequenz, die eine Identität von mindestens 90 % auf Nukleinsäureebene mit der Sequenz SEQ.ID.NO. 1 aufweist oder
C) eine Nukleinsäuresequenz, die mit der Nukleinsäuresequenz SEQ.ID.NO. 1 unter stringenten Bedingungen hybridisiert, reguliert.
, wobei die Gene in Bezug auf die Nukleinsäuren mit Promotoraktivität heterolog sind.

Dies wird bevorzugt dadurch erreicht, dass man
b1) eine oder mehrere Nukleinsäuren mit Promotoraktivität gemäß Anspruch 1, gebenenfalls mit veränderter spezifischer Promotoraktivität, in das Genom des Mikroorganismus einbringt, so dass die Transkription eines oder mehrerer endogenen Gene unter der Kontrolle der eingebrachten Nukleinsäure mit Promotoraktivität, erfolgt oder
b2) ein oder mehrere Gene in das Genom des Mikrorganismus einbringt, so dass die Transkription eines oder mehrerer der eingebrachten Gene unter der Kontrolle der endogenen, Nukleinsäuren mit Promotoraktivität gemäß Anspruch 1, gegebenenfalls mit veränderter spezifischer Promotoraktivität, erfolgt oder
b3) ein oder mehrere Nukleinsäurekonstrukte, enthaltend eine Nukleinsäure mit Promotoraktivität gemäß Anspruch 1, gegebenenfalls mit veränderter spezifischer Promotoraktivität, und funktionell verknüpft eine oder mehrere, zu transkribierende Nukleinsäuren, in den Mikroorganismus einbringt.

Es ist somit möglich, die Transkriptionsrate eines endogenen Gens des Wildtyps zu verändern, also zu erhöhen oder zu erniedrigen indem man
gemäß Ausführungsform b1) eine oder mehrere Nukleinsäuren mit Promotoraktivität, gebenenfalls mit veränderter spezifischer Promotoraktivität, in das Genom des Mikroorganismus einbringt, so dass die Transkription eines oder mehrerer endogenen Gene unter der Kontrolle der eingebrachten Nukleinsäure mit Promotoraktivität, erfolgt oder

gemäß Ausführungsform b2) ein oder mehrere endogene Gene in das Genom des Mikrorganismus einbringt, so dass die Transkription eines oder mehrerer der eingebrachten, endogenen Gene unter der Kontrolle der endogenen, Nukleinsäuren mit Promotoraktivität, gegebenenfalls mit veränderter spezifischer Promotoraktivität, erfolgt oder

Gemäß Ausführungsform b3) ein oder mehrere Nukleinsäurekonstrukte, enthaltend eine Nukleinsäure mit Promotoraktivität, gegebenenfalls mit veränderter spezifischer Promotoraktivität, und funktionell verknüpft eine oder mehrere, zu transkribierende endogene Nukleinsäuren, in den Mikroorganismus einbringt.

Ferner ist es somit möglich, die Transkriptionsrate eines exogenen Gens im Vergleich zum Wildtyps zu verursachen, indem man

gemäß Ausführungsform b2) ein oder mehrere exogene Gene in das Genom des Mikrorganismus einbringt, so dass die Transkription eines oder mehrerer der eingebrachten, exogenen Gene unter der Kontrolle der endogenen, Nukleinsäuren mit Promotoraktivität, gegebenenfalls mit veränderter spezifischer Promotoraktivität, erfolgt oder

Gemäß Ausführungsform b3) ein oder mehrere Nukleinsäurekonstrukte, enthaltend eine Nukleinsäure mit Promotoraktivität, gegebenenfalls mit veränderter spezifischer Promotoraktivität, und funktionell verknüpft eine oder mehrere, zu transkribierende exogene Nukleinsäuren, in den Mikroorganismus einbringt.

Die Insertion von Genen gemäß Ausführungsform b2) kann dabei so erfolgen, dass das Gen in kodierende Bereiche oder nicht-kodierende Bereiche integriert wird. Vorzugsweise erfolgt die Insertion in nicht-kodierende Bereiche.

Die Insertion von Nukleinsäurekonstrukten gemäß Ausführungsform b3) kann dabei chromosomal oder extrachromosomal erfolgen. Vorzugsweise erfolgt die Insertion der Nukleinsäurekonstrukte chromosomal. Eine "chromosomate" Integration ist die Insertion eines exogenen DNA-Fragmentes in das Chromosom einer Wirtszelle. Dieser Begriff wird auch für die homologe Rekombination zwischen einem exogenen DNA-Fragment und der entsprechenden Region auf dem Chromosom der Wirtszelle genutzt.

Unter "endogen" werden genetische Informationen, wie beispielsweise Gene, verstanden, die bereits im Wildtypgenom enthalten sind.

Unter "exogen" werden genetische Informationen, wie beispielsweise Gene, verstanden, die im Wildtypgenom nicht enthalten sind.

Unter dem Begriff "Gene" in Bezug auf Regulation der Transkription durch die Nukleinsäuren mit Promotoraktivität werden vorzugsweise Nukleinsäuren verstanden, die einen zu transkriplerenden Bereich, also beispielswelse einen Bereich der die Translation reguliert, einen kodierenden Bereich, sowie gegebenenfalls weitere Regulationselemente, wie beispielsweise einen Terminator, enthalten.

Unter dem Begriff "Gene" in Bezug auf die nachstehend beschriebene Regulation der Expression durch die Expressionseinheiten werden vorzugsweise Nukleinsäuren verstanden, die einen kodierenden Bereich, sowie gegebenenfalls weitere Regulationselemente, wie beispielsweise einen Terminator, enthalten.

Unter einem "kodierenden Bereich" wird eine Nukleinsäuresequenz verstanden, die ein Protein kodiert.

Unter "heterolog" in Bezug auf Nukleinsären mit Promotoraktivität und Gene wird verstanden, dass die verwendeten Gene im Wildtyp nicht unter Regulation der Nukleinsäuren mit Promotoraktivitat gemäß Anspruch 1 transkribiert werden, sondern das eine neue, im Wildtyp nicht vorkommende funktionelle Verknüpfung entsteht und die funktionelle Kombination aus Nukleinsäure mit Promotoraktivität und spezifisches Gen im Wildtyp nicht vorkommt.

Unter "heterolog" in Bezug auf Expressionseinheiten und Gene wird verstanden, dass die verwendeten Gene im Wildtyp nicht unter Regulation der vorgenannten Expressionseinheiten exprimiert werden, sondern das eine neue, im Wildtyp nicht vorkommende funktionelle Verknüpfung entsteht und die funktionelle Kombination aus Expressionseinheit und spezifisches Gen im Wildtyp nicht vorkommt.

Die Erfindung betrifft ferner in einer bevorzugten Ausführungsform ein Verfahren zur Erhöhung oder Verursachung der Transkriptionsrate von Genen in Mikroorganismen im Vergleich zum Wildtyp indem man
die Transkription von Genen im Mikroorganismus durch Nukleinsäuren mit Promotoraktivität
A) die Nukleinsäuresequenz SEQ.lD.NO. 1 oder
B) eine von dieser Sequenz durch Substitution, Insertion oder Deletion von Nukleotiden abgeleitete Sequenz, die eine Identität von mindestens 90 % auf Nukleinsäureebene mit der Sequenz SEQ.ID.NO. 1 aufweist oder
C) eine Nukleinsäuresequenz, die mit der Nukleinsäuresequenz SEQ.ID.NO. 1 unter stringenten Bedingungen hybridisiert, reguliert, wobei die Gene in Bezug auf die Nukleinsäuren mit Promotoraktivität heterolog sind.

Die Veränderung oder Verursachung der Expressionsrate eines Gens in Mikroorganismen im Vergleich zum Wildtyp kann durch Regulation der Expression von Genen im Mikroorganismus durch vorgenannte Expressionseinheiten , wobei die Gene im Bezug auf die Expressionseinheiten heterolog sind, erfolgen

Die Veränderung oder Verursachung der Expressionsrate von Genen in Mikroorganisrmen im Vergleich zum Wildtyp kann dadurch erfolgen, dass man die Expression von Genen im Mikroorganismus durch Expressionseinheiten derter reguliert, wobei die Gene in Bezug auf die Expressionseinheiten heterotog sind.

Dies wird bevorzugt dadurch erreicht, dass man
d1) eine oder mehrere Expressionseinheiten, gegebenenfalls mit veränderter spezifischer Expressionsaktivität, in das Genom des Mikroorganismus einbringt, so dass die Expression eines oder mehrerer endogenen Gene unter der Kontrolle der eingebrachten Expressionseinheiten erfolgt oder
d2) ein oder mehrere Gene in das Genom des Mikrorganismus einbringt, so dass die Expression eines oder mehrerer der eingebrachten Gene unter der Kontrolle der endogenen Expressionseinheiten, gegebenenfalls mit veränderter spezifischer Expressionsaktivität, erfolgt oder
d3) ein oder mehrere Nukleinsäurekonstrukte, enthaltend eine Expressionseinheit, gegebenenfalls mit veränderter spezifischer Expressionsaktivität, und funktionell verknüpft eine oder mehrere, zu exprimierende Nukleinsäuren, in den Mikroorganismus einbringt.

Es ist somit möglich, die Expressionsrate eines endogenen Gens des Wildtyps zu verändern, also zu erhöhen oder zu erniedrigen indem man
gemäß Ausführungsform d1) eine oder mehrere Expressionseinheiten, gegebenenfalls mit veränderter spezifischer Expressionsaktivität, in das Genom des Mikroorganismus einbringt, so dass die Expression eines oder mehrerer endogenen Gene unter der Kontrolle der eingebrachten Expressionseinheiten erfolgt oder
gemäß Ausführungsform d2) ein oder mehrere Gene in das Genom des Mikrorganismus einbringt, so dass die Expression eines oder mehrerer der eingebrachten Gene unter der Kontrolle der endogenen Expressionseinheiten, gegebenenfalls mit veränderter spezifischer Expressionsaktivität, erfolgt oder
gemäß Ausführungsform d3) ein oder mehrere Nukleinsäurekonstrukte, enthaltend eine Expressionseinheit, gegebenenfalls mit veränderter spezifischer Expressionsaktivität, und funktionell verknüpft eine oder mehrere, zu exprimierende Nukleinsäuren, in den Mikroorganismus einbringt.

Ferner ist es somit möglich, die Expressionssrate eines exogenen Gens im Vergleich zum Wildtyps zu verursachen, indem man gemäß Ausführungsform d2) ein oder mehrere exogene Gene in das Genom des Mikrorganismus einbringt, so dass die Expression eines oder mehrerer der eingebrachten Gene unter der Kontrolle der endogenen Expressionseinheiten, gegebenenfalls mit veränderter spezifischer Expressionsaktivität, erfolgt oder

gemäß Ausführungsform d3) ein oder mehrere Nukleinsäurekonstrukte, enthaltend eine Expressionseinheit, gegebenenfalls mit veränderter spezifischer Expressionsaktivität, und funktionell verknüpft eine oder mehrere, zu exprimierende, exogene Nukleinsäuren, in den Mikroorganismus einbringt.

Die Insertion von Genen gemäß Ausführungsform d2) kann dabei so erfolgen, dass das Gen in kodierende Bereiche oder nicht-kodierende Bereiche integriert wird. Vorzugsweise erfolgt die Insertion in nicht-kodierende Bereiche.

Die Insertion von Nukleinsäurekonstrukten gemäß Ausführungsform d3) kann dabei chromosomal oder extrachromosomal erfolgen. Vorzugsweise erfolgt die Insertion der Nukleinsäurekonstrukte chromosomal.

Die Nukleinsäurekonstrukte werden im folgenden auch als Expressionskasetten bezeichnet.

Die Erhöhung oder Verursachung der Expressionsrate eines Gens in Mikroorganismen im Vergleich zum Wildtyp kann erfolgen indem man
ch) die spezifische Expressionsaktivität im Mikroorganismus von endogenen Expressionseinheiten, die die Expression der endogenen Gene regulieren, im Vergleich zum Wildtyp erhöht oder
dh) die Expression von Genen im Mikroorganismus durch erfindungsgemäße Expressionseinheiten wobei die Gene im Bezug auf die Expressionseinheiten heterolog sind.

Vorzugsweise wird die Regulation der Expression von Genen im Mikroorganismus durch Expressionseinheiten dadurch erreicht, dass man
dh1) eine oder mehrere Expressionseinheiten, gegebenenfalls mit erhöhter spezifischer Expressionsaktivität, in das Genom des Mikroorganismus einbringt, so dass die Expression eines oder mehrerer endogenen Gene unter der Kontrolle der eingebrachten Expressionseinheiten, gegebenenfalls mit erhöhter spezifischer Expressionsaktivität, erfolgt oder
dh2) ein oder mehrere Gene in das Genom des Mikrorganismus einbringt, so dass die Expression eines oder mehrerer der eingebrachten Gene unter der Kontrolle der endogenen Expressionseinheiten, gegebenenfalls mit erhöhter spezifischer Expressionsaktivität, erfolgt oder
dh3) ein oder mehrere Nukleinsäurekonstrukte, enthaltend eine Expressionseinheit, gegebenenfalls mit erhöhter spezifischer Expressionsaktivität, und funktionell verknüpft eine oder mehrere, zu exprimierende Nukleinsäuren, in den Mikroorganismus einbringt.

In einer bevorzugten Ausführungsform des vorstehend beschriebenen erfindungsgemäßen Verfahren zur Veränderung oder Verursachung der Transkriptionsrate von Genen in Mikroorganismen sind die Gene ausgewählt aus der Gruppe Nukleinsäuren kodierend ein Protein aus den Biosyntheseweg von Feinchemikalien, wobei die Gene gegebenenfalls weitere Regulationselemente enthalten können.

In einer besonders bevorzugten Ausführungsform des vorstehend beschriebenen erfindungsgemäßen Verfahren zur Veränderung oder Verursachung der Transkriptionsrate von Genen in Mikroorganismen sind die Gene ausgewählt aus der Gruppe Nukleinsäuren kodierend ein Protein aus den Biosyntheseweg von proteinogenen und nicht-proteinogenen Aminosäuren, Nukleinsäuren kodierend ein Protein aus dem Biosyntheseweg von Nukleotiden und Nukleosiden, Nukleinsäuren kodierend ein Protein aus dem Biosyntheseweg von organischen Säuren, Nukleinsäuren kodierend ein Protein aus dem Biosyntheseweg von Lipiden und Fettsäuren, Nukleinsäuren kodierend ein Protein aus dem Biosyntheseweg von Diolen, Nukleinsäuren kodierend ein Protein aus dem Biosyntheseweg von Konhlenhydraten, Nukleinsäuren kodierend ein Protein aus dem Biosyntheseweg von aromatischen Verbindung, Nukleinsäuren kodierend ein Protein aus dem Biosyntheseweg von Vitaminen, Nukleinsäuren kodierend ein Protein aus dem Biosyntheseweg von Cofaktoren und Nukleinsäuren kodierend ein Protein aus dem Biosyntheseweg von Enzymen, wobei die Gene gegebenenfalls weitere Regulationselemente enthalten können.

In einer besondere bevorzugten Ausführungsform sind die Proteine aus dem Biosyntheseweg von Aminosäuren ausgewählt aus der Gruppe Aspartatkinase, Aspartat-Semialdehyd-Dehydrogenase, Diaminopimelat-Dehydrogenase, Diaminopimelat-Decarboxylase, Dihydrodipicolinate-Synthetase, Dihydrodipicolinate-Reduktase, Glycerinaldehyd-3-Phosphat-Dehydrogenase, 3-Phosphoglycerat-Kinase, Pyruvat-Carboxylase, Triosephosphat-Isomerase, Transkriptioneller Regulator LuxR, Transkriptioneller Regulator LysR1, Transkriptioneller Regulator LysR2, Malat-Quinon-Oxidoreduktase, Glucose-6-Phosphat-Deydrogenase, 6-Phosphogluconat-Dehydrognease, Transketolase, Transaldolase, Homoserin-O-Acetyltransferase, Cystahionin-gamma-Synthase, Cystahionin-beta-Lyase, Serin-Hydroxymethyltransferase, O-Acetylhomoserin-Sulfhydrylase, Methylen-Tetrahydrofolat-Reduktase, Phosphoserin-Aminotransferase, Phosphoserin-Phosphatase, Serin-Acetyl-Transferase, Homoserin-Dehydrogenase, Homoserin-Kinase, Threonin-Synthase, Threonin-Exporter-Carrier, Threonin-Dehydratase, Pyruvat-Oxidase, Lysin-Exporter, Biotin-Ligase, Cystein-Synthasel, Cystein-Synthase II, Coenzym B12-abhängige Methionin-Synthase, Coenzym B12-unabhängige Methionin-Synthase-Aktivität, Sulfatadenyltransferase Untereinheit 1 und 2, Phosphoadenosin Phosphosulfat Reduktase, Ferredoxin-sulfit-reductase, Ferredoxin NADP Reduktase, 3-Phosphoglycerat Dehydrogenase, RXA00655 Regulator, RXN2910-Regulator, Arginyl-t-RNA-Synthetase, Phosphoenolpyruvat-Carboxylase, Threonin Efflux-Protein, Serinhydroxymethyltransferase, Fruktose-1,6-bisphosphatase, Protein der Sulfat-Reduktion RXA077, Protein der Sulfat-Reduktion RXA248, Protein der Sulfat-Reduktion RXA247, Protein OpcA, 1-Phosphofruktokinase und 6-Phosphofruktokinase.

Bevorzugte Proteine und Nukleinsäuren kodierend diese Proteine der vorstehend beschriebenen Proteine aus dem Biosyntheseweg von Aminosäuren sind Proteinsequenzen bzw. Nukleinsäuresequenzen mikrobiellen Ursprungs, vorzusgweise aus Bakterien der Gattung Corynebacterium oder Brevibacterium, bevorzugt aus coryneformen Bakterien, besonders bevorzugt aus Corynebakterium glutamicum.

Beispiele für besonders bevorzugte Proteinsequenzen und die entsprechenden Nukleinsäuresequenzen kodierend diese Proteine aus dem Biosyntheseweg von Aminosäuren, deren Bezugsdokument, sowie deren Bezeichnung im Bezugsdokument sind in Tabelle 1 aufgelistet:

**Tabelle 1**

| Protein | Nukleinsäure kodierend Protein | Bezugsdokument | SEQ. ID. NO. im Bezugsdokument |
|---|---|---|---|
| Aspartatkinase | ask oder lysC | EP1108790 | DNA: 281 |
| | | | Protein: 3781 |
| Aspartat-Semialdehyd-Dehydrogenase | asd | EP1108790 | DNA: 331 |
| | | | Protein: 3831 |
| Dihydrodipicolinate-Synthetase | dapA | WO 0100843 | DNA: 55 |
| | | | Protein: 56 |
| Dihydrodipicolinate-Reduktase | dapB | WO 0100843 | DNA: 35 |
| | | | Protein: 36 |
| Meso-Diaminopimelat-D-Dehydrogenase | ddh | EP1108790 | DNA : 3494 |
| | | | Protein : 6944 |
| Diaminopicolinat-Decarboxylase | lysA | EP1108790 | DNA: 3451 |
| | | | Prot.:6951 |
| Lysin-Exporter | lysE | EP1108790 | DNA: 3455 |
| | | | Prot.: 6955 |
| Arginyl-t-RNA Synthetase | argS | EP1108790 | DNA: 3450 |
| | | | Prot.: 6950 |
| Glucose-6-Phosphat-Dehydrognease | zwf | WO 0100844 | DNA: 243 |
| | | | Prot.: 244 |
| Glycerinaldehyd-3-Phosphat-Dehydrogenase | gap | WO 0100844 | DNA: 187 |
| | | | Prot.: 188 |
| 3-Phosphoglycerat-kinase | pgk | WO 0100844 | DNA: 69 |
| | | | Prot.: 70 |
| Pyruvat-Carboxylase | pycA | EP1108790 | DNA 765 |
| | | | Prot.: 4265 |
| Triosephosphat-Isomerase | tpi | WO 0100844 | DNA: 61 |
| | | | Prot.: 62 |
| Biotin-Ligase | birA | EP1108790 | DNA: 786 |
| | | | Prot.: 4286 |
| PEP-Carboxylase | pck | EP1108790 | DNA: 3470 |
| | | | Prot.: 6970 |
| Homoserin Kinase | thrB | WO 0100843 | DNA: 173 |
| | | | Prot.: 174 |
| Threonin Synthese | thrC | WO 0100843 | DNA: 175 |
| | | | Prot.: 176 |
| Threonin Export Carrier | thrE | WO 0251231 | DNA: 41 |
| | | | Prot.: 42 |
| Threonin Efflux Protein | RXA2390 | WO 0100843 | DNA: 7 |
| | | | Prot.: 8 |
| Threonin Dehydratase | ilvA | EP 1108790 | DNA: 2328 |
| | | | Prot.: 5828 |
| Homoserin-O-Acetyltransferase | metA | EP 1108790 | DNA:727 |
| | | | Prot: 4227 |
| Cystathionin-gamma-synthase | metB | EP 1108790 | DNA:3491 |
| | | | Prot: 6991 |
| Cystathionin-beta-Lyase | metC | EP 1108790 | DNA:2535 |
| | | | Prot: 6035 |
| Coenzym B12-abhängige Methionin-Synthase, | metH | EP 1108790 | DNA:1663 |
| | | | Prot: 5163 |
| | | | |
| O-Acetylhomoserin-Sulfhydrylase | metY | EP 1108790 | DNA:726 |
| | | | Prot: 4226 |
| Methylentetrahydrofolat-Reduktase | metF | EP 1108790 | DNA:2379 |
| | | | Prot: 5879 |
| D-3-Phosphoglycerat-Dehydrogenase | serA | EP 1108790 | DNA:1415 |
| | | | Prot: 4915 |
| Phosphoserin-Phosphatase 1 | serB | WO 0100843 | DNA: 153 |
| | | | Prot.:154 |
| Phosphoserin-Phosphatase 2 | serB | EP 1108790 | DNA: 467 |
| | | | Prot: 3967 |
| Phosphoserin-Phosphatase 3 | serB | EP 1108790 | DNA: 334 |
| | | | Prot.: 3834 |
| Phosphoserin-Aminotransferase | serC | WO 0100843 | DNA: 151 |
| | | | Prot.: 152 |
| Serin Acetyl-Transferase | cysE | WO 0100843 | DNA: 243 |
| | | | Prot.: 244 |
| Cystein-Synthase I | cysK | EP 1108790 | DNA: 2817 |
| | | | Prot.: 6317 |
| Cystein Synthase II | CysM | EP 1108790 | DNA: 2338 |
| | | | Prot.: 5838 |
| Homoserin-Dehydrogenase | hom | EP 1108790 | DNA: 3452 |
| | | | Prot.: 6952 |
| Coenzym B12-unabhängige Methionin-Synthase | metE | WO 0100843 | DNA:755 |
| | | | Prot.: 756 |
| | | | |
| Serin-Hydroxymethyltransferase | glyA | WO 0100843 | DNA: 143 |
| | | | Prot.: 144 |
| | | | |
| Protein in Sulfat-Reduktion | RXA247 | EP 1108790 | DNA: 3089 |
| | | | Prot.: 6589 |
| Protein in Sulfat-Reduktion | RXA248 | EP 1108790 | DNA: 3090 |
| | | | Prot.: 6590 |
| Sulfatadenyltransferase Untereinheit 1 | CysN | EP 1108790 | DNA: 3092 |
| | | | Prot.: 6592 |
| Sulfatadenyltransferase Untereinheit 2 | CysD | EP 1108790 | DNA: 3093 |
| | | | Prot.: 6593 |
| Phosphoadenosin Phosphosulfat Reduktase | CysH | WO 02729029 | DNA: 7 |
| | | | Prot.: 8 |
| Ferredoxin-Sulfit-Reduktase | RXA073 | WO 0100842 | DNA: 329 |
| | | | Prot.: 330 |
| Ferredoxin NADP Reduktase | RXA076 | WO 0100843 | DNA: 79 |
| | | | Prot.: 80 |
| Transkriptioneller Regulator LuxR | luxR | WO 0100842 | DNA: 297 |
| | | | Protein: 298 |
| Transkriptioneller Regulator LysR1 | lysR1 | EP 1108790 | DNA: 676 |
| | | | Protein: 4176 |
| Transkriptioneller Regulator LysR2 | lysR2 | EP 1108790 | DNA: 3228 |
| | | | Protein: 6728 |
| Transkriptioneller Regulator LysR3 | lysR3 | EP 1108790 | DNA: 2200 |
| | | | Protein: 5700 |
| Malat-Quinon-Oxodoreduktase | mqo | WO 0100844 | DNA: 569 |
| | | | Protein: 570 |
| Transketolase | RXA2739 | EP 1108790 | DNA: 1740 |
| | | | Prot: 5240 |
| Transaldolase | RXA2738 | WO 0100844 | DNA: 245 |
| | | | Prot: 246 |
| OPCA | opcA | WO 0100804 | DNA: 79 |
| | | | Prot: 80 |
| 1-Phosphofructokinase 1 | pfk1 | WO0100844 | DNA: 55 |
| | | | Protein: 56 |
| 1-Phosphofructokinase 2 | pfk2 | WO0100844 | DNA: 57 |
| | | | Protein: 58 |
| 6-Phosphofructokinase 1 | 6-pfk1 | EP 1108790 | DNA: 1383 |
| | | | Protein: 4883 |
| 6-Phosphofructokinase 2 | 6-pfk2 | DE 10112992 | DNA: 1 |
| | | | Protein: 2 |
| Fructose-1,6-bisphosphatase 1 | fbr1 | EP1108790 | DNA:1136 |
| | | | Protein: 4636 |
| Pyruvat Oxidase | poxB | WO 0100844 | DNA : 85 |
| | | | Protein: 86 |
| RXA00655-Regulator | RXA655 | US2003162267 .2 | DNA: 1 |
| | | | Prot.: 2 |
| RXN02910-Regulator | RXN2910 | US2003162267 .2 | DNA: 5 |
| | | | Prot.: 6 |
| 6-phosphogluconolactonase | RXA2735 | WO 0100844 | DNA: 1 |
| | | | Prot.: 2 |

Ein weiteres Beispiel für eine besonders bevorzugte Proteinsequenz und die entsprechenden Nukleinsäuresequenz kodierend dieses Protein aus dem Biosyntheseweg von Aminosäuren, ist die Sequenz der Fructose-1,6-bisphosphatase 2, oder auch fbr2 genannt, (SEQ. ID. NO.20) und die entsprechenden Nukleinsäuresequenz kodierend eine Fructose-1,6-bisphosphatase 2 (SEQ. ID. NO.19).

Ein weiteres Beispiel für eine besonders bevorzugte Proteinsequenz und die entsprechenden Nukleinsäuresequenz kodierend dieses Protein aus dem Biosyntheseweg von Aminosäuren, ist die Sequenz des Proteins in Sulfat-Reduktion, oder auch RXA077 genannt, (SEQ. ID. NO. 3) und die entsprechenden Nukleinsäuresequenz kodierend ein Protein in Sulfat-Reduktion (SEQ. ID. NO. 4)

Weitere besonders bevorzugte Proteinsequenzen aus dem Biosyntheseweg von Aminosäuren, weisen jeweils die in Tabelle 1 für dieses Protein angegebene Aminosäuresequenz auf, wobei das jeweilige Protein jeweils an mindestens einer der in Tabelle 2/Spalte2 für diese Aminosäuresequenz angegebenen Aminosäurepositionen eine andere proteinogene Aminosäure aufweist als die jeweilige in Tabelle2/Spalte3 in der gleichen Zeile angegebene Aminosäure. In einer weiter bevorzugten Ausführungsform, weisen die Proteine an mindestens einer der in Tabelle 2/Spalte 2 für die Aminosäuresequenz angegebenenen Aminosäureposition die in Tabelle2/Spalte4 in der gleichen Zeile angegebene Aminosäure auf. Es handelt sich bei den in Tabelle 2 angegebenen Proteine um mutierte Proteine des Biosyntheseweges von Aminosäuren, die besonders vorteilhafte Eigenschaften aufweisen und sich deshalb insbesondere zur Expression der entsprechenden Nukleinsäuren durch den erfinungsgemäßen Promotor und zur Herstellung von Aminosäuren eignen. Beispielsweise führt die Mutation T3111 zu einem Ausschalten der feedback-Inhibierung von ask.

Die entsprechenden Nukleinsäuren, die ein vorstehend beschriebenes mutiertes Protein aus Tabelle 2 kodieren, lassen sich durch übliche verfahren herstellen.

Als Ausgangspunkt zur Herstellung der Nukleinsäuresequenzen kodierend ein mutiertes Protein eignet sich beispielsweise das Genom eines Corynebacterium glutamicum-Stammes, der von der American Type Culture Collection unter der Bezeichnung ATCC 13032 erhältlich ist oder die in Tabelle 1 in Bezug genommenen Nukleinsäuresequenzen. Für die Rückübersetzung der Aminosäuresequenz der mutierten Proteine in die Nukleinsäuresequenzen kodierend diese Proteine ist es vorteilhaft, die codon usage desjenigen Organismus zu verwenden, in den die Nukleinsäuresequenz eingebracht werden soll oder in der die Nukleinsäuresequenz vorliegt. Beispielsweise ist es vorteihaft für Corynebakterium glutamicum die codon usage von Corynebakterium glutamicum zu verwenden. Die codon usage des jeweiligen Organismus lässt sich in an sich bekannter Weise aus Datenbanken oder Patentanmeldungen ermitteln, die zumindest ein Protein und ein Gen, das dieses Protein kodiert, aus dem gewünschten Organismus beschreiben.

Die Angaben in Tabelle 2 sind folgendermassen zu verstehen:

In Spalte 1"Identifikation" wird eine eindeutige Bezeichnung für jede Sequenz in Bezug auf Tabelle 1 aufgeführt.

In Spalte 2 "AS-POS" bezieht sich die jeweilige Zahl auf die Aminosäureposition der entsprechenden Polypeptidsequenz aus Tabelle 1. Eine "26" in der Spalte "AS-POS" bedeutet demzufolge die Aminosäureposition 26 der entsprechend angegebenen Polypeptidsequenz. Die Zählung der Position beginnt N-Terminal bei +1.

In Spalte 3 "AS-Wildtyp" bezeichnet der jeweilige Buchstabe die Aminosäure - dargestellt im Ein-Buchstaben-Code- an der in Spalte 2 angegebenen Position beim entsprechenden Wildtyp-Stamm der Sequenz aus Tabelle 1.

In Spalte 4 "AS-Mutante" bezeichnet der jeweilige Buchstabe die Aminosäure - dargestellt im Ein-Buchstaben-Code- an der in Spalte 2 angegebenen Position beim entsprechenden Mutanten-Stamm.

In Spalte 5 "Funktion" wird die physiologische Funktion der entsprechenden Polypeptidsequenz aufgeführt.

Für ein mutiertes Protein mit einer bestimmten Funktion (Spalte 5) und einer bestimmten Ausgangsaminosäuresequenz (Tabelle 1) werden in den Spalten 2,3 und 4 mindestens eine Mutation, bei einigen Sequenzen auch mehrere Mutationen beschrieben. Diese mehreren Mutationen beziehen sich immer auf die jeweils obenstehende, nächstliegendste Ausgangsaminosäuresequenz (Tabelle 1). Unter dem Begriff "mindestens eine der Aminsäurepositionen" einer bestimmten Aminosäuresequenz wird vorzugsweise mindestens eine der für diese Aminosäuresequenz in Spalte 2, 3 und 4 beschriebenen Mutationen verstanden.

Ein-Buchstaben-Code der proteinogenen Aminosäuren:
A Alanin
C Cystein
D Aspartat
E Glutamat
FPhenylalanin
G Glycin
H His
1 Isoleucin
K Lysin
LLeucin
M Methionin
N Asparagin
P Prolin
Q Glutamin
R Arginin
S Serin
TThreonin
V Valin
W Tryptophan
Y Tyrosin

**Tabelle 2**

| Spalte 1 | Spalte 2 | Spalte 3 | Spalte 4 | Spalte 5 |
|---|---|---|---|---|
| Identifikation | AS Position | AS Wildtyp | AS Mutante | Funktion |
| ask | 317 | S | A | Aspartatkinase |
| | 311 | T | I | |
| | 279 | A | T | |
| asd | 66 | D | G | Aspartat-Semialdehyd-Dehydrogenase |
| | 234 | R | H | |
| | 272 | D | E | |
| | 285 | K | E | |
| | 20 | L | F | |
| dapA | 2 | S | A | Dihydrodipicolinat Synthetase |
| | 84 | K | N | |
| | 85 | L | V | |
| dapB | 91 | D | A | Dihydrodipicolinat-Reduktase |
| | 83 | D | N | |
| ddh | 174 | D | E | Meso-Diaminopimelat-D-Dehydrogenase |
| | 235 | F | L | |
| | 237 | S | A | |
| IysA | 265 | A | D | Diaminopicolinat-Decarboxylase |
| | 320 | D | N | |
| | 332 | I | V | |
| argS | 355 | G | D | Arginyl-t-RNA-Synthetase |
| | 156 | A | S | |
| | 513 | V | A | |
| | 540 | H | R | |
| zwf | 8 | S | T | Glucose-6-Phosphat-Dehydrogenase |
| | 150 | T | A | |
| | 321 1 | G | S | |
| gap | 264 | G | S | Glycerinaldehyd-3-Phosphat-Dehydrogenase |
| pycA | 7 | S | L | Pyrwat-Carboxylase |
| | 153 | E | D | |
| | 182 | A | S | |
| | 206 | A | S | |
| | 227 | H | R | |
| | 455 | A | G | |
| | 458 | P | S | |
| | 639 | S | T | |
| | 1008 | R | H | |
| | 1059 | S | P | |
| | 1120 | D | E | |
| pck | 162 | H | Y | PEP-Carboxylase |
| | 241 | G | D | |
| | 829 | T | R | |
| thrB | 103 | S | A | Homoserin Kinase |
| | 190 | T | A | |
| | 133 | A | V | |
| | 138 | P | S | |
| thrC | 69 | G | R | Threonin Synthase |
| | 478 | T | I | |
| RXA330 | 85 | I | M | Threonin-Efflux Protein |
| | 161 | F | I | |
| | 195 | G | D | |
| hom | 104 | V | I | Homoserin-Deydrogenase |
| | 116 | T | I | |
| | 148 | G | A | |
| | 59 | V | A | |
| | 270 | T | S | |
| | 345 | R | P | |
| | 268 | K | N | |
| | 61 | D | H | |
| | 72 | E | Q | |
| lysR1 | 80 | R | H | transkriptioneller Regulator LysR1 |
| lysR3 | 142 | R | W | transkriptioneller Regulator LysR3 |
| | 179 | A | T | |
| RXA2739 | 75 | N | D | Transketolase |
| | 329 | A | T | |
| | 332 | A | T | |
| | 556 | V | I | |
| RXA2738 | 242 | K | M | Transaldolase |
| opcA | 107 | Y | H | OpcA |
| | 219 | K | N | |
| | 233 | P | S | |
| | 261 | Y | H | |
| | 312 | S | F | |
| | 65 | G | R | Aspartat-1-Decarboxylase |
| | 33 | G | S | 6- Phosphogluconolactonase |

In derm vorstehend beschriebenen erfindungsgemäßen Verfahren zur Veränderung oder Verursachung der Transkriptionsrate von Genen in Mikroorganismen sowie den nachstehend beschriebenen Verfahren zur Herstellung von genetisch veränderten Mikroorganismen, den nachstehend beschriebenen genetisch veränderten Mikroorganoismen und den nachstehend beschriebenen Verfahren zur Herstellung von biosynthetischen Produkten erfolgt das Einbringen der Nukleinsäuren mit Promotoraktivität gemäß Anspruch 1, der Expressionseinheiten, der vorstehend beschriebenen Gene und der vorstehend beschriebnen Nukleinsäurekonstrukte oder Expressionskasetten in den Mikroorganismus, insbeondere in corynefome Bakterien, vorzugsweise durch die SacB-Methode.

Die SacB-Methode ist dem Fachmann bekannt und beispielsweise in Schäfer A, Tauch A, Jäger W, Kalinowski J, Thierbach G, Pühler A.; Small mobilizable multi-purpose cloning vectors derived from the Escherichia coli plasmids pK18 and pK19: selection of defined deletions in the chromosome of Corynebacterium glutamicum, Gene. 1994 Jul 22;145(1):69-73 und Blomfield IC, Vaughn V, Rest RF, Eisenstein BI; Allelic exchange in Escherichia coli using the Bacillus subtilis sacB gene and a temperature-sensitive pSC101 replicon; Mol Microbiol. 1991 Jun;5(6):1447-57 beschrieben.

In einer bevorzugten Ausführungsform dem vorstehend beschriebenen erfindungsgemäßen Verfahren erfolgt die Veränderung oder Verursachung der Transkriptionsrate von Genen in Mikroorganismen durch Einbringen von Nukleinsäuren mit Promotoraktivität gemäß Anspruch 1 bzw. Expressionseinheiten in den Mikroorganismus.

In einer weiteren bevorzugten Ausführungsform des orstehend beschriebenen erfindungsgemäßen Verfahren erfolgt die Veränderung oder Verursachung der Transkriptionsrate von Genen in Mikroorganismen durch Einbringen der vorstehend beschriebenen Nukleinsäurekonstrukte oder Expressionskasetten in den Mikroorganismus.

Vektoren sind dem Fachmann wohl bekannt und können beispielsweise aus "Cloning Vectors" (Pouwels P. H. et al., Hrsg, Elsevier, Amsterdam-New York-Oxford, 1985) entnommen werden. Unter Vektoren sind außer Plasmiden auch alle anderen dem Fachmann bekannten Vektoren, wie beispielsweise Phagen, Transposons, IS-Elemente, Phasmide, Cosmide, und lineare oder zirkuläre DNA zu verstehen. Diese Vektoren können autonom im Wirtsorganismus repliziert oder chromosomal repliziert werden.

Als Plasmide eignen sich solche besonders bevorzugt, die in coryneformen Bakterien repliziert werden. Zahlreiche bekannte Plasmidvektoren, wie z. B. pZ1 (Menkel et al., Applied and Environmental Microbiology (1989) 64: 549-554), pEKEx1 (Eikmanns et al., Gene 102: 93-98 (1991)) oder pHS2-1 (Sonnen et al., Gene 107: 69-74 (1991)) beruhen auf den kryptischen Plasmiden pHM1519, pBL1 oder pGA1. Andere Plasmidvektoren, wie z. B. pCLiK5MCS, oder solche, die auf pCG4 (US-A 4,489,160) oder pNG2 (Serwold-Davis et al., FEMS Microbiology Letters 66,119-124 (1990)) oder pAG1 (US-A 5,158,891) beruhen, können in gleicher Weise verwendet werden.

Weiterhin eignen sich auch solche Plasmidvektoren mit Hilfe derer man das Verfahren der Genamplifikation durch Integration in das Chromosom anwenden kann, so wie es beispielsweise von Remscheid et al. (Applied and Environmental Microbiology 60,126-132 (1994)) zur Duplikation bzw. Amplifikation des hom-thrB-Operons beschrieben wurde. Bei dieser Methode wird das vollständige Gen in einen Plasmidvektor kloniert, der in einem Wirt (typischerweise E. coli), nicht aber in C. glutamicum replizieren kann. Als Vektoren kommen beispielsweise pSUP301 (Simon et al., Bio/ Technology 1,784-791 (1983)), pK18mob oder pK19mob (Schäfer et al., Gene 145,69-73 (1994)), Bernard et al., Journal of Molecular Biology, 234: 534-541 (1993)), pEM1 (Schrumpf et al. 1991, Journal of Bacteriology 173: 4510-4516) oder pBGS8 (Spratt et al., 1986, Gene 41: 337-342) in Frage. Der Plasmidvektor, der das zu amplifizierende Gen enthält, wird anschließend durch Transformation in den gewünschten Stamm von C. glutamicum überführt. Methoden zur Transformation sind beispielsweise bei Thierbach et al. (Applied Microbiology and Biotechnology 29, 356-362 (1988)), Dunican und Shivnan (Biotechnology 7, 1067-1070 (1989)) und Tauch et al. (FEMS Microbiological Letters 123,343-347 (1994)) beschrieben.

Die Erfindung betrifft ferner einen genetisch verändertern Mikroorganismus, wobei die genetische Veränderung zu einer Veränderung oder Verursachung der Transkriptionsrate von mindestens einem Gen im Vergleich zum Wildtyp führt und bedingt ist durch

Regulation der Transkription von Genen im Mikroorganismus durch Nukleinsäuren mit Promotoraktivität gemäß Anspruch 7 wobei die Gene in Bezug auf die Nukleinsäuren mit Promotoraktivität heterolog sind.

Wie vorstehend bei den Verfahren beschrieben, wird die Regulation der Transkription von Genen im Mikroorganismus durch Nukleinsäuren mit Promotoraktivität gemäß Anspruch 7 dadurch erreicht, dass man
b1) eine oder mehrere Nukleinsäuren mit Promotoraktivität gemäß Anspruch 7, gebenenfalls mit veränderter spezifischer Promotoraktivität, in das Genom des Mikroorganismus einbringt, so dass die Transkription eines oder mehrerer endogenen Gene unter der Kontrolle der eingebrachten Nukleinsäure mit Promotoraktivität gemäß Anspruch 7, gegebenenfalls mit veränderter spezifischer Promotoraktivität, erfolgt oder
b2) ein oder mehrere Gene in das Genom des Mikrorganismus einbringt, so dass die Transkription eines oder mehrerer der eingebrachten Gene unter der Kontrolle der endogenen Nukleinsäuren mit Promotoraktivität gemäß Anspruch 7, gegebenenfalls mit veränderter spezifischer Promotoraktivität, erfolgt oder
b3) ein oder mehrere Nukleinsäurekonstrukte, enthaltend eine Nukleinsäure mit Promotoraktivität gemäß Anspruch 7, gegebenenfalls mit veränderter spezifischer Promotoraktivität, und funktionell verknüpft eine oder mehrere, zu transkribierende Nukleinsäuren, in den Mikroorganismus einbringt.

Die Erfindung betrifft ferner eine genetisch verändertern Mikroorganismus mit erhöhter oder verursachter Transkriptionsrate von mindestens einem Gen im Vergleich zum Wildtyp, wobei die Transkription von Genen im Mikroorganismus durch Nukleinsäuren mit Promotoraktivität gemäß Anspruch 7 reguliert wird, wobei die Gene in Bezug auf die Nukleinsäuren mit Promotoraktivität heterolog sind.

Wie vorstehend bei den Verfahren beschrieben, wird die Regulation der Transkription von Genen im Mikroorganismus durch Nukleinsäuren mit Promotoraktivität gemäß Anspruch 7 dadurch erreicht, dass man
bh1) eine oder mehrere Nukleinsäuren mit Promotoraktivität gemäß Anspruch 7, gegebenenfalls mit erhöhter spezifischer Promotoraktivität, in das Genom des Mikroorganismus einbringt, so dass die Transkription eines oder mehrerer endogenen Gene unter der Kontrolle der eingebrachten Nukleinsäure mit Promotoraktivität, gegebenenfalls mit erhöhter spezifischer Promotoraktivität, erfolgt oder
bh2) ein oder mehrere Gene in das Genom des Mikrorganismus einbringt, so dass die Transkription eines oder mehrerer der eingebrachten Gene unter der Kontrolle der endogenen Nukleinsäuren mit Promotoraktivität gemäß Anspruch 7, gegebenenfalls mit erhöhter spezifischer Promotoraktivität, erfolgt oder
bh3) ein oder mehrere Nukleinsäurekonstrukte, enthaltend eine Nukleinsäure mit Promotoraktivität gemäß Anspruch 7, gegebenenfalls mit erhöhter spezifischer Promotoraktivität, und funktionell verknüpft eine oder mehrere, zu transkribierende Nukleinsäuren, in den Mikroorganismus einbringt.

Genetisch verändertern Mikroorganismus, können auch genetische veränderungen enthalten, die zu einer Veränderung oder Verursachung der Expressionsrate mindestens eines Gens im Vergleich zum Wildtyp führen und bedingt sind durch die Regulation der Expression von Genen im Mikroorganismus durch Expressionseinheiten ), wobei die Gene im Bezug auf die Expressionseinheiten heterolog sind.

Wie vorstehend bei den Verfahren beschrieben, wird die Regulation der Expression von Genen im Mikroorganismus durch Expressionseinheiten dadurch erreicht, dass man
d1) eine oder mehrere Expressionseinheiten gegebenenfalls mit veränderter spezifischer Expressionsaktivität, in das Genom des Mikroorganismus einbringt, so dass die Expression eines oder mehrerer endogenen Gene unter der Kontrolle der eingebrachten Expressionseinheiten gegebenenfalls mit veränderter spezifischer Expressionsaktivität, erfolgt oder
d2) ein oder mehrere Gene in das Genom des Mikrorganismus einbringt, so dass die Expression eines oder mehrerer der eingebrachten Gene unter der Kontrolle der endogenen Expressionseinheiten gegebenenfalls mit veränderter spezifischer Expressionsaktivität, erfolgt oder
d3) ein oder mehrere Nukleinsäurekonstrukte, enthaltend eine Expressionseinheit , gegebenenfalls mit veränderter spezifischer Expressionsaktivität, und funktionell verknüpft eine oder mehrere, zu exprimierende Nukleinsäuren, in den Mikroorganismus einbringt.

Besonders bevorzugt betrifft die vorliegende Erfindung gentisch veränderte Mikroorgansismen, insbesondere coryneforme Bakterien, die einen Vektor, insbesondere Pendelvektor oder Plasmidvektor, der wenigstens ein rekombinantes Nukleinsäurekonstrukt erfindungsgemäßer Definition trägt, enthalten.

In einer bevorzugten Ausführungsform der genetisch veränderten Mikroorganismen sind die vorstehend beschriebenen Gene mindestens eine Nukleinsäuren kodierend ein Protein aus den Biosyntheseweg von Feinchemikalien.

In einer besonders bevorzugten Ausführungsform der genetisch veränderten Mikroorganismen sind die vorstehend beschriebenen Gene ausgewählt sind aus der Gruppe Nukleinsäuren kodierend ein Protein aus den Biosyntheseweg von proteinogenen und nicht-proteinogenen Aminosäuren, Nukleinsäuren kodierend ein Protein aus dem Biosyntheseweg von Nukleotiden und Nukleosiden, Nukleinsäuren kodierend ein Protein aus dem Biosyntheseweg von organischen Säuren, Nukleinsäuren kodierend ein Protein aus dem Biosyntheseweg von Lipiden und Fettsäuren, Nukleinsäuren kodierend ein Protein aus dem Biosyntheseweg von Diolen, Nukleinsäuren kodierend ein Protein aus dem Biosyntheseweg von Konhlenhydraten, Nukleinsäuren kodierend ein Protein aus dem Biosyntheseweg von aromatischen Verbindung, Nukleinsäuren kodierend ein Protein aus dem Biosyntheseweg von Vitaminen, Nukleinsäuren kodierend ein Protein aus dem Biosyntheseweg von Cofaktoren und Nukleinsäuren kodierend ein Protein aus dem Biosyntheseweg von Enzymen, wobei die Gene gegebenenfalls weitere Regulationselemente enthalten können.

Bevorzugte Proteine aus dem Biosyntheseweg von Aminosäuren sind ausgewählt aus der Gruppe Aspartatkinase, Aspartat-Semialdehyd-Dehydrogenase, Diaminopimelat-Dehydrogenase, Diaminopimelat-Decarboxylase, Dihydrodipicolinate-Synthetase, Dihydrodipicolinate-Reduktase, Glycerinaldehyd-3-Phosphat-Dehydrogenase, 3-Phosphoglycerat-Kinase, Pyruvat-Carboxylase, Triosephosphat-Isomerase, Transkriptioneller Regulator LuxR, Transkriptioneller Regulator LysR1, Transkriptioneller Regulator LysR2, Malat-Quinon-Oxidoreduktase, Glucose-6-Phosphat-Deydrogenase, 6-Phosphogluconat-Dehydrognease, Transketolase, Transaldolase, Homoserin-O-Acetyltransferase, Cystahionin-gamma-Synthase, Cystahionin-beta-Lyase, Serin-Hydroxymethyltransferase, O-Acetylhomoserin-Sulfhydrylase, Methylen-Tetrahydrofolat-Reduktase, Phosphoserin-Aminotransferase, Phosphoserin-Phosphatase, Serin-Acetyl-Transferase, Homoserin-Dehydrogenase, Homoserin-Kinase, Threonin-Synthase, Threonin-Exporter-Carrier, Threonin-Dehydratase, Pyruvat-Oxidase, Lysin-Exporter, Biotin-Ligase, Cystein-Synthasel, Cystein-Synthase II Coenzym B12-abhängige Methionin-Synthase, Coenzym B12-unabhängige Methionin-Synthase-Aktivität, Sulfatadenyltransferase Untereinheit 1 und 2, Phosphoadenosin Phosphosulfat Reduktase, Ferredoxin-sulfit-reductase, Ferredoxin NADP Reduktase, 3-Phosphoglycerat Dehydrogenase, RXA00655 Regulator, RXN2910-Regulator, Arginyl-t-RNA-Synthetase, Phosphoenolpyruvat-Carboxylase, Threonin Efflux-Protein, Serinhydroxymethyltransferase, Fruktose-1,6-bisphosphatase, Protein der Sulfat-Reduktion RXA077, Protein der Sulfat-Reduktion RXA248, Protein der Sulfat-Reduktion RXA247, Protein OpcA, 1-Phosphofruktokinase und 6-Phosphofruktokinase

Besonders bevorzugte Beispiele der Proteine und Gene aus dem Biosyntheseweg von Aminosäuren sind vorstehend in Tabelle 1 und Tabelle 2 beschrieben.

Bevorzugte Mikroorganismen oder genetisch veränderte Mikroorganismen sind Bakterien, Algen, Pilze oder Hefen.

Besonders bevorzugte Mikroorgansimen sind insbeondere coryneforme Bakterien.

Bevorzugte coryneforme Bakterien sind Bakterien der Gattung Corynebacterium, insbesondere der Arten Corynebacterium glutamicum, Corynebacterium acetoglutamicum, Corynebacterium acetoacidophilum, Corynebacterium thermoaminogenes, Corynebacterium melassecola und Corynebacterium efficiens oder der Gattung Brevibacterium, insbesondere der Arten Brevibacterium flavum, Brevibacterium lactofermentum und Brevibacterium divaricatum.

Besonders bevorzugte Bakterien der Gattungen Corynebacterium und Brevibacterium sind ausgewählt aus der Gruppe Corynebacterium glutamicum ATCC 13032, Corynebacterium acetoglutamicum ATCC 15806, Corynebacterium acetoacidophilum ATCC 13870, Corynebacterium thermoaminogenes FERM BP-1539, Corynebacterium melassecola ATCC 17965, Corynebacterium efficiens DSM 44547, Corynebacterium efficiens DSM 44548. Corynebacterium efficiens DSM 44549, Brevibacterium flavum ATCC 14067, Brevibacterium lactofermentum ATCC 13869, Brevibacterium divaricatum ATCC 14020, Corynebacterium glutamicum KFCC10065 und Corynebacterium glutamicum ATCC21608.

Mit der Abkürzung KFCC ist die Korean Federation of Culture Collection gemeint, mit der Abkürzung ATCC die American type strain culture collection, mit der Abkürzung DSM die Deutsche Sammlung von Mikroorganismen.

Weitere besonders bevorzugte Bakterien der Gattungen Corynebacterium und Brevibacterium sind in Tabelle 3 aufgelistet:

| Bakterium | | Hinterlegungsnummer | | | | | | | |
|---|---|---|---|---|---|---|---|---|---|
| Genus | species | ATCC | FER | NRRL | CEC T | NCIMB | CBS | NCTC | DSMZ |
| Brevibacterium | ammoniagenes | 21054 | | | | | | | |
| Brevibacterium | ammoniagenes | 19350 | | | | | | | |
| Brevibacterium | ammoniagenes | 19351 | | | | | | | |
| Brevibacterium | ammoniagenes | 19352 | | | | | | | |
| Brevibacterium | ammoniagenes | 19353 | | | | | | | |
| Brevibacterium | ammoniagenes | 19354 | | | | | | | |
| Brevibacterium | ammoniagenes | 19355 | | | | | | | |
| Brevibacterium | ammoniagenes | 19356 | | | | | | | |
| Brevibacterium | ammoniagenes | 21055 | | | | | | | |
| Brevibacterium | ammoniagenes | 21077 | | | | | | | |
| Brevibacterium | ammoniagenes | 21553 | | | | | | | |
| Brevibacterium | ammoniagenes | 21580 | | | | | | | |
| Brevibacterium | ammoniagenes | 39101 | | | | | | | |
| Brevibacterium | butanicum | 21196 | | | | | | | |
| Brevibacterium | divaricatum | 21792 | P928 | | | | | | |
| Brevibacterium | flavum | 21474 | | | | | | | |
| Brevibacterium | flavum | 21129 | | | | | | | |
| Brevibacterium | flavum | 21518 | | | | | | | |
| Brevibacterium | flavum | | | B11474 | | | | | |
| Brevibacterium | flavum | | | B11472 | | | | | |
| Brevibacterium | flavum | 21127 | | | | | | | |
| Brevibacterium | flavum | 21128 | | | | | | | |
| Brevibacterium | flavum | 21427 | | | | | | | |
| Brevibacterium | flavum | 21475 | | | | | | | |
| Brevibacterium | flavum | 21517 | | | | | | | |
| Brevibacterium | flavum | 21528 | | | | | | | |
| Brevibacterium | flavum | 21529 | | | | | | | |
| Brevibacterium | flavum | | | B11477 | | | | | |
| Brevibacterium | flavum | | | B11478 | | | | | |
| Brevibacterium | flavum | 21127 | | | | | | | |
| Brevibacterium | flavum | | | B11474 | | | | | |
| Brevibacterium | healii | 15527 | | | | | | | |
| Brevibacterium | ketoglutamicum | 21004 | | | | | | | |
| Brevibacterium | ketoglutamicum | 21089 | | | | | | | |
| Brevibacterium | ketosoreductum | 21914 | | | | | | | |
| Brevibacterium | lactofermentum | | | | 70 | | | | |
| Brevibacterium | lactofermentum | | | | 74 | | | | |
| Brevibacterium | lactofermentum | | | | 77 | | | | |
| Brevibacterium | lactofermentum | 21798 | | | | | | | |
| Brevibacterium | lactofermentum | 21799 | | | | | | | |
| Brevibacterium | lactofermentum | 21800 | | | | | | | |
| Brevibacterium | lactofermentum | 21801 | | | | | | | |
| Brevibacterium | lactofermentum | | | B11470 | | | | | |
| Brevibacterium | lactofermentum | | | B11471 | | | | | |
| Brevibacterium | lactofermentum | 21086 | | | | | | | |
| Brevibacterium | lactofermentum | 21420 | | | | | | | |
| Brevibacterium | lactofermentum | 21086 | | | | | | | |
| Brevibacterium | lactofermentum | 31269 | | | | | | | |
| Brevibacterium | linens | 9174 | | | | | | | |
| Brevibacterium | linens | 19391 | | | | | | | |
| Brevibacterium | linens | 8377 | | | | | | | |
| Brevibacterium | paraffinolyticum | | | | | 11160 | | | |
| Brevibacterium | spec. | | | | | | 717.7 3 | | |
| Brevibacterium | spec. | | | | | | 717.7 3 | | |
| Brevibacterium | spec. | 14604 | | | | | | | |
| Brevibacterium | spec. | 21860 | | | | | | | |
| Brevibacterium | spec. | 21864 | | | | | | | |
| Brevibacterium | spec. | 21865 | | | | | | | |
| Brevibacterium | spec. | 21866 | | | | | | | |
| Brevibacterium | spec. | 19240 | | | | | | | |
| Corynebacterium | acetoacidophilum | 21476 | | | | | | | |
| Corynebacterium | acetoacidophilum | 13870 | | | | | | | |
| Corynebacterium | acetoglutamicum | | | B11473 | | | | | |
| Corynebacterium | acetoglutamicum | | | B11475 | | | | | |
| Corynebacterium | acetoglutamicum | 15806 | | | | | | | |
| Corynebacterium | acetoglutamicum | 21491 | | | | | | | |
| Corynebacterium | acetoglutamicu | 31270 | | | | | | | |
| Corynebacterium | acetophilum | | | B3671 | | | | | |
| Corynebacterium | ammoniagenes | 6872 | | | | | | 2399 | |
| Corynebacterium | ammoniagenes | 15511 | | | | | | | |
| Corynebacterium | fujiokense | 21496 | | | | | | | |
| Corynebacterium | glutamicum | 14067 | | | | | | | |
| Corynebacterium | glutamicum | 39137 | | | | | | | |
| Corynebacterium | glutamicum | 21254 | | | | | | | |
| Corynebacterium | glutamicum | 21255 | | | | | | | |
| Corynebacterium | glutamicum | 31830 | | | | | | | |
| Corynebacterium | glutamicum | 13032 | | | | | | | |
| Corynebacterium | glutamicum | 14305 | | | | | | | |
| Corynebacterium | glutamicum | 15455 | | | | | | | |
| Corynebacterium | glutamicum | 13058 | | | | | | | |
| Corynebacterium | glutamicum | 13059 | | | | | | | |
| Corynebacterium | glutamicum | 13060 | | | | | | | |
| Corynebacterium | glutamicum | 21492 | | | | | | | |
| Corynebacterium | glutamicum | 21513 | | | | | | | |
| Corynebacterium | glutamicum | 21526 | | | | | | | |
| Corynebacterium | glutamicum | 21543 | | | | | | | |
| Corynebacterium | glutamicum | 13287 | | | | | | | |
| Corynebacterium | glutamicum | 21851 | | | | | | | |
| Corynebacterium | glutamicum | 21253 | | | | | | | |
| Corynebacterium | glutamicum | 21514 | | | | | | | |
| Corynebacterium | glutamicum | 21516 | | | | | | | |
| Corynebacterium | glutamicum | 21299 | | | | | | | |
| Corynebacterium | glutamicum | 21300 | | | | | | | |
| Corynebacterium | glutamicum | 39684 | | | | | | | |
| Corynebacterium | glutamicum | 21488 | | | | | | | |
| Corynebacterium | glutamicum | 21649 | | | | | | | |
| Corynebacterium | glutamicum | 21650 | | | | | | | |
| Corynebacterium | glutamicum | 19223 | | | | | | | |
| Corynebacterium | glutamicum | 13869 | | | | | | | |
| Corynebacterium | glutamicum | 21157 | | | | | | | |
| Corynebacterium | glutamicum | 21158 | | | | | | | |
| Corynebacterium | glutamicum | 21159 | | | | | | | |
| Corynebacterium | glutamicum | 21355 | | | | | | | |
| Corynebacterium | glutamicum | 31808 | | | | | | | |
| Corynebacterium | glutamicum | 21674 | | | | | | | |
| Corynebacterium | glutamicum | 21562 | | | | | | | |
| Corynebacterium | glutamicum | 21563 | | | | | | | |
| Corynebacterium | glutamicum | 21564 | | | | | | | |
| Corynebacterium | glutamicum | 21565 | | | | | | | |
| Corynebacterium | glutamicum | 21566 | | | | | | | |
| Corynebacterium | glutamicum | 21567 | | | | | | | |
| Corynebacterium | glutamicum | 21568 | | | | | | | |
| Corynebacterium | glutamicum | 21569 | | | | | | | |
| Corynebacterium | glutamicum | 21570 | | | | | | | |
| Corynebacterium | glutamicum | 21571 | | | | | | | |
| Corynebacterium | glutamicum | 21572 | | | | | | | |
| Corynebacterium | glutamicum | 21573 | | | | | | | |
| Corynebacterium | glutamicum | 21579 | | | | | | | |
| Corynebacterium | glutamicum | 19049 | | | | | | | |
| Corynebacterium | glutamicum | 19050 | | | | | | | |
| Corynebacterium | glutamicum | 19051 | | | | | | | |
| Corynebacterium | glutamicum | 19052 | | | | | | | |
| Corynebacterium | glutamicum | 19053 | | | | | | | |
| Corynebacterium | glutamicum | 19054 | | | | | | | |
| Corynebacterium | glutamicum | 19055 | | | | | | | |
| Corynebacterium | glutamicum | 19056 | | | | | | | |
| Corynebacterium | glutamicum | 19057 | | | | | | | |
| Corynebacterium | glutamicum | 19058 | | | | | | | |
| Corynebacterium | glutamicum | 19059 | | | | | | | |
| Corynebacterium | glutamicum | 19060 | | | | | | | |
| Corynebacterium | glutamicum | 19185 | | | | | | | |
| Corynebacterium | glutamicum | 13286 | | | | | | | |
| Corynebacterium | glutamicum | 21515 | | | | | | | |
| Corynebacteriu m | glutamicum | 21527 | | | | | | | |
| Corynebacterium | glutamicum | 21544 | | | | | | | |
| Corynebacterium | glutamicum | 21492 | | | | | | | |
| Corynebacterium | glutamicum | | | B8183 | | | | | |
| Corynebacterium | glutamicum | | | B8182 | | | | | |
| Corynebacterium | glutamicum | | | B12416 | | | | | |
| Corynebacterium | glutamicum | | | B12417 | | | | | |
| Corynebacterium | glutamicum | | | 812418 | | | | | |
| Corynebacterium | glutamicum | | | B11476 | | | | | |
| Corynebacterium | glutamicum | 21608 | | | | | | | |
| Corynebacterium | lilium | | P973 | | | | | | |
| Corynebacterium | nitrilophilus | 21419 | | | | 11594 | | | |
| Corynebacterium | spec. | | P4445 | | | | | | |
| Corynebacterium | spec. | | P4446 | | | | | | |
| Corynebacterium | spec. | 31088 | | | | | | | |
| Corynebacterium | spec. | 31089 | | | | | | | |
| Corynebacterium | spec. | 31090 | | | | | | | |
| Corynebacterium | spec. | 31090 | | | | | | | |
| Corynebacterium | spec. | 31090 | | | | | | | |
| Corynebacterium | spec. | 15954 | | | | | | | 20145 |
| Corynebacterium | spec. | 21857 | | | | | | | |
| Corynebacterium | spec. | 21862 | | | | | | | |
| Corynebacterium | spec. | 21863 | | | | | | | |

Die Abkürzungen haben folgende Bedeutung:
ATCC: American Type Culture Collection, Rockville, MD, USA
FERM: Fermentation Research Institute, Chiba, Japan
NRRL: ARS Culture Collection, Northem Regional Research Laboratory, Peoria, IL, USA
CECT: Coleccion Espanola de Cultivos Tipo, Valencia, Spain
NCIMB: National Collection of Industrial and Marine Bacteria Ltd., Aberdeen, UK
CBS: Centraalbureau voor Schimmelcultures, Baarn, NL
NCTC: National Collection of Type Cultures, London, UK
DSMZ: Deutsche Sammlung von Mikroorganismen und Zellkulturen, Braunschweig, Germany

Durch die Nukleinsäuren mit Promotoraktivität gemäß Anspruch 1 ist es mit Hilfe der vorstehend beschriebenen, erfindungsgemäßen Verfahren möglich in den vorstehend beschriebenen, erfindungsgemäßen genetisch veränderten Mikroorganismen die Stoffwechselwege zu spezifischen biosynthetischen Produkten zu regulieren.

Dazu werden beispielsweise Stoffwechselwege die zu einem spezifischen biosynthetischen Produkt führen durch Verursachung oder Erhöhung der Transkriptionrate bzw. Expressionsrate von Genen dieses Biosyntheseweges verstärkt in dem die erhöhte Proteinmenge zu einer erhöhten Gesamtaktivität dieser Proteine des gewünschten Biosyntheseweges und damit zu einem verstärkten Stoffwechselfluß zu dem gewünschen biosynthetischen Produkt führt.

Weiterhin können Stoffwechselwege die von einem spezifischen biosynthetischen Produkt wegführen durch Reduzierung der Transkriptionrate bzw. Expressionsrate von Genen dieses wegführenden Biosyntheseweges abgeschwächt werden in dem die reduzierte Proteinmenge zu einer reduzierten Gesamtaktivität dieser Proteine des unerwünschten Biosyntheseweges und damit zusätzlich zu einem verstärkten Stoffwechselfluß zu dem gewünschen biosynthetischen Produkt führt.

Die erfindungsgemäßen genetisch veränderten Mikroorganismen sind beispielsweise in der Lage aus Glucose, Saccharose, Lactose, Fructose, Maltose, Melasse, Stärke, Cellulose oder aus Glycerin und Ethanol biosynthetische Produkte herzustellen.

Je nach gewünschtem biosynthetischen Produkt muss die Transkriptionsrate verschiedener Gene erhöht bzw. reduziert werden. In der Regel ist es vorteilhaft die Transkrioptionsrate mehrere Gene zu verändern, d.h. die Transkrioptionsrate einer Kombination von Gene zu Erhöhen und/oder die Transkrioptionsrate einer Kombination von Gene zu reduzieren.

In den erfindungsgemäßen genetisch veränderten Mikroorganismen ist mindestens eine veränderte, dass heißt erhöhte oder reduzierte Transkriptionsrate eines Gens auf eine Nukleinsäure mit Promotoraktivität gemäß Anspruch 1 zurückzuführen.

Weitere, zusätzliche veränderte, d.h. zusätzlich erhöhte oder zusätzlich reduzierte Transkriptionsraten von weiteren Genen im genetisch veränderten Mikroorganismus können, müssen aber nicht auf die Nukleinsäuren mit Promotoraktivität gemäß Anspruch 1 zurück gehen.

Die erfindungsgemäßen, genetisch veränderten Mikroorganismen können in einem Verfahren zur Herstellung von biosynthetischen Produkten genutzt werden.

Bevorzugte biosynthetische Produkte sind Feinchemikalien.

Der Begriff "Feinchemikalie" ist im Fachgebiet bekannt und beinhaltet Verbidnungen, die von einem Organismus produziert werden und in verschiedenen Industriezweigen Anwendungen finden, wie bspw., jedoch nicht beschränkt auf die pharmazeutische Industrie, die Landwirtschafts-, Kosmetik , Food und Feed-Industrie. Diese Verbindungen umfassen organische Säuren, wie beispielsweise Weinsäure, Itaconsäure und Diaminopimelinsäure, sowohl proteinogene als auch nicht-proteinogene Aminosäuren, Purin- und Pyrimidinbasen, Nukleoside und Nukleotide (wie bspw. beschrieben in Kuninaka, A. (1996) Nucleotides and related compounds, S. 561-612, in Biotechnology Bd. 6, Rehm et al., Hrsg. VCH: Weinheim und den darin enthaltenen Zitaten), Lipide, gesättigte und ungesättigte Fettsäuren (bspw. Arachidonsäure), Diole (bspw. Propandiol und Butandiol), Kohlenhydrate (bspw. Hyaluronsäure und Trehalose), aromatische Verbindungen (bspw. aromatische Amine, Vanillin und Indigo), Vitamine und Cofaktoren (wie beschrieben in Ullmann's Encyclopedia of Industrial Chemistry, Bd. A27, "Vitamins", S. 443-613 (1996) VCH: Weinheim und den darin enthaltenen Zitaten; und Ong, A.S., Niki, E. und Packer, L. (1995) "Nutrition, Lipids, Health and Disease" Proceedings of the UNESCO/Confederation of Scientific and Technological Associations in Malaysia and the Society for Free Radical Research - Asien, abgehalten am 1.-3. Sept. 1994 in Penang, Malysia, AOCS Press (1995)), Enzyme und sämtliche anderen von Gutcho (1983) in Chemicals by Fermentation, Noyes Data Corporation, ISBN: 0818805086 und den darin angegebenen Literaturstellen, beschriebenen Chemikalien. Der Metabolismus und die Verwendungen bestimmter Feinchemikalien sind nachstehend weiter erläutert.

### I. Aminosäure-Metabolismus und Verwendungen

Die Aminosäuren umfassen die grundlegenden Struktureinheiten sämtlicher Proteine und sind somit für die normalen Zellfunktionen essentiell. Der Begriff "Aminosäure" ist im Fachgebiet bekannt. Die proteinogenen Aminosäuren, von denen es 20 Arten gibt, dienen als Struktureinheiten für Proteine, in denen sie über Peptidbindungen miteinander verknüpft sind, wohingegen die nicht-proteinogenen Aminosäuren (von denen Hunderte bekannt sind) gewöhnlich nicht in Proteinen vorkommen (siehe Ullmann's Encyclopedia of Industrial Chemistry, Bd. A2, S. 57-97 VCH: Weinheim (1985)). Die Aminosäuren können in der D- oder L-Konfiguration vorliegen, obwohl L-Aminosäuren gewöhnlich der einzige Typ sind, den man in natürlich vorkommenden Proteinen vorfindet. Biosynthese- und Abbauwege von jeder der 20 proteinogenen Aminosäuren sind sowohl bei prokaryotischen als auch eukaryotischen Zellen gut charakterisiert (siehe bspw. Stryer, L. Biochemistry, 3. Auflage, S. 578-590 (1988)). Die "essentiellen" Aminosäuren (Histidin, Isoleucin, Leucin, Lysin, Methionin, Phenylalanin, Threonin, Tryptophan und Valin), so bezeichnet, da sie aufgrund der Komplexität ihrer Biosynthese mit der Ernährung aufgenommen werden müssen, werden durch einfache Biosyntheseswege in die übrigen 11 "nichtessentiellen" Aminosäuren (Alanin, Arginin, Asparagin, Aspartat, Cystein, Glutamat, Glutamin, Glycin, Prolin, Serin und Tyrosin) umgewandelt. Höhere Tiere besitzen die Fähigkeit, einige dieser Aminosäuren zu synthetisieren, jedoch müssen die essentiellen Aminosäuren mit der Nahrung aufgenommen werden, damit eine normale Proteinsynthese stattfindet.

Abgesehen von ihrer Funktion bei der Proteinbiosynthese sind diese Aminosäuren interessante Chemikalien an sich, und man hat entdeckt, daß viele bei verschiedenen Anwendungen in der Nahrungsmittel-, Futter-, Chemie-, Kosmetik-, Landwirtschafts- und pharmazeutischen Industrie zum Einsatz kommen. Lysin ist nicht nur für die Ernährung des Menschen eine wichtige Aminosäure, sondern auch für monogastrische Tiere, wie Geflügel und Schweine. Glutamat wird am häufigsten als Geschmacksadditiv (Mononatriumglutamat, MSG) sowie weithin in der Nahrungsmittelindustrie verwendet, wie auch Aspartat, Phenylalanin, Glycin und Cystein. Glycin, L-Methionin und Tryptophan werden sämtlich in der pharmazeutischen Industrie verwendet. Glutamin, Valin, Leucin, Isoleucin, Histidin, Arginin, Prolin, Serin und Alanin werden in der pharmazeutischen Industrie und der Kosmetikindustrie verwendet. Threonin, Tryptophan und D-/L-Methionin sind weitverbreitete Futtermittelzusätze (Leuchtenberger, W. (1996) Amino acids - technical production and use, S. 466-502 in Rehm et al., (Hrsg.) Biotechnology Bd. 6, Kapitel 14a, VCH: Weinheim). Man hat entdeckt, daß sich diese Aminosäuren außerdem als Vorstufen für die Synthese von synthetischen Aminosäuren und Proteinen, wie N-Acetylcystein, S-Carboxymethyl-L-cystein, (S)-5-Hydroxytryptophan und anderen, in Ullmann's Encyclopedia of Industrial Chemistry, Bd. A2, S. 57-97, VCH, Weinheim, 1985 beschriebenen Substanzen eignen.

Die Biosynthese dieser natürlichen Aminosäuren in Organismen, die sie produzieren können, bspw. Bakterien, ist gut charakterisiert worden (für einen Überblick der bakteriellen Aminosäure-Biosynthese und ihrer Regulation, s. Umbarger, H.E. (1978) Ann. Rev. Biochem. 47: 533 - 606). Glutamat wird durch reduktive Aminierung von - Ketoglutarat, einem Zwischenprodukt im Citronensäure-Zyklus, synthetisiert. Glutamin, Prolin und Arginin werden jeweils nacheinander aus Glutamat erzeugt. Die Biosynthese von Serin erfolgt in einem Dreischritt-Verfahren und beginnt mit 3-Phosphoglycerat (einem Zwischenprodukt bei der Glykolyse), und ergibt nach Oxidations-, Transaminierungs- und Hydrolyseschritten diese Aminosäure. Cystein und Glycin werden jeweils aus Serin produziert, und zwar die erstere durch Kondensation von Homocystein mit Serin, und die letztere durch Übertragung des Seitenketten- -Kohlenstoffatoms auf Tetrahydrofolat, in einer durch Serintranshydroxymethylase katalysierten Reaktion. Phenylalanin und Tyrosin werden aus den Vorstufen des Glycolyse- und Pentosephosphatweges, Erythrose-4-phosphat und Phosphoenolpyruvat in einem 9-Schritt-Biosyntheseweg synthetisiert, der sich nur in den letzten beiden Schritten nach der Synthese von Prephenat unterscheidet. Tryptophan wird ebenfalls aus diesen beiden Ausgangsmolekülen produziert, jedoch erfolgt dessen Synthese in einem 11-Schritt-Weg. Tyrosin läßt sich in einer durch Phenylalaninhydroxylase katalysierten Reaktion auch aus Phenylalanin herstellen. Alanin, Valin und Leucin sind jeweils Biosyntheseprodukte aus Pyruvat, dem Endprodukt der Glykolyse. Aspartat wird aus Oxalacetat, einem Zwischenprodukt des Citratzyklus, gebildet. Asparagin, Methionin, Threonin und Lysin werden jeweils durch Umwandlung von Aspartat produziert. Isoleucin wird aus Threonin gebildet. In einem komplexen 9-Schritt-Weg erfolgt die Bildung von Histidin aus 5-Phosphoribosyl-1-pyrophosphat, einem aktivierten Zucker.

Aminosäuren, deren Menge den Proteinbiosynthesebedarf der Zelle übersteigt, können nicht gespeichert werden, und werden stattdessen abgebaut, so daß Zwischenprodukte für die Haupt-Stoffwechselwege der Zelle bereitgestellt werden (für einen Überblick siehe Stryer, L., Biochemistry, 3. Aufl. Kap. 21 "Amino Acid Degradation and the Urea Cycle"; S 495-516 (1988)). Die Zelle ist zwar in der Lage, ungewünschte Aminosäuren in nützliche Stoffwechsel-Zwischenprodukte umzuwandeln, jedoch ist die Aminosäureproduktion hinsichtlich der Energie, der Vorstufenmoleküle und der für ihre Synthese nötigen Enzyme aufwendig. Es überrascht daher nicht, daß die Aminosäure-Biosynthese durch Feedback-Hemmung reguliert wird, wobei das Vorliegen einer bestimmten Aminosäure ihre eigene Produktion verlangsamt oder ganz beendet (für einen Überblick über den Rückkopplungs-Mechanismus bei Aminosäure-Biosynthesewegen, siehe Stryer, L., Biochemistry, 3. Aufl., Kap. 24, "Biosynthesis of Amino Acids and Heme", S. 575-600 (1988)). Der Ausstoß einer bestimmten Aminosäure wird daher durch die Menge dieser Aminosäure in der Zelle eingeschränkt.

### II. Vitamine, Cofaktoren und Nutrazeutika-Metabolismus sowie Verwendungen

Vitamine, Cofaktoren und Nutrazeutika umfassen eine weitere Gruppe von Molekülen. Höhere Tiere haben die Fähigkeit verloren, diese zu synthetisieren und müssen sie somit aufnehmen, obwohl sie leicht durch andere Organismen, wie Bakterien, synthetisiert werden. Diese Moleküle sind entweder biologisch aktive Moleküle an sich oder Vorstufen von biologisch aktiven Substanzen, die als Elektronenträger oder Zwischenprodukte bei einer Reihe von Stoffwechselwegen dienen. Diese Verbindungen haben neben ihrem Nährwert auch einen signifikanten industriellen Wert als Farbstoffe, Antioxidantien und Katalysatoren oder andere Verarbeitungs-Hilfsstoffe. (Für einen Überblick über die Struktur, Aktivität und die industriellen Anwendungen dieser Verbindungen siehe bspw. Ullmann's Encyclopedia of Industrial Chemistry, "Vitamins", Bd. A27, S. 443-613, VCH: Weinheim, 1996). Der Begriff "Vitamin" ist im Fachgebiet bekannt und umfaßt Nährstoffe, die von einem Organismus für eine normale Funktion benötigt werden, jedoch nicht von diesem Organismus selbst synthetisiert werden können. Die Gruppe der Vitamine kann Cofaktoren und nutrazeutische Verbindungen umfassen. Der Begriff "Cofaktor" umfaßt nicht-proteinartige Verbindungen, die für das Auftreten einer normalen Enzymaktivität nötig sind. Diese Verbindungen können organisch oder anorganisch sein; die erfindungsgemäßen Cofaktor-Moleküle sind vorzugsweise organisch. Der Begriff "Nutrazeutikum" umfaßt Nahrungsmittelzusätze, die bei Pflanzen und Tieren, insbesondere dem Menschen, gesundheitsfördernd sind. Beispiele solcher Moleküle sind Vitamine, Antioxidantien und ebenfalls bestimmte Lipide (z.B. mehrfach ungesättigte Fettsäuren).

Die Biosynthese dieser Moleküle in Organismen, die zu ihrer Produktion befähigt sind, wie Bakterien, ist umfassend charakterisiert worden (Ullmann's Encyclopedia of Industrial Chemistry, "Vitamins", Bd. A27, S. 443-613, VCH: Weinheim, 1996, Michal, G. (1999) Biochemical Pathways: An Atlas of Biochemistry and Molecular Biology, John Wiley & Sons: Ong, A.S., Niki, E. und Packer, L. (1995) "Nutrition, Lipids, Health and Disease" Proceedings of the UNESCO/Confederation of Scientific and Technological Associations in Malaysia and the Society for free Radical Research - Asien, abgehalten am 1.-3. Sept. 1994 in Penang, Malaysia, AOCS Press, Champaign, IL X, 374 S).

Thiamin (Vitamin B₁) wird durch chemisches Kuppeln von Pyrimidin und Thiazol-Einheiten gebildet. Riboflavin (Vitamin B₂) wird aus Guanosin-5'-triphosphat (GTP) und Ribose-5'-phosphat synthetisiert. Riboflavin wiederum wird zur Synthese von Flavinmononukleotid (FMN) und Flavinadenindinukleotid (FAD) eingesetzt. Die Familie von Verbindungen, die gemeinsam als "Vitamin B6" bezeichnet werden (bspw. Pyridoxin, Pyridoxamin, Pyridoxal-5'-phosphat und das kommerziell verwendete Pyridoxinhydrochlorid), sind alle Derivate der gemeinsamen Struktureinheit 5-Hydroxy-6-methylpyridin. Panthothenat (Pantothensäure, R-(+)-N-(2,4-Dihydroxy-3,3-dimethyl-1-oxobutyl)- -alanin) kann entweder durch chemische Synthese oder durch Fermentation hergestellt werden. Die letzten Schritte bei der Pantothenat-Biosynthese bestehen aus der ATP-getriebenen Kondensation von -Alanin und Pantoinsäure. Die für die Biosyntheseschritte für die Umwandlung in Pantoinsäure, in -Alanin und zur Kondensation in Pantothensäure verantwortlichen Enzyme sind bekannt. Die metabolisch aktive Form von Pantothenat ist Coenzym A, dessen Biosynthese über 5 enzymatische Schritte verläuft. Pantothenat, Pyridoxal-5'-phosphat, Cystein und ATP sind die Vorstufen von Coenzym A. Diese Enzyme katalysieren nicht nur die Bildung von Pantothenat, sondern auch die Produktion von (R)-Pantoinsäure, (R)-Pantolacton, (R)-Panthenol (Provitamin B₅), Pantethein (und seinen Derivaten) und Coenzym A.

Die Biosynthese von Biotin aus dem Vorstufenmolekül Pimeloyl-CoA in Mikroorganismen ist ausführlich untersucht worden, und man hat mehrere der beteiligten Gene identifiziert. Es hat sich herausgestellt, daß viele der entsprechenden Proteine an der Fe-Cluster-Synthese beteiligt sind und zu der Klasse der nifS-Proteine gehören. Die Liponsäure wird von der Octanonsäure abgeleitet und dient als Coenzym beim Energie-Metabolismus, wo sie Bestandteil des Pyruvatdehydrogenasekomplexes und des -Ketoglutaratdehydrogenasekomplexes wird. Die Folate sind eine Gruppe von Substanzen, die alle von der Folsäure abgeleitet werden, die wiederum von L-Glutaminsäure, p-Aminobenzoesäure und 6-Methylpterin hergeleitet ist. Die Biosynthese der Folsäure und ihrer Derivate, ausgehend von den metabolischen Stoffwechselzwischenprodukten Guanosin-5'-triphosphat (GTP), L-Glutaminsäure und p-Aminobenzoesäure ist in bestimmten Mikroorganismen eingehend untersucht worden.

Corrinoide (wie die Cobalamine und insbesondere Vitamin B₁₂) und die Porphyrine gehören zu einer Gruppe von Chemikalien, die sich durch ein Tetrapyrrol-Ringsystem auszeichnen. Die Biosynthese von Vitamin B₁₂ ist hinreichend komplex, daß sie noch nicht vollständig charakterisiert worden ist, jedoch ist inzwischen ein Großteil der beteiligten Enzyme und Substrate bekannt. Nikotinsäure (Nikotinat) und Nikotinamid sind Pyridin-Derivate, die auch als "Niacin" bezeichnet werden. Niacin ist die Vorstufe der wichtigen Coenzyme NAD (Nikotinamidadenindinukleotid) und NADP (Nikotinamidadenindinukleotidphosphat) und ihrer reduzierten Formen.

Die Produktion dieser Verbindungen im Großmaßstab beruht größtenteils auf zellfreien chemischen Synthesen, obwohl einige dieser Chemikalien ebenfalls durch großangelegte Anzucht von Mikroorganismen produziert worden sind, wie Riboflavin, Vitamin B₆, Pantothenat und Biotin. Nur Vitamin B₁₂ wird aufgrund der Komplexität seiner Synthese lediglich durch Fermentation produziert. In-vitro-Verfahren erfordern einen erheblichen Aufwand an Materialien und Zeit und häufig an hohen Kosten.

### III. Purin-, Pyrimidin-, Nukleosid- und Nukleotid-Metabolismus und Verwendungen

Gene für den Purin- und Pyrimidin-Stoffwechsel und ihre entsprechenden Proteine sind wichtige Ziele für die Therapie von Tumorerkrankungen und Virusinfektionen. Der Begriff "Purin" oder "Pyrimidin" umfaßt stickstoffhaltige Basen, die Bestandteil der Nukleinsäuren, Coenzyme und Nukleotide sind. Der Begriff "Nukleotid" beinhaltet die grundlegenden Struktureinheiten der Nukleinsäuremoleküle, die eine stickstoffhaltige Base, einen Pentose-Zucker (bei RNA ist der Zucker Ribose, bei DNA ist der Zucker D-Desoxyribose) und Phosphorsäure umfassen. Der Begriff "Nukleosid" umfaßt Moleküle, die als Vorstufen von Nukleotiden dienen, die aber im Gegensatz zu den Nukleotiden keine Phosphorsäureeinheit aufweisen. Durch Hemmen der Biosynthese dieser Moleküle oder ihrer Mobilisation zur Bildung von Nukleinsäuremolekülen ist es möglich, die RNA- und DNA-Synthese zu hemmen; wird diese Aktivität zielgerichtet bei kanzerogenen Zellen gehemmt, läßt sich die Teilungs- und Replikations-Fähigkeit von Tumorzellen hemmen.

Es gibt zudem Nukleotide, die keine Nukleinsäuremoleküle bilden, jedoch als Energiespeicher (d.h. AMP) oder als Coenzyme (d.h. FAD und NAD) dienen.

Mehrere Veröffentlichungen haben die Verwendung dieser Chemikalien für diese medizinischen Indikationen beschrieben, wobei der Purin- und/oder Pyrimidin-Metabolismus beeinflußt wird (bspw. Christopherson, R.I. und Lyons, S.D. (1990) "Potent inhibitors of de novo pyrimidine and purine biosynthesis as chemotherapeutic agents", Med. Res. Reviews 10: 505-548). Untersuchungen an Enzymen, die am Purin- und Pyrimidin-Metabolismus beteiligt sind, haben sich auf die Entwicklung neuer Medikamente konzentriert, die bspw. als Immunsuppressionsmittel oder Antiproliferantien verwendet werden können (Smith, J.L. "Enzymes in Nucleotide Synthesis" Curr. Opin. Struct. Biol. 5 (1995) 752-757; Biochem. Soc. Transact. 23 (1995) 877-902). Die Purin- und Pyrimidinbasen, Nukleoside und Nukleotide haben jedoch auch andere Einsatzmöglichkeiten: als Zwischenprodukte bei der Biosysnthese verschiedener Feinchemikalien (z.B. Thiamin, S-Adenosyl-methionin, Folate oder Riboflavin), als Energieträger für die Zelle (bspw. ATP oder GTP) und für Chemikalien selbst, werden gewöhnlich als Geschmacksverstärker verwendet (bspw. IMP oder GMP) oder für viele medizinische Anwendungen (siehe bspw. Kuninaka, A., (1996) "Nucleotides and Related Compounds in Biotechnology Bd. 6, Rehm et al., Hrsg. VCH: Weinheim, S. 561-612). Enzyme, die am Purin-, Pyrimidin-, Nukleosid- oder Nukleotid-Metabolismus beteiligt sind, dienen auch immer stärker als Ziele, gegen die Chemikalien für den Pflanzenschutz, einschließlich Fungiziden, Herbiziden und Insektiziden entwickelt werden.

Der Metabolismus dieser Verbindungen in Bakterien ist charakterisiert worden (für Übersichten siehe bspw. Zalkin, H. und Dixon, J.E. (1992) "De novo purin nucleotide biosynthesis" in Progress in Nucleic Acids Research and Molecular biology, Bd. 42, Academic Press, S. 259-287; und Michal, G. (1999) "Nucleotides and Nucleosides"; Kap. 8 in : Biochemical Pathways: An Atlas of Biochemistry and Molecular Biology, Wiley, New York). Der Purin-Metabolismus, das Objekt intesiver Forschung, ist für das normale Funktionieren der Zelle essentiell. Ein gestörter Purin-Metabolismus in höheren Tieren kann schwere Erkrankungen verursachen, bspw. Gicht. Die Purinnukleotide werden über eine Reihe von Schritten über die Zwischenverbindung Inosin-5'-phosphat (IMP) aus Ribose-5-phosphat synthetisiert, was zur Produktion von Guanosin-5'-monophosphat (GMP) oder Adenosin-5'-monophosphat (AMP) führt, aus denen sich die als Nukleotide verwendeten Triphosphatformen leicht herstellen lassen. Diese Verbindungen werden auch als Energiespeicher verwendet, so daß ihr Abbau Energie für viele verschiedene biochemische Prozesse in der Zelle liefert. Die Pyrimidinbiosynthese erfolgt über die Bildung von Uridin-5'-monophosphat (UMP) aus Ribose-5-phosphat. UMP wiederum wird in Cytidin-5'-triphosphat (CTP) umgewandelt. Die Desoxyformen sämtlicher Nukleotide werden in einer Einschritt-Reduktionsreaktion aus der Diphosphat-Riboseform des Nukleotides zur Diphosphat-Desoxyriboseform des Nukleotides hergestellt. Nach der Phosphorylierung können diese Moleküle an der DNA-Synthese teilnehmen.

### IV.Trehalose-Metabolismus und Verwendungen

Trehalose besteht aus zwei Glucosemolekülen, die über , -1,1-Bindung miteinander verknüpft sind. Sie wird gewöhnlich in der Nahrungsmittelindustrie als Süßstoff, als Additiv für getrocknete oder gefrorene Nahrungsmittel sowie in Getränken verwendet. Sie wird jedoch auch in der pharmazeutischen Industrie, der Kosmetik- und Biotechnologie-Industrie angewendet (s. bspw. Nishimoto et al., (1998) US-Patent Nr. 5 759 610; Singer, M.A. und Lindquist, S. Trends Biotech. 16 (1998) 460-467; Paiva, C.L.A. und Panek, A.D. Biotech Ann. Rev. 2 (1996) 293-314; und Shiosaka, M. J. Japan 172 (1997) 97-102). Trehalose wird durch Enzyme von vielen Mikroorganismen produziert und auf natürliche Weise in das umgebende Medium abgegeben, aus dem sie durch im Fachgebiet bekannte Verfahren gewonnen werden kann.

Besonders bevorzugte biosynthetische Produkte sind ausgewählt aus der Gruppe organische Säuren, Proteine, Nukleotide und Nukleoside, sowohl proteinogene als auch nicht-proteinogene Aminosäuren, Lipide und Fettsäuren, Diole, Kohlehydrate, aromatische Verbindungen, Vitamine und Cofaktoren, Enzyme und Proteine.

Bevorzugte organische Säuren sind Weinsäure, Itaconsäure und Diaminopimelinsäure

Bevorzugte Nukleoside und Nukleotide sind beispielsweise beschrieben in Kuninaka, A. (1996) Nucleotides and related compounds, S. 561-612, in Biotechnology Bd. 6, Rehm et al., Hrsg. VCH: Weinheim und den darin enthaltenen Zitaten.

Bevorzugte biosynthetische Produkte sind weiterhin Lipide, gesättigte und ungesättigte Fettsäuren, wie beispielsweise Arachidonsäure, Diole wie beispielsweise Propandiol und Butandiol, Kohlenhydrate, wie beispielsweise Hyaluronsäure und Trehalose, aromatische Verbindungen, wie beispielsweise aromatische Amine, Vanillin und Indigo, Vitamine und Cofaktoren, wie beispielsweise beschrieben in Ullmann's Encyclopedia of Industrial Chemistry, Bd. A27, "Vitamins", S. 443-613 (1996) VCH: Weinheim und den darin enthaltenen Zitaten; und Ong, A.S., Niki, E. und Packer, L. (1995) "Nutrition, Lipids, Health and Disease" Proceedings of the UNESCO/Confederation of Scientific and Technological Associations in Malaysia and the Society for Free Radical Research - Asien, abgehalten am 1.-3. Sept. 1994 in Penang, Malysia, AOCS Press (1995)), Enzyme Polyketide (Cane et al. (1998) Science 282: 63-68), und sämtliche anderen von Gutcho (1983) in Chemicals by Fermentation, Noyes Data Corporation, ISBN: 0818805086 und den darin angegebenen Literaturstellen, beschriebenen Chemikalien.

Besonders bevorzugte biosynthetische Produkte sind Aminosäuren, besonders bevorzugt essentielle Aminosäuren, insbeondere L-Glycin, L-Alanin, L-leucin, L-Methionin, L-Phenylalanin, L-Tryptophan, L-Lysin, L-Glutamin, L-Glutaminsäure, L-Serin, L-Prolin, L-Valin, L-Isoleucin, L-Cystein, L-Tyrosin, L-Histidin, L-Arginin, L-Asparagin, L-Asparaginsäure und L-Threonin, L-Homoserin, insbesondere L-Lysin, L-Methionin und L-Threonin. Im folgenden wird unter unter einer Aminosäure, wie beispielsweise Lysin, Methionin und Threonin, sowohl jeweils die L- und die D-Form der Aminosäure, vorzugsweise die L-Form, also beispielsweise L-Lysin, L-Methionin und L-Threonin verstanden.

Im Verfahren zur Herstellung von biosynthetischen Produkten wird vorzugsweise dem Kultivierungsschritt der genetisch veränderten Mikroorganismen ein Isolieren von biosynthetischen Produkten aus den Mikroorganismen oder/oder aus der Fermentationsbrühe angeschlossen. Diese Schritte können gleichzeitig und/oder vor zugsweisie nach dem Kultivierungsschritt stattfinden.

Die erfindungsgemäßen genetisch veränderten Mikroorganismen können kontinuierlich oder diskontinuierlich im batch- Verfahren (Satzkultivierung) oder im fed batch (Zulauf verfahren) oder repeated fed batch Verfahren (repetitives Zulaufverfahren) zur Produktion von biosynthetischen Produkten, insbesondere L-Lysin, L-Methionin und L-Threonin, kultiviert werden. Eine Zusammenfassung über bekannte Kultivierungsmethoden ist im Lehrbuch von Chmiel (Bioprozeßtechnik 1. Einführung in die Bioverfahrenstechnik (Gustav Fischer Verlag, Stuttgart, 1991)) oder im Lehrbuch von Storhas (Bioreaktoren und periphere Einrichtungen (Vieweg Verlag, Braunschweig/Wiesbaden, 1994)) zu finden.

Das zu verwendende Kulturmedium hat in geeigneter Weise den Ansprüchen der jeweiligen Stämme zu genügen. Beschreibungen von Kulturmedien verschiedener Mikroorganismen sind im Handbuch "Manual of Methods für General Bacteriology" der American Society für Bacteriology (Washington D. C., USA, 1981) enthalten.

Diese einsetzbaren Medien umfassen gewöhnlich eine oder mehrere Kohlenstoffquellen, Stickstoffquellen, anorganische Salze, Vitamine und/oder Spurenelemente.

Bevorzugte Kohlenstoffquellen sind Zucker, wie Mono-, Di- oder Polysaccharide. Sehr gute Kohlenstoffquellen sind beispielsweise Glucose, Fructose, Mannose, Galactose, Ribose, Sorbose, Ribulose, Lactose, Maltose, Saccharose, Raffinose, Stärke oder Cellulose. Man kann Zucker auch über komplexe Verbindungen, wie Melassen, oder andere Nebenprodukte der Zucker-Raffinierung zu den Medien geben. Es kann auch vorteilhaft sein, Gemische verschiedener Kohlenstoffquellen zuzugeben. Andere mögliche Kohlenstoffquellen sind Öle und Fette wie z. B. Sojaöl. Sonnenblumenöl. Erdnußöl und Kokosfett, Fettsäuren wie z. B. Palmitinsäure, Stearinsäure oder Linolsäure, Alkohole wie z. B. Glycerin, Methanol oder Ethanol und organische Säuren wie z. B. Essigsäure oder Milchsäure.

Stickstoffquellen sind gewöhnlich organische oder anorganische Stickstoffverbindungen oder Materialien, die diese Verbindungen enthalten. Beispielhafte Stickstoffquellen umfassen Ammoniak-Gas oder Ammoniumsalze, wie Ammoniumsulfat, Ammoniumchlorid, Ammoniumphosphat, Ammoniumcarbonat oder Ammoniumnitrat, Nitrate, Harnstoff, Aminosäuren oder komplexe Stickstoffquellen, wie Maisquellwasser, Sojamehl, Sojaprotein, Hefeextrakt, Fleischextrakt und andere. Die Stickstoffquellen können einzeln oder als Mischung verwendet werden.

Anorganische Salzverbindungen, die in den Medien enthalten sein können, umfassen die Chlorid-, Phosphor oder Sulfatsalze von Calcium, Magnesium, Natrium, Kobalt, Molybdän, Kalium, Mangan, Zink, Kupfer und Eisen

Als Schwefelquelle für die Herstellung von Feinchemikalien, insbesondere von Methionin, können anorganische Verbindungen wie beispielsweise Sulfate, Sulfite, Dithionite, Tetrathionate, Thiosulfate, Sulfide aber auch organische Schwefelverbindungen, wie Mercaptane und Thiole, verwendet werden.

Als Phosphorquelle können Phosphorsäure, Kaliumdihydrogenphosphat oder Dikaliumhydrogenphosphat oder die entsprechenden Natrium haltigen Salze verwendet werden.

Chelatbildner können zum Medium gegeben werden, um die Metallionen in Lösung zu halten. Besonders geeignete Chelatbildner umfassen Dihydroxyphenole, wie Catechol oder Protocatechuat, oder organische Säuren, wie Citronensäure.

Die eingesetzten Fermentationsmedien enthalten üblicherweise auch andere Wachstumsfaktoren, wie Vitamine oder Wachstumsförderer, zu denen beispielsweise Biotin, Riboflavin, Thiamin, Folsäure, Nikotinsäure, Panthothenat und Pyridoxin gehören. Wachstumsfaktoren und Salze stammen häufig von komplexen Medienkomponenten, wie Hefeextrakt, Melassen, Maisquellwasser und dergleichen. Dem Kulturmedium können überdies geeignete Vorstufen zugesetzt werden. Die genaue Zusammensetzung der Medienverbindungen hängt stark vom jeweiligen Experiment ab und wird für jeden spezifischen Fall individuell entschieden. Information über die Medienoptimierung ist erhältlich aus dem Lehrbuch "Applied Microbiol. Physiology, A Practical Approach" (Hrsg. P.M. Rhodes, P.F. Stanbury, IRL Press (1997) S. 53-73, ISBN 0 19 963577 3). Wachstumsmedien lassen sich auch von kommerziellen Anbietern beziehen, wie Standard 1 (Merck) oder BHI (Brain heart infusion, DIFCO) und dergleichen.

Sämtliche Medienkomponenten werden, entweder durch Hitze (20 min bei 1,5 bar und 121°C) oder durch Sterilfiltration, sterilisiert. Die Komponenten können entweder zusammen oder nötigenfalls getrennt sterilisiert werden. Sämtliche Medienkomponenten können zu Beginn der Anzucht zugegen sein oder wahlfrei kontinuierlich oder chargenweise hinzugegeben werden.

Die Temperatur der Kultur liegt normalerweise zwischen 15°C und 45°C, vorzugsweise bei 25°C bis 40°C und kann während des Experimentes konstant gehalten oder verändert werden. Der pH-Wert des Mediums sollte im Bereich von 5 bis 8,5, vorzugsweise um 7,0 liegen. Der pH-Wert für die Anzucht läßt sich während der Anzucht durch Zugabe von basische Verbindungen wie Natriumhydroxid, Kaliumhydroxid, Ammoniak bzw. Ammoniakwasser oder saure Verbindungen wie Phosphorsäure oder Schwefelsäure kontrollieren. Zur Kontrolle der Schaumentwicklung können Antischaummittel, wie z. B. Fettsäurepolyglykolester, eingesetzt werden. Zur Aufrechterhaltung der Stabilität von Plasmiden können dem Medium geeignete selektiv wirkende Stoffe, wie z. B. Antibiotika, hinzugefügt werden. Um aerobe Bedingungen aufrechtzuerhalten, werden Sauerstoff oder Sauerstoff haltige Gasmischungen, wie z. B. Umgebungsluft, in die Kultur eingetragen. Die Temperatur der Kultur liegt normalerweise bei 20°C bis 45°C. Die Kultur wird solange fortgesetzt, bis sich ein Maximum des gewünschten Produktes gebildet hat. Dieses Ziel wird normalerweise innerhalb von 10 Stunden bis 160 Stunden erreicht.

Die so erhaltenen Fermentationsbrühen haben üblicherweise eine Trockenmasse von 7,5 bis 25 Gew.-%.

Vorteilhaft ist außerdem auch, wenn die Fermentation zumindest am Ende, insbesondere jedoch über mindestens 30% der Fermentationsdauer zuckerlimitiert gefahren wird. Das heißt, dass während dieser Zeit die Konzentration an verwertbarem Zucker im Fermentationsmedium auf 0 bis 3 g/l gehalten, beziehungsweise abgesenkt wird. Die Isolierung von biosynthetischen Produkten aus der Fermentationsbrühe und/oder den Mikroorganismen erfolgt in an sich bekannter Weise entsprechend den physikalisch-chemischen Eigenschaften des biosynthetischen Wertprodukts und den biosynthetischen Nebenprodukten.

Die Fermentationsbrühe kann anschließend beispielsweise weiterverarbeitet werden. Je nach Anforderung kann die Biomasse ganz oder teilweise durch Separationsmethoden, wie z. B. Zentrifugation, Filtration, Dekantieren oder einer Kombination dieser Methoden aus der Fermentationsbrühe entfernt oder vollständig in ihr belassen werden.

Anschließend kann die Fermentationsbrühe mit bekannten Methoden, wie z. B. mit Hilfe eines Rotationsverdampfers, Dünnschichtverdampfers, Fallfilmverdampfers, durch Umkehrosmose, oder durch Nanofiltration, eingedickt beziehungsweise aufkonzentriert werden. Diese aufkonzentrierte Fermentationsbrühe kann anschließend durch Gefriertrocknung, Sprühtrocknung, Sprühgranulation oder durch anderweitige Verfahren aufgearbeitet werden.

Es ist aber auch möglich die blosynthetischen Produkte, insbesonder L-Lysin, L-Methionin und L-Threonin, weiter aufzureinigen. Hierzu wird die produkthaltige Brühe nach dem Abtrennen der Biomasse einer Chromatographie mit einem geeigneten Harz unterworfen, wobei das gewünschte Produkt oder die Verunreinigungen ganz oder teilweise auf dem Chromatographieharz zurückgehalten werden. Diese Chromatographieschritte können nötigenfalls wiederholt werden, wobei die gleichen oder andere Chromatographieharze verwendet werden. Der Fachmann ist in der Auswahl der geeigneten Chromatographieharze und ihrer wirksamsten Anwendung bewandert. Das gereinigte Produkt kann durch Filtration oder Ultrafiltration konzentriert und bei einer Temperatur aufbewahrt werden, bei der die Stabilität des Produktes maximal ist.

Die biosynthetischen Produkte können in unterschiedlichen Formen anfallen, beispielsweise in Form ihrer Salze oder Ester.

Die Identität und Reinheit der isolierten Verbindung(en) kann durch Techniken des Standes der Technik bestimmt werden. Diese umfassen Hochleistungs-Flüssigkeitschromatographie (HPLC), spektroskopische Verfahren, Färbeverfahren, Dünnschichtchromatographie, NIRS, Enzymtest oder mikrobiologische Tests. Diese Analyseverfahren sind zusammengefaßt in: Patek et al. (1994) Appl. Environ. Microbiol. 60:133-140; Malakhova et al. (1996) Biotekhnologiya 11 27-32; und Schmidt et al. (1998) Bioprocess Engineer. 19:67-70. Ulmann's Encyclopedia of Industrial Chemistry (1996) Bd. A27, VCH: Weinheim, S. 89-90, S. 521-540, S. 540-547, S. 559-566, 575-581 und S. 581-587; Michal, G (1999) Biochemical Pathways: An Atlas of Biochemistry and Molecular Biology, John Wiley and Sons; Fallon, A. et al. (1987) Applications of HPLC in Biochemistry in: Laboratory Techniques in Biochemistry and Molecular Biology, Bd. 17.

Die Erfindung wird nun anhand der folgenden nicht-limitierenden Beispiele näher beschrieben:

### Beispiel 1

### Herstellung des Vektors pCLiK5MCS

Zunächst wurden Ampicillinresistenz und Replikationsursprung des Vektors pBR322 mit den Oligonukleotidprimern SEQ ID NO 5: und SEQ ID NO 6:mit Hilfe der Polymerase-Kettenreaktion (PCR) amplifiziert.
SEQ ID NO 5:
   5'-CCCGGGATCCGCTAGCGGCGCGCCGGCCGGCCCGGTGTGAAATACCGCACA G-3'
SEQ ID NO 6:
   5'-TCTAGACTCGAGCGGCCGCGGCCGGCCTTTAAATTGAAGACGAAAGGGCCTC G-3'

Neben den zu pBR322 komplementären Sequenzen, enthält der Oligonukleotidprimer SEQ ID NO 5: in 5'-3' Richtung die Schnittstellen für die Restriktionsendonukleasen SmaI, BamHI, NheI und AscI und der Oligonukleotidprimer SEQ ID NO 6: in 5'-3' Richtung die Schnittstellen für die Restriktionsendonukleasen XbaI, XhoI, NotI und Dral. Die PCR Reaktion wurde nach Standardmethode wie Innis et al. (PCR Protocols. A Guide to Methods and Applications, Academic Press (1990)) mit PfuTurbo Polymerase (Stratagene, La Jolla, USA) durchgeführt. Das erhaltene DNA Fragment mit einer Größe von ungefähr 2,1 kb wurde mit dem GFX™PCR, DNA and Gel Band Purification Kit (Amersham Pharmacia, Freiburg) nach Angaben des Herstellers gereinigt. Die stumpfen Enden des DNA-Fragmentes wurden mit dem Rapid DNA Ligation Kit (Roche Diagnostics, Mannheim) nach Angaben des Herstellers miteinander ligiert und der Ligationsansatz nach Standardmethoden wie in Sambrook et al. (Molecular Cloning. A Laboratory Manual, Cold Spring Harbor, beschrieben (1989)), in kompetente E.coli XL-1 Blue (Stratagene, La Jolla, USA) transformiert. Eine Selektion auf Plasmid tragende Zellen wurde durch das Ausplattieren auf Ampicillin (50µg/ml) haltigen LB Agar (Lennox, 1955, Virology, 1:190) erreicht.

Die Plasmid-DNA eines individuellen Klons wurde mit dem Qiaprep Spin Miniprep Kit (Qiagen, Hilden) nach Angaben des Herstellers isoliert und über Restriktionsverdaus überprüft. Das so erhaltene Plasmid erhält den Namen pCLiK1.

Ausgehend vom Plasmid pWLT1 (Liebl et a1.,1992) als Template für eine PCR Reaktion wurde mit den Oligonukleotidprimern SEQ ID NO:7 und SEQ ID NO:8 eine Kanamycin-Resistenzcassette amplifiziert.
SEQ ID NO:7:
   5'-GAGATCTAGACCCGGGGATCCGCTAGCGGGCTGCTAAAGGAAGCGGA-3'
SEQ 10 NO:8
   5'-GAGAGGCGCGCCGCTAGCGTGGGCGAAGAACTCCAGCA-3'

Neben den zu pWLT1 komplementären Sequenzen, enthält der Oligonukleotidprimer SEQ ID NO: 7 in 5'-3' Richtung die Schnittstellen für die Restriktionsendonukleasen XbaI, SmaI, BamHI, NheI und der Oligonukleotidprimer SEQ ID NO:8 in 5'-3' Richtung die Schnittstellen für die Restriktionsendonukleasen AscI und NheI. Die PCR Reaktion wurde nach Standardmethode wie Innis et al. (PCR Protocols. A Guide to Methods and Applications, Academic Press (1990)) mit PfuTurbo Polymerase (Stratagene, La Jolla, USA) durchgeführt. Das erhaltene DNA Fragment mit einer Größe von ungefähr 1,3 kb wurde mit dem GFX™PCR, DNA and Gel Band Purification Kit (Amersham Pharmacia, Freiburg) nach Angaben des Herstellers gereinigt. Das DNA-Fragment wurde mit den Restriktionsendonukleasen XbaI und AscI (New England Biolabs, Beverly, USA) geschnitten und im Anschluß daran erneut mit dem GFX™PCR, DNA and Gel Band Purification Kit (Amersham Pharmacia, Freiburg) nach Angaben des Herstellers gereinigt. Der Vektor pCLiK1 wurde ebenfalls mit den Restriktionsendonukleasen XbaI und AscI geschnitten und mit alkalischer Phosphatase (1 (Roche Diagnostics, Mannheim)) nach Angaben des Herstellers dephosphoryliert. Nach Elektrophorese in einem 0,8%igen Agarosegel wurde der linearisierte Vektor (ca. 2,1 kb) mit dem GFX™PCR, DNA and Gel Band Purification Kit (Amersham Pharmacia, Freiburg) nach Angaben des Herstellers isoliert. Dieses Vektor-Fragment wurde mit Hilfe des Rapid DNA Ligation Kit (Roche Diagnostics, Mannheim) nach Angaben des Herstellers mit dem geschnittenen PCR Fragment ligiert und der Ligationsansatz nach Standardmethoden wie in Sambrook et al. (Molecular Cloning. A Laboratory Manual, Cold Spring Harbor, beschrieben(1989)), in kompetente E.coli XL-1 Blue (Stratagene, La Jolla, USA) transformiert. Eine Selektion auf Plasmid tragende Zellen wurde durch das Ausplattieren auf Ampicillin (50µg/ml) und Kanamycin (20µg/ml) haltigen LB Agar (Lennox, 1955, Virology, 1:190) erreicht.

Die Plasmid-DNA eines individuellen Klons wurde mit dem Qiaprep Spin Miniprep Kit (Qiagen, Hilden) nach Angaben des Herstellers isoliert und über Restriktionsverdaus überprüft. Das so erhaltene Plasmid erhält den Namen pCLiK2.

Der Vektor pCLiK2 wurde mit der Restriktionsendonuklease Dral (New England Biolabs, Beverly, USA) geschnitten. Nach Elektrophorese in einem 0,8%igen Agarosegel wurde ein ca. 2,3 kb großes Vektorfragment mit dem GFX™PCR, DNA and Gel Band Purification Kit (Amersham Pharmacia, Freiburg) nach Angaben des Herstellers isoliert. Dieses Vektor-Fragment wurde mit Hilfe des Rapid DNA Ligation Kit (Roche Diagnostics, Mannheim) nach Angaben des Herstellers religiert und der Ligationsansatz nach Standardmethoden wie in Sambrook et al. (Molecular Cloning. A Laboratory Manual, Cold Spring Harbor, beschrieben(1989)), in kompetente E.coli XL-1 Blue (Stratagene, La Jolla, USA) transformiert. Eine Selektion auf Plasmid tragende Zellen wurde durch das Ausplattieren auf Kanamycin (20µg/ml) haltigen LB Agar (Lennox, 1955, Virology, 1:190) erreicht.

Die Plasmid-DNA eines individuellen Klons wurde mit dem Qiaprep Spin Miniprep Kit (Qiagen, Hilden) nach Angaben des Herstellers isoliert und über Restriktionsverdaus überprüft. Das so erhaltene Plasmid erhält den Namen pCLiK3.

Ausgehend vom Plasmid pWLQ2 (Liebl et al., 1992) als Template für eine PCR Reaktion wurde mit den Oligonukleotidprimern SEQ ID NO:9 und SEQ ID NO:10 der Replikationsursprung pHM1519 amplifiziert.
SEQ ID NO:9:
   5'-GAGAGGGCGGCCGCGCAAAGTCCCGCTTCGTGAA-3'
SEQ ID NO:10:
   5'-GAGAGGGCGGCCGCTCAAGTCGGTCAAGCCACGC-3'

Neben den zu pWLQ2 komplementären Sequenzen, enthalten die Oligonukleotidprimer SEQ ID NO:9 und SEI ID NO:10 Schnittstellen für die Restriktionsendonuklease NotI. Die PCR Reaktion wurde nach Standardmethode wie Innis et al. (PCR Protocols. A Guide to Methods and Applications, Academic Press (1990)) mit PfuTurbo Polymerase (Stratagene, La Jolla, USA) durchgeführt. Das erhaltene DNA Fragment mit einer Größe von ungefähr 2,7 kb wurde mit dem GFX™PCR, DNA and Gel Band Purification Kit (Amersham Pharmacia, Freiburg) nach Angaben des Herstellers gereinigt. Das DNA-Fragment wurde mit der Restriktionsendonuklease NotI (New England Biolabs, Beverly, USA) geschnitten und im Anschluß daran erneut mit dem GFX™PCR, DNA and Gel Band Purification Kit (Amersham Pharmacia, Freiburg) nach Angaben des Herstellers gereinigt. Der Vektor pCLiK3 wurde ebenfalls mit der Restriktionsendonuklease NotI geschnitten und mit alkalischer Phosphatase (1 (Roche Diagnostics, Mannheim)) nach Angaben des Herstellers dephosphoryliert. Nach Elektrophorese in einem 0,8%igen Agarosegel wurde der linearisierte Vektor (ca. 2,3kb) mit dem GFX™PCR, DNA and Gel Band Purification Kit (Amersham Pharmacia, Freiburg) nach Angaben des Herstellers isoliert. Dieses Vektor-Fragment wurde mit Hilfe des Rapid DNA Ligation Kit (Roche Diagnostics, Mannheim) nach Angaben des Herstellers mit dem geschnittenen PCR Fragment ligiert und der Ligationsansatz nach Standardmethoden wie in Sambrook et al. (Molecular Cloning. A Laboratory Manual, Cold Spring Harbor, beschrieben(1989)), in kompetente E.coli XL-1 Blue (Stratagene, La Jolla, USA) transformiert. Eine Selektion auf Plasmid tragende Zellen wurde durch das Ausplattieren auf Kanamycin (20µg/ml) haltigen LB Agar (Lennox, 1955, Virology, 1:190) erreicht.

Die Plasmid-DNA eines individuellen Klons wurde mit dem Qiaprep Spin Miniprep Kit (Qiagen, Hilden) nach Angaben des Herstellers isoliert und über Restriktionsverdaus überprüft. Das so erhaltene Plasmid erhält den Namen pCLiK5.

Für die Erweiterung von pCLiK5 um eine "multiple cloning site" (MCS) wurden die beide synthetischen, weitestgehend komplementären Oligonukleotide SEQ ID NO:11 und SEQ ID NO:12, die Schnittstellen für die Restriktionsendonukleasen Swal, XhoI, AatI, Apal, Asp718, MluI, NdeI, SpeI, EcoRV, SalI, ClaI, BamHI, XbaI und SmaI enthalten, durch gemeinsames erhitzen auf 95°C und langsames abkühlen zu einem doppelsträngigen DNA-Fragment vereinigt.
SEQ ID NO:11:
SEQ ID NO:12:

Der Vektor pCLiK5 wurde mit den Restriktionsendonuklease XhoI und BamHI (New England Biolabs, Beverly, USA) geschnitten und mit alkalischer Phosphatase (1 (Roche Diagnostics, Mannheim)) nach Angaben des Herstellers dephosphoryliert. Nach Elektrophorese in einem 0,8%igen Agarosegel wurde der linearisierte Vektor (ca. 5,0 kb) mit dem GFX™PCR, DNA and Gel Band Purification Kit (Amersham Pharmacia, Freiburg) nach Angaben des Herstellers isoliert. Dieses Vektor-Fragment wurde mit Hilfe des Rapid DNA Ligation Kit (Roche Diagnostics, Mannheim) nach Angaben des Herstellers mit dem synthetischen Doppelsträngigen DNA-Fragment ligiert und der Ligationsansatz nach Standardmethoden wie in Sambrook et al. (Molecular Cloning. A Laboratory Manual, Cold Spring Harbor, beschrieben(1989)), in kompetente E.coli XL-1Blue (Stratagene, La Jolla, USA) transformiert. Eine Selektion auf Plasmid tragende Zellen wurde durch das Ausplattieren auf Kanamycin (20µg/ml) haltigen LB Agar (Lennox, 1955, Virology, 1:190) erreicht.

Die Plasmid-DNA eines individuellen Klons wurde mit dem Qiaprep Spin Miniprep Kit (Qiagen, Hilden) nach Angaben des Herstellers isoliert und über Restriktionsverdaus überprüft. Das so erhaltene Plasmid erhält den Namen pCLiK5MCS.

Sequenzierungsreaktionen wurden nach Sanger et al. (1977) Proceedings of the National Academy of Sciences USA 74:5463-5467 durchgeführt. Die Sequenzierreaktionen wurden mittels ABI Prism 377 (PE Applied Biosystems, Weiterstadt) aufgetrennt und ausgewertet.

Das entstandene Plasmid pCLiK5MCS ist als SEQ ID NO:13 aufgeführt.

### Beispiel 2

### Herstellung des Plasmids P EF-TS metA

Chromosomale DNA aus C. glutamicum ATCC 13032 wurde nach Tauch et al. (1995) Plasmid 33:168-179 oder Eikmanns et al. (1994) Microbiology 140:1817-1828 präpariert. Mit den Oligonukleotidprimer BK 1849 SEQ ID NO 14 und BK 1862 SEQ ID NO 15 33, der chromosomalen DNA als Template und Pfu Turbo Polymerase (Fa. Stratagene) wurde mit Hilfe der Polymerase-Kettenreaktion (PCR) nach Standardmethoden, wie in Innis et al. (1990) PCR Protocols. A Guide to Methods and Applications, Academic Press beschrieben, das metA Gen, das für die Homoserin-O-Acetyltransferase kodiert, amplifiziert.
BK 1849 SEC ID NO 14
   5'- gtgtgtcgacttagatgtagaactcgatgtag -3'
   und
BK 1862 SEC ID NO 15
   5'-atgcccaccctcgcgcc -3'

Das erhaltene DNA Fragment von ca. 1134 bp Größe wurde mit dem GFX™PCR, DNA and Gel Band Purification Kit (Amersham Pharmacia, Freiburg) nach Angaben des Herstellers gereinigt.

Mit den Oligonukleotidprimer Haf 26 SEQ ID NO 16 und Haf 27 SEQ ID NO 17, der chromosomalen DNA als Template und Pfu Turbo Polymerase (Fa. Stratagene) wurde mit Hilfe der Polymerase-Kettenreaktion (PCR) nach Standardmethoden, wie in Innis et al. (1990) PCR Protocols. A Guide to Methods and Applications, Academic Press beschrieben, die Expressioneinheit des Gens (SEQ ID 2), das für den Elongationsfaktor TS kodiert, amplifiziert.
Haf 26 SEQ ID NO 16
   5'-gagaggatcccccccacgacaatggaac-3'
   und
Haf 27 SEO ID NO 17
   5'-cctgaaggcgcgagggtgggcattacggggcgatcctccttatg -3'

Das erhaltene DNA Fragment von ca. 195 bp Größe wurde mit dem GFX™PCR, DNA and Gel Band Purification Kit (Amersham Pharmacia, Freiburg) nach Angaben des Herstellers gereinigt.

Die Primer Haf 27 und BK 1862 enthalten eine überlappende Sequenz und sind an ihren 5'-Enden homolog zueinander.

Die oben erhaltenen PCR-Produkte wurden als Template für eine weitere PCR einsetzt, in der die Primer BK 1849 (SEQ. ID. NO. 14) und Haf 26 (SEQ. ID. NO. 16) genutzt wurden.

Mit diesem Ansatz konnte ein DNA-Fragment amplifiziert werden, das der erwarteten Größe von 1329 bp entsprach. Diese P EF-TS/metA-Fusion wurde dann über die Restriktionschnittstellen BamH1 und Sall in den Vektor pClik 5a MCS (SEQ. ID. NO. 13)kloniert.

Das Vektorfragment wurde zusammen mit dem PCR-Fragment mit Hilfe des Rapid DNA Ligation Kit (Roche Diagnostics, Mannheim) nach Angaben des Herstellers ligiert und der Ligationsansatz nach Standardmethoden wie in Sambrook et al. (Molecular Cloning. A Laboratory Manual, Cold Spring Harbor, beschrieben(1989)), in kompetente E.coli XL-1 Blue (Stratagene, La Jolla, USA) transformiert. Eine Selektion auf Plasmid tragende Zellen wurde durch das Ausplattieren auf Kanamycin (20µg/ml) haltigen LB Agar (Lennox, 1955, Virology, 1:190) erreicht.

Die Präparation der Plasmid DNA wurde nach Methoden und mit Materialien der Fa. Quiagen durchgeführt. Sequenzierungsreaktionen wurden nach Sanger et al. (1977) Proceedings of the National Academy of Sciences USA 74:5463-5467 durchgeführt. Die Sequenzierreaktionen wurden mittels ABI Prism 377 (PE Applied Biosystems, Weiterstadt) aufgetrennt und ausgewertet.

Das resultierende Plasmid wurde mit pClik 5a MCS P EF-TS metA (SEQ. ID. NO. 18) bezeichnet.

### Beispiel 3

### MetA-Aktivitäten

Der Stamm Corynebacterium glutamicum ATCC13032 wurde jeweils mit den Plasmiden pClikS MCS, pClik MCS EF-TS metA nach der beschriebenen Methode (Liebl, et al. (1989) FEMS Microbiology Letters 53:299-303) transformiert. Die Transformationsmischung wurde auf CM-Platten plattiert, die zusätzlich 20mg/l Kanamycin enthielten, um eine Selektion auf Plasmid-haltige Zellen zu erreichen. Erhaltene Kan-resistente Klone wurden gepickt und vereinzelt.

C. glutamicum Stämme, die eines dieser Plasmidkonstrukte enthielten, wurden in MMA-Medium ((40 g/l Saccharose, 20 g/l (NH₄)₂SO₄, 1 g/l KH₂PO₄, 1 g/l K₂HPO₄, 0,25g/l MgSO₄ x 7H₂O, 54 g Aces, 1 ml CaCl2 (10 g/l), 1 ml Protocatechoat (300 mg/10 ml), 1 ml Spurenelementelösung (10 g/l FeSO₄ x /H₂O,10 g/l MnSO₄ x H₂O, 2 g/l ZnSO₄ x 7 H₂O, 0,2 g/l CUSO₄, 0,02 g/l NiCl₂ x 6 H₂O),100 µg/l Vitamin B₁₂, 0,3 mg/l Thiamin, 1mM Leucin, 1 mg/l Pyridoxal HCI, 1 ml Biotin (100 mg/l), pH7,0) bei 30°C über Nacht angezogen. Die Zellen wurden bei 4°C abzentrifugiert und das zweimal mit kaltem Tris-HCI-Puffer (0,1 %, pH 8,0) gewaschen. Nach erneuter Zentrifugation wurden die Zellen in kalten Tris-HCI-Puffer (0,1%, pH 8,0) aufgenommen und eine OD₆₀₀ von 160 eingestellt. Für den Zellaufschluß wurden 1 ml dieser Zellsuspension in 2 ml Ribolyserröhrchen der Fa. Hybaid überführt und in einem Ribolyser der Fa. Hybaid bei einer Rotationseinstellung von 6,0 dreimal für jeweils 30 sec lysiert. Das Lysat wurde durch 30minütige Zentrifugation bei 15.000 rpm bei 4°C in einer Eppendorfzentrifuge geklärt und der Überstand in ein neues Eppendororfcup überführt. Der Proteingehalt wurde nach Bradford, M.M. (1976) Anal. Biochem. 72:248-254 bestimmt.

Die Messung der enzymatischen Aktivität von MetA wurde wie folgt durchgeführt. Die Reaktionsansätze von 1 ml enthielten 100 mM Kaliumphosphatpuffer (pH 7,5), 5mM MgC12, 100 µM Acetyl CoA, 5mM L-Homoserine, 500 µM DTNB (Ellmans Reagenz) und Zellextrakt. Der Test wurde durch Zugabe von dem jeweiligen Proteinlysat gestartet und bei Raumtemperatur inkubiert. Es wurde dann eine Kinetik bei 412 nm über 10 min aufgenommen.

Die Ergebnisse sind in Tabelle 4 gezeigt.

**Tabelle 4**

| Stamm | spezifische Aktivität [nMol/mg/min] |
|---|---|
| ATCC 13032 pClik5MCS | 12,6 |
| ATCC 13032 pClik5MCS P EF-TS metA | 2339,1 |

Die Aktivität von MetA konnte durch die Verwendung der heterologen Expressionseinheit P_{EF-TS} erheblich gesteigert werden.

## Patentansprüche

1. Verfahren zur Veränderung oder Verursachung der Transkriptionsrate von Genen in Mikroorganismen im Vergleich zum Wildtyp durch Regulation der Transkription von Genen im Mikroorganismus durch Nukleinsäuren mit Promotoraktivität, enthaltend
A) die Nukleinsäuresequenz SEQ.ID.NO. 1 oder
B) eine von dieser Sequenz durch Substitution, Insertion oder Deletion von Nukleotiden abgeleitete Sequenz, die eine Identität von mindestens 90 % auf Nukleinsäureebene mit der Sequenz SEQ.ID.NO. 1 aufweist oder
C) eine Nukleinsäuresequenz, die mit der Nukleinsäuresequenz SEQ.ID.NO. 1 unter stringenten Bedingungen hybridisiert,
wobei die Gene in Bezug auf die Nukleinsäuren mit Promotoraktivität heterolog sind.

2. Verfahren nach Anspruch 1, **dadurch gekennzeichnet, dass** die Regulation der Transkription von Genen im Mikroorganismus durch die Nukleinsäuren mit Promotoraktivität **dadurch** erreicht wird, dass man
b1) eine oder mehrere der Nukleinsäuren mit Promotoraktivität gemäss Anspruch 1, gegebenenfalls mit veränderter spezifischer Promotoraktivität, in das Genom des Mikroorganismus einbringt, so dass die Transkription eines oder mehrerer endogenen Gene unter der Kontrolle der eingebrachten Nukleinsäure mit Promotoraktivität erfolgt oder
b2) ein oder mehrere Gene in das Genom des Mikroorganismus einbringt, so dass die Transkription eines oder mehrerer der eingebrachten Gene unter der Kontrolle der endogenen Nukleinsäuren mit Promotoraktivität gemäss Anspruch 1, gegebenenfalls mit veränderter spezifischer Promotoraktivität, erfolgt oder
b3) ein oder mehrere Nukleinsäurekonstrukte, enthaltend eine Nukleinsäure mit Promotoraktivität gemäss Anspruch 1, gegebenenfalls mit veränderter spezifischer Promotoraktivität, und funktionell verknüpft eine oder mehrere zu transkribierende Nukleinsäuren in den Mikroorganismus einbringt.

3. Verfahren nach Anspruch 1 oder 2, **dadurch gekennzeichnet, dass** man zur Erhöhung oder Verursachung der Transkriptionsrate von Genen in Mikroorganismen im Vergleich zum Wildtyp
die Transkription von Genen im Mikroorganismus durch Nukleinsäuren mit Promotoraktivität gemäss Anspruch 1 reguliert, wobei die Gene in Bezug auf die Nukleinsäuren mit Promotoraktivität heterolog sind.

4. Verfahren nach Anspruch 3, **dadurch gekennzeichnet, dass** die Regulation der Transkription von Genen im Mikroorganismus durch Nukleinsäuren mit Promotoraktivität **dadurch** erreicht wird, dass man
bh1) eine oder mehrere der Nukleinsäuren mit Promotoraktivität gemäss Anspruch 1, gegebenenfalls mit erhöhter spezifischer Promotoraktivität, in das Genom des Mikroorganismus einbringt, so dass die Transkription eines oder mehrerer endogenen Gene unter der Kontrolle der eingebrachten Nukleinsäure mit Promotoraktivität erfolgt oder
bh2) ein oder mehrere Gene in das Genom des Mikroorganismus einbringt, so dass die Transkription eines oder mehrerer der eingebrachten Gene unter der Kontrolle der endogenen Nukleinsäuren mit Promotoraktivität gemäss Anspruch 1, gegebenenfalls mit erhöhter spezifischer Promotoraktivität, erfolgt oder
bh3) ein oder mehrere Nukleinsäurekonstrukte, enthaltend eine Nukleinsäure mit Promotoraktivität gemäss Anspruch 1, gegebenenfalls mit erhöhter spezifischer Promotoraktivität, und funktionell verknüpft eine oder mehrere zu transkribierende Nukleinsäuren in den Mikroorganismus einbringt.

5. Verfahren nach einem der Ansprüche 1 bis 4, **dadurch gekennzeichnet, dass** die Gene ausgewählt sind aus der Gruppe Nukleinsäuren kodierend ein Protein aus dem Biosyntheseweg von proteinogenen und nicht-proteinogenen Aminosäuren, Nukleinsäuren kodierend ein Protein aus dem Biosyntheseweg von Nukleotiden und Nukleosiden, Nukleinsäuren kodierend ein Protein aus dem Biosyntheseweg von organischen Säuren, Nukleinsäuren kodierend ein Protein aus dem Biosyntheseweg von Lipiden und Fettsäuren, Nukleinsäuren kodierend ein Protein aus dem Biosyntheseweg von Diolen, Nukleinsäuren kodierend ein Protein aus dem Biosyntheseweg von Kohlenhydraten, Nukleinsäuren kodierend ein Protein aus dem Biosyntheseweg von aromatischen Verbindungen, Nukleinsäuren kodierend ein Protein aus dem Biosyntheseweg von Vitaminen, Nukleinsäuren kodierend ein Protein aus dem Biosyntheseweg von Cofaktoren und Nukleinsäuren kodierend ein Protein aus dem Biosyntheseweg von Enzymen, wobei die Gene gegebenenfalls weitere Regulationselemente enthalten können.

6. Verfahren nach Anspruch 5, **dadurch gekennzeichnet, dass** die Proteine aus dem Biosyntheseweg von Aminosäuren ausgewählt sind aus der Gruppe Aspartatkinase, Aspartat-Semialdehyd-Dehydrogenase, Diaminopimelat-Dehydrogenase, Diaminopimelat-Decarboxylase, Dihydrodipicolinate-Synthetase, Dihydrodipicolinate-Reduktase, Glycerinaldehyd-3-Phosphat-Dehydrogenase, 3-Phosphoglycerat-Kinase, Pyruvat-Carboxylase, Triosephosphat-Isomerase, Transkriptioneller Regulater LuxR, Transkriptioneller Regulator LysR!, Transkriptioneller Regulator LysR2, Malat-Quinon-Oxireduktase, Glucose-6-Phosphat-Dehydrogenase, 6-Phosphogluconat-Dehydrogenase, Transketolase, Transaldolase, Homoserin-O-Acetyltransferase, Cystahionin-gamma-Synthase, Cystahionin-beta-Lyase, Serin-Hydroxymethyltransferase, O-Acetylhomoserin-Sulfhydrylase, Methylen-Tetrahydrofolat-Reduktase, Phosphoserin-Aminotransferase, Phosphoserin-Phosphatase, Serin-Acetyl-Transferase, Homoserin-Dehydrogenase, Homoserin-Kinase,Threonin-Synthase, Threonin-Exporter-Carrier, Threonin-Dehydratase, Pyruvat-Oxidase, Lysin-Exporter, Biotin-Ligase, Cystein-Synthase I, Cystein-Synthase II, Coenzym B12-abhängige Methionin-Synthase, Coenzym B12-unabhängige Methionin-Synthase, Sulfatadenyltransferase Untereinheit 1 und 2, Phosphoadenosin Phosphosulfat Reduktase, Ferredoxin-Sulfit-Reduktase, Ferredoxin NADP Reduktase, 3-Phosphoglycerat Dehydrogenase, RXA00655 Regulator, RXN2910-Regulator, Arginyl-t-RNA-Synthetase, Phosphoenolpyruvat-Carboxylase, Threonin Efflux-Protein, Serinhydroxymethyltransferase, Frukose-1,6-biphosphatase, Protein der Sulfat-Reduktion RXA077, Protein der Sulfat-Reduktion RXA248, Protein der Sulfat-Reduktion RXA247, Protein OpcA, 1-Phosphofruktokinase und 6-Phosphofruktokinase.

7. Genetisch veränderter Mikroorganismus, wobei die genetische Veränderung zu einer Veränderung oder Verursachung der Transkriptionsrate von mindestens einem Gen im Vergleich zum Wildtyp führt und bedingt ist durch Regulation der Transkription von Genen im Mikroorganismus durch Nukleinsäuren mit Promotoraktivität, enthaltend
A) die Nukleinsäuresequenz SEQ.ID.NO. 1 oder
B) eine von dieser Sequenz durch Substitution, Insertion oder Deletion von Nukleotiden abgeleitete Sequenz, die eine Identität von mindestens 90 % auf Nukleinsäureebene mit der Sequenz SEQ.ID.NO. 1 aufweist oder
C) eine Nukleinsäuresequenz, die mit der Nukleinsäuresequenz SEQ.ID.NO. 1 unter stringenten Bedingungen hybridisiert,
wobei die Gene in Bezug auf die Nukleinsäuren mit Promotoraktivität heterolog sind.

8. Genetisch veränderter Mikroorganismus nach Anspruch 7, **dadurch gekennzeichnet, dass** die Regulation der Transkription von Genen im Mikroorganismus durch Nukleinsäuren mit Promotoraktivität **dadurch** erreicht wird, dass man
b1) eine oder mehrere der Nukleinsäuren mit Promotoraktivität gemäss Anspruch 7, gegebenenfalls mit veränderter spezifischer Promotoraktivität, in das Genom des Mikroorganismus einbringt, so dass die Transkription eines oder mehrerer endogenen Gene unter der Kontrolle der eingebrachten Nukleinsäure mit Promotoraktivität erfolgt oder
b2) ein oder mehrere Gene in das Genom des Mikroorganismus einbringt, so dass die Transkription eines oder mehrerer der eingebrachten Gene unter der Kontrolle der endogenen Nukleinsäuren mit Promotoraktivität gemäss Anspruch 7, gegebenenfalls mit veränderter spezifischer Promotoraktivität, erfolgt oder
b3) ein oder mehrere Nukleinsäurekonstrukte, enthaltend eine Nukleinsäure mit Promotoraktivität gemäss Anspruch 7, gegebenenfalls mit veränderter spezifischer Promotoraktivität, und funktionell verknüpft eine oder mehrere zu transkribierende Nukleinsäuren in den Mikroorganismus einbringt.

9. Genetisch veränderter Mikroorganismus nach Anspruch 7 oder 8 mit erhöhter oder verursachter Transkriptionsrate von mindestens einem Gen im Vergleich zum Wildtyp, **dadurch gekennzeichnet, dass**
die Transkription von Genen im Mikroorganismus durch Nukleinsäuren mit Promotoraktivität gemäss Anspruch 7 reguliert wird, wobei die Gene in Bezug auf die Nukleinsäuren mit Promotoraktivität heterolog sind.

10. Genetisch veränderter Mikroorganismus nach Anspruch 9, **dadurch gekennzeichnet, dass** die Regulation der Transkription von Genen im Mikroorganismus durch die Nukleinsäuren mit Promotoraktivität **dadurch** erreicht wird, dass man
bh1) eine oder mehrere der Nukleinsäuren mit Promotoraktivität gemäss Anspruch 7, gegebenenfalls mit erhöhter spezifischer Promotoraktivität, in das Genom des Mikroorganismus einbringt, so dass die Transkription eines oder mehrerer endogenen Gene unter der Kontrolle der eingebrachten Nukleinsäure mit Promotoraktivität erfolgt oder
bh2) ein oder mehrere Gene in das Genom des Mikroorganismus einbringt, so dass die Transkription eines oder mehrerer der eingebrachten Gene unter der Kontrolle der endogenen Nukleinsäuren mit Promotoraktivität gemäss Anspruch 7, gegebenenfalls mit erhöhter spezifischer Promotoraktivität, erfolgt oder
bh3) ein oder mehrere Nukleinsäurekonstrukte, enthaltend eine Nukleinsäure mit Promotoraktivität gemäss Anspruch 7, gegebenenfalls mit erhöhter spezifischer Promotoraktivität, und funktionell verknüpft eine oder mehrere zu transkribierende Nukleinsäuren in den Mikroorganismus einbringt.

11. Genetisch veränderter Mikroorganismus nach einem der Ansprüche 7 bis 10, **dadurch gekennzeichnet, dass** die Gene ausgewählt sind aus der Gruppe Nukleinsäuren kodierend ein Protein aus dem Biosyntheseweg von proteinogenen und nicht-proteinogenen Aminosäuren, Nukleinsäuren kodierend ein Protein aus dem Biosyntheseweg von Nukleotiden und Nukleosiden, Nukleinsäuren kodierend ein Protein aus dem Biosyntheseweg von organischen Säuren, Nukleinsäuren kodierend ein Protein aus dem Biosyntheseweg von Lipiden und Fettsäuren, Nukleinsäuren kodierend ein Protein aus dem Biosyntheseweg von Diolen, Nukleinsäuren kodierend ein Protein aus dem Biosyntheseweg von Kohlenhydraten, Nukleinsäuren kodierend ein Protein aus dem Biosyntheseweg von aromatischen Verbindungen, Nukleinsäuren kodierend ein Protein aus dem Biosyntheseweg von Vitaminen, Nukleinsäuren kodierend ein Protein aus dem Biosyntheseweg von Cofaktoren und Nukleinsäuren kodierend ein Protein aus dem Biosyntheseweg von Enzymen, wobei die Gene gegebenenfalls weitere Regulationselemente enthalten können.

12. Genetisch veränderter Mikroorganismus gemäß Anspruch 11, **dadurch gekennzeichnet, dass** die Proteine aus dem Biosyntheseweg von Aminosäuren ausgewählt sind aus der Gruppe Aspartatkinase, Aspartat-Semialdehyd-Dehydrogenase, Diaminopimelat-Dehydrogenase, Diaminopimelat-Decarboxylase, Dihydrodipicolinate-Synthetase, Dihydrodipicolinate-Reduktase, Glycerinaldehyd-3-Phosphat-Dehydrogenase, 3-Phosphoglycerat-Kinase, Pyruvat-Carboxylase, Triosephosphat-Isomerase, Transkriptioneller Regulater LuxR, Transkriptioneller Regulator LysR!, Transkriptioneller Regulator LysR2, Malat-Quinon-Oxireduktase, Glucose-6-Phosphat-Dehydrogenase, 6-Phosphogluconat-Dehydrogenase, Transketolase, Transaldolase, Homoserin-O-Acetyltransferase, Cystahionin-gamma-Synthase, Cystahionin-beta-Lyase, Serin-Hydroxymethyltransferase, O-Acetylhomoserin-Sulfhydrylase, Methylen-Tetrahydrofolat-Reduktase, Phosphoserin-Aminotransferase, Phosphoserin-Phosphatase, Serin-Acetyl-Transferase, Homoserin-Dehydrogenase, Homoserin-Kinase,Threonin-Synthase, Threonin-Exporter-Carrier, Threonin-Dehydratase, Pyruvat-Oxidase, Lysin-Exporter, Biotin-Ligase, Cystein-Synthase I, Cystein-Synthase II, Coenzym B12-abhängige Methionin-Synthase, Coenzym B12-unabhängige Methionin-Synthase, Sulfatadenyltransferase Untereinheit 1 und 2, Phosphoadenosin Phosphosulfat Reduktase, Ferredoxin-Sulfit-Reduktase, Ferredoxin NADP Reduktase, 3-Phosphoglycerat Dehydrogenase, RXA00655 Regulator, RXN2910-Regulator, Arginyl-t-RNA-Synthetase, Phosphoenolpyruvat-Carboxylase, Threonin Efflux-Protein, Serinhydroxymethyltransferase, Frukose-1,6-biphosphatase, Protein der Sulfat-Reduktion RXA077, Protein der Sulfat-Reduktion RXA248, Protein der Sulfat-Reduktion RXA247, Protein OpcA, 1-Phosphofruktokinase und 6-Phosphofruktokinase.

## Claims

1. A method for altering or causing the transcription rate of genes in microorganisms compared with the wild type by having promoter activity, comprising
A) the nucleic acid sequence SEQ. ID. NO. 1 or
B) a sequence derived from this sequence by substitution, insertion or deletion of nucleotides and having an identity of at least 90% at the nucleic acid level with the sequence SEQ. ID. NO. 1, or
C) a nucleic acid sequence which hybridizes with the nucleic acid sequence SEQ. ID. NO. 1 under stringent conditions, or where the genes are heterologous in relation to the nucleic acids having promoter activity.
a) altering the specific promoter activity in the microorganism of endogenous nucleic acids having promoter activity according to claim 1, which regulate the transcription of endogenous genes, compared with the wild type or
b)regulating the transcription of genes in the microorganism by nucleic acids having promoter activity according to claim 1 or by nucleic acids having promoter activity according to claim 1 with altered specific promoter activity according to embodiment a), where the genes are heterologous in relation to the nucleic acids having promoter activity.

2. The method according to claim 1, wherein the regulation of the transcription of genes in the microorganism by the nucleic acids having promoter activity is achieved by
b1) introducing one or more of the nucleic acids having promoter activity according to claim 1, if appropriate with altered specific promoter activity, into the genome of the microorganism so that transcription of one or more endogenous genes takes place under the control of the introduced nucleic acid having promoter activity, or
b2) introducing one or more genes into the genome of the microorganism so that transcription of one or more of the introduced genes takes place under the control of the endogenous nucleic acids having promoter activity according to claim 1, if appropriate with altered specific promoter activity, or
b3) introducing one or more nucleic acid constructs comprising a nucleic acid having promoter activity according to claim 1, if appropriate with altered specific promoter activity, and functionally linked one or more nucleic acids to be transcribed, into the microorganism.

3. The method according to claim 1 or 2, wherein to increase or cause the transcription rate of genes in microorganisms compared with the wild type the transcription of genes in the microorganism is regulated by nucleic acids having promoter activity according to claim 1, where the genes are heterologous in relation to the nucleic acids having promoter activity.

4. The method according to claim 3, wherein the regulation of the transcription of genes in the microorganism by nucleic acids having promoter activity is achieved by
bh1) introducing one or more of the nucleic acids having promoter activity according to claim 1, if appropriate with increased specific promoter activity, into the genome of the microorganism so that transcription of one or more endogenous genes takes place under the control of the introduced nucleic acid having promoter activity, or
bh2) introducing one or more genes into the genome of the microorganism so that transcription of one or more of the introduced genes takes place under the control of the endogenous nucleic acids having promoter activity according to claim 1, if appropriate with increased specific promoter activity, or
bh3) introducing one or more nucleic acid constructs comprising a nucleic acid having promoter activity according to claim 1, if appropriate with increased specific promoter activity, and functionally linked one or more nucleic acids to be transcribed, into the microorganism.

5. The method according to any of claims 1 to 4, wherein the genes are selected from the group of nucleic acids encoding a protein from the biosynthetic pathway of proteinogenic and non-proteinogenic amino acids, nucleic acids encoding a protein from the biosynthetic pathway of nucleotides and nucleosides, nucleic acids encoding a protein from the biosynthetic pathway of organic acids, nucleic acids encoding a protein from the biosynthetic pathway of lipids and fatty acids, nucleic acids encoding a protein from the biosynthetic pathway of diols, nucleic acids encoding a protein from the biosynthetic pathway of carbohydrates, nucleic acids encoding a protein from the biosynthetic pathway of aromatic compounds, nucleic acids encoding a protein from the biosynthetic pathway of vitamins, nucleic acids encoding a protein from the biosynthetic pathway of cofactors and nucleic acids encoding a protein from the biosynthetic pathway of enzymes, where the genes may, if appropriate comprise further regulatory elements.

6. The method according to claim 5, wherein the proteins from the biosynthetic pathway of amino acids are selected from the group of aspartate kinase, aspartate-semialdehyde dehydrogenase, diaminopimelate dehydrogenase, diaminopimelate decarboxylase, dihydrodipicolinate synthetase, dihydrodipicolinate reductase, glyceraldehyde-3-phosphate dehydrogenase, 3-phosphoglycerate kinase, pyruvate carboxylase, triosephosphate isomerase, transcriptional regulator LuxR, transcriptional regulator LysR!, transcriptional regulator LysR2, malate-quinone oxireductase, glucose-6-phosphate dehydrogenase, 6-phosphogluconate dehydrogenase, transketolase, transaldolase, homoserine O-acetyltransferase, cystathionine gamma-synthase, cystathionine beta-lyase, serine hydroxymethyltransferase, O-acetylhomoserine sulfhydrylase, methylenetetrahydrofolate reductase, phosphoserine aminotransferase, phosphoseri_ne phosphatase, serine acetyltransferase, homoserine dehydrogenase, homoserine kinase, threonine synthase, threonine exporter carrier, threonine dehydratase, pyruvate oxidase, lysine exporter, biotin ligase, cysteine synthase I, cysteine synthase II, coenzyme B12-dependent methionine synthase, coenzyme B12-independent methionine synthase, sulfate adenylyltransferase subunit 1 and 2, phosphoadenosine-phosphosulfate reductase, ferredoxin-sulfite reductase, ferredoxin NADP reductase, 3-phosphoglycerate dehydrogenase, RXA00655 regulator, RXN2910 regulator, arginyl-tRNA synthetase, phosphoenolpyruvate carboxylase, threonine efflux protein, serine hydroxymethyltransferase, fructose-1,6-biphosphatase, protein of sulfate reduction RXA077, protein of sulfate reduction RXA248, protein of sulfate reduction RXA247, protein OpcA, 1-phosphofructokinase and 6-phosphofructokinase.

7. A genetically modified microorganism, where the genetic modification leads to an alteration or causing of the transcription rate of at least one gene compared with the wild type, and is dependent on regulating the transcription of genes in the microorganism by nucleic acids having promoter activity, comprising
A) the nucleic acid sequence SEQ. ID. NO. 1 or
B) a sequence derived from this sequence by substitution, insertion or deletion of nucleotides and having an identity of at least 90% at the nucleic acid level with the sequence SEQ. ID. NO. 1, or
C) a nucleic acid sequence which hybridizes with the nucleic acid sequence SEQ. ID. NO. 1 under stringent conditions.

8. The genetically modified microorganism according to claim 7, wherein the regulation of the transcription of genes in the microorganism by nucleic acids having promoter activity is achieved by
b1) introducing one or more of the nucleic acids having promoter activity according to claim 7, if appropriate with altered specific promoter activity, into the genome of the microorganism so that transcription of one or more endogenous genes takes place under the control of the introduced nucleic acid having promoter activity, or
b2) introducing one or more genes into the genome of the microorganism so that transcription of one or more of the introduced genes takes place under the control of the endogenous nucleic acids having promoter activity according to claim 7, if appropriate with altered specific promoter activity, or
b3) introducing one or more nucleic acid constructs comprising a nucleic acid having promoter activity according to claim 7, if appropriate with altered specific promoter activity, and functionally linked one or more nucleic acids to be transcribed, into the microorganism

9. The genetically modified microorganism according to claim 7 or 8 having increased or caused transcription rate of at least one gene compared with the wild type, wherein the transcription of genes in the microorganism is regulated by nucleic acids having promoter activity according to claim 7 where the genes are heterologous in relation to the nucleic acids having promoter activity.

10. The genetically modified microorganism according to claim 9, wherein the regulation of the transcription of genes in the microorganism by the nucleic acids having promoter activity is achieved by
bh1) introducing one or more of the nucleic acids having promoter activity according to claim 7, if appropriate with increased specific promoter activity, into the genome of the microorganism so that transcription of one or more endogenous genes takes place under the control of the introduced nucleic acid having promoter activity, or
bh2) introducing one or more genes into the genome of the microorganism so that transcription of one or more of the introduced genes takes place under the control of the endogenous nucleic acids having promoter activity according to claim 7, if appropriate with increased specific promoter activity, or
bh3) introducing one or more nucleic acid constructs comprising a nucleic acid having promoter activity according to claim 7, if appropriate with increased specific promoter activity, and functionally linked one or more nucleic acids to be transcribed, into the microorganism.

11. The genetically modified microorganism according to any of claims 7 to 10, wherein the genes are selected from the group of nucleic acids encoding a protein from the biosynthetic pathway of proteinogenic and non-proteinogenic amino acids, nucleic acids encoding a protein from the biosynthetic pathway of nucleotides and nucleosides, nucleic acid encoding a protein from the biosynthetic pathway of organic acids, nucleic acids encoding a protein from the biosynthetic pathway of lipids and fatty acids, nucleic acids encoding a protein from the biosynthetic pathway of diols, nucleic acids encoding a protein from the biosynthetic pathway of carbohydrates, nucleic acids encoding a protein from the biosynthetic pathway of aromatic compounds, nucleic acids encoding a protein from the biosynthetic pathway of vitamins, nucleic acids encoding a protein from the biosynthetic pathway of cofactors and nucleic acids encoding a protein from the biosynthetic pathway of enzymes, where the genes may, if appropriate comprise further regulatory elements.

12. The genetically modified microorganism according to claim 11, wherein the proteins from the biosynthetic pathway of amino acids are selected from the group of aspartate kinase, aspartate-semialdehyde dehydrogenase, diaminopimelate dehydrogenase, diaminopimelate decarboxylase, dihydrodipicolinate synthetase, dihydrodipicolinate reductase, glyceraldehyde-3-phosphate dehydrogenase, 3-phosphoglycerate kinase, pyruvate carboxylase, triosephosphate isomerase, transcriptional regulator LuxR, transcriptional regulator LysR!, transcriptional regulator LysR2, malate-quinone oxireductase, glucose-6-phosphate dehydrogenase, 6-phosphogluconate dehydrogenase, transketolase, transaldolase, homoserine O-acetyltransferase, cystathionine gamma-synthase, cystathionine beta-lyase, serine hydroxymethyltransferase, O-acetylhomoserine sulfhydrylase, methylenetetrahydrofolate reductase, phosphoserine aminotransferase, phosphoserine phosphatase, serine acetyltransferase, homoserine dehydrogenase, homoserine kinase, threonine synthase, threonine exporter carrier, threonine dehydratase, pyruvate oxidase, lysine exporter, biotin ligase, cysteine synthase I, cysteine synthase II, coenzyme B12-dependent methionine synthase, coenzyme B12-independent methionine synthase, sulfate adenylyltransferase subunit 1 and 2, phosphoadenosine-phosphosulfate reductase, ferredoxin-sulfite reductase, ferredoxin NADP reductase, 3-phosphoglycerate dehydrogenase, RXA00655 regulator, RXN2910 regulator, arginyl-tRNA synthetase, phosphoenolpyruvate carboxylase, threonine efflux protein, serine hyd-roxymethyltransferase, fructose-1,6-biphosphatase, protein of sulfate reduction RXA077, protein of sulfate reduction RXA248, protein of sulfate reduction RXA247, protein OpcA, 1-phosphofructokinase and 6-phosphofructokinase.

## Revendications

1. Procédé pour modifier ou créer le taux de transcription de gènes dans des micro-organismes par comparaison avec le type sauvage, par régulation de la transcription de gènes dans le micro-organisme par des acides nucléiques ayant une activité de promoteur, contenant
A) la séquence d'acide nucléique SEQ ID N° 1, ou
B) une séquence qui dérive de cette séquence par substitution, insertion ou délétion de nucléotides, qui présente une identité d'au moins 90 %, au niveau des acides nucléiques, avec la séquence SEQ ID N° 1, ou
C) une séquence d'acide nucléique qui s'hybride à la séquence d'acide nucléique SEQ ID N° 1 dans des conditions stringentes,
les gènes étant hétérologues pour ce qui est des acides nucléiques ayant une activité de promoteur.

2. Procédé selon la revendication 1, **caractérisé en ce que** la régulation de la transcription de gènes dans des micro-organismes par les acides nucléiques ayant une activité de promoteur est atteinte par le fait que
b1) on introduit un ou plusieurs des acides nucléiques ayant une activité de promoteur selon la revendication 1, ayant éventuellement une activité de promoteur spécifique modifiée, dans le génome du micro-organisme de telle sorte que la transcription d'un ou plusieurs gènes endogènes s'effectue sous le contrôle de l'acide nucléique introduit, ayant une activité de promoteur, ou
b2) on introduit un ou plusieurs gènes dans le génome du micro-organisme de telle sorte que la transcription d'un ou plusieurs des gènes introduits soit effectuée sous le contrôle des acides nucléiques endogènes ayant une activité de promoteur selon la revendication 1, ayant éventuellement une activité de promoteur spécifique modifiée, ou
b3) on introduit dans le micro-organisme une ou plusieurs constructions d'acides nucléiques, contenant un acide nucléique ayant une activité de promoteur selon la revendication 1, ayant éventuellement une activité de promoteur spécifique modifiée, et, fonctionnellement liés, un ou plusieurs des acides nucléiques à transcrire.

3. Procédé selon la revendication 1 ou 2, **caractérisé en ce que**, pour augmenter ou créer le taux de transcription de gènes dans des micro-organismes par comparaison avec le type sauvage, on régule la transcription de gènes dans le micro-organisme par des acides nucléiques ayant une activité de promoteur selon la revendication 1, les gènes étant hétérologues pour ce qui est des acides nucléiques ayant une activité de promoteur.

4. Procédé selon la revendication 3, **caractérisé en ce que** la régulation de la transcription de gènes dans le micro-organisme par des acides nucléiques ayant une activité de promoteur est réalisée par le fait que
bh1) on introduit dans le génome du micro-organisme un ou plusieurs des acides nucléiques ayant une activité de promoteur selon la revendication 1, ayant éventuellement une activité de promoteur spécifique augmentée, de telle sorte que la transcription d'un ou plusieurs gènes endogènes soit effectuée sous le contrôle de l'acide nucléique introduit ayant une activité de promoteur, ou
bh2) on introduit un ou plusieurs gènes dans le génome du micro-organisme, de telle sorte que la transcription d'un ou plusieurs des gènes introduits soit effectuée sous le contrôle des acides nucléiques endogènes ayant une activité de promoteur selon la revendication 1, ayant éventuellement une activité de promoteur spécifique augmentée, ou
bh3) on introduit dans le micro-organisme une ou plusieurs constructions d'acides nucléiques, contenant un acide nucléique ayant une activité de promoteur selon la revendication 1, ayant éventuellement une activité de promoteur spécifique augmentée, et, fonctionnellement liés, un ou plusieurs des acides nucléiques à transcrire.

5. Procédé selon l'une des revendications 1 à 4, **caractérisé en ce que** les gènes sont choisis dans le groupe consistant en les acides nucléiques codant pour une protéine de la voie de biosynthèse des acides aminés protéinogènes et non protéinogènes, les acides nucléiques codant pour une protéine de la voie de biosynthèse des nucléotides et des nucléosides, les acides nucléiques codant pour une protéine de la voie de biosynthèse des acides organiques, les acides nucléiques codant pour une protéine de la voie de biosynthèse des lipides et des acides gras, les acides nucléiques codant pour une protéine de la voie de biosynthèse des diols, les acides nucléiques codant pour une protéine de la voie de biosynthèse des hydrates de carbone, les acides nucléiques codant pour une protéine de la voie de biosynthèse des composés aromatiques, les acides nucléiques codant pour une protéine de la voie de biosynthèse des vitamines, les acides nucléiques codant pour une protéine de la voie de biosynthèse des co-facteurs, et les acides nucléiques codant pour une protéine de la biosynthèse des enzymes, les gènes pouvant éventuellement contenir des éléments régulateurs supplémentaires.

6. Procédé selon la revendication 5, **caractérisé en ce que** les protéines de la voie de biosynthèse des acides aminés sont choisies dans le groupe consistant en l'aspartatekinase, l'aspartate-semi-aldéhyde-déshydrogénase, la diaminopimélate-déshydrogénase, la diaminopimélate-décarboxylase, la dihydrodipicolinate-synthétase, la dihydrodipicolinate-réductase, la glycérolaldéhyde-3-phosphate-déshydrogénase, la 3-phosphoglycérate-kinase, la pyruvate-carboxylase, la triosophosphate-isomérase, le régulateur transcriptionnel LuxR, le régulateur transcriptionnel LysR!, le régulateur transcriptionnel LysR2, la malate-quinone-oxyréductase, la glucose-6-phosphate-déshydrogénase, la 6-phosphogluconate-déshydrogénase, la transkétolase, la transaldolase, l'homosérine-O-acétyltransférase, la cystahionine-gamma-synthase, la cystahionine-bêta-lyase, la sérine-hydroxyméthyltransférase, l'O-acétylhomosérine-sulfhydrylase, la méthylène-tétrahydrofolate-réductase, la phosphosérine-aminotransférase, la phosphosérine-phosphatase, la sérine-acétyl-transférase, l'homosérine-déshydrogénase, l'homosérine-kinase, la thréonine-synthase, l'exportateur-porteur de thréonine, la thrêonine-déshydratase, la pyruvate-oxydase, l'exportateur de lysine, la biotine-ligase, la cystéine-synthase I, la cystéine-synthase II, la méthionine-synthase coenzyme B12-dépendante, la méthionine-synthase coenzyme B12-indépendante, la sous-unité 1 et 2 de la sulfate-adényltransférase, la phosphoadénosine phosphosulfate réductase, la ferrédoxine-sulfite-réductase, la ferrédoxine NADP réductase, la 3-phosphoglycérate déshydrogénase, le régulateur RXA00655, le régulateur RXN2910, l'arginyl-ARNt-synthétase, la phosphoénolpyruvate-carboxylase, la protéine d'efflux de la thréonine, la sérine hydroxyméthyltransférase, la fructose-1,6-biphosphatase, la protéine de réduction des sulfates RXA077, la protéine de réduction des sulfates RXA248,
la protéine de réduction des sulfates RXA247, la protéine OpcA, la 1-phosphofructokinase et la 6-phosphofructokinase.

7. Micro-organisme génétiquement modifié, dans lequel la modification génétique conduit à une modification ou à une création du taux de transcription d'au moins un gène par comparaison au type sauvage, et est provoquée par une régulation de la transcription de gènes dans le micro-organisme par des acides nucléiques ayant une activité de promoteur, contenant
A) la séquence d'acide nucléique SEQ ID N° 1, ou
B) une séquence qui dérive de cette séquence par substitution, insertion ou délétion de nucléotides, qui présente une identité d'au moins 90 %, au niveau des acides nucléiques, avec la séquence SEQ ID N° 1, ou
C) une séquence d'acide nucléique qui s'hybride à la séquence d'acide nucléique SEQ ID N° 1 dans des conditions stringentes,
les gènes étant hétérologues pour ce qui est des acides nucléiques ayant une activité de promoteur.

8. Micro-organisme génétiquement modifié selon la revendication 7, **caractérisé en ce que** la régulation de la transcription de gènes dans des micro-organismes par les acides nucléiques ayant une activité de promoteur est atteinte par le fait que
b1) on introduit un ou plusieurs des acides nucléiques ayant une activité de promoteur selon la revendication 7, ayant éventuellement une activité de promoteur spécifique modifiée, dans le génome du micro-organisme de telle sorte que la transcription d'un ou plusieurs gènes endogènes s'effectue sous le contrôle de l'acide nucléique introduit, ayant une activité de promoteur, ou
b2) on introduit un ou plusieurs gènes dans le génome du micro-organisme de telle sorte que la transcription d'un ou plusieurs des gènes introduits soit effectuée sous le contrôle des acides nucléiques endogènes ayant une activité de promoteur selon la revendication 7, ayant éventuellement une activité de promoteur spécifique modifiée, ou
b3) on introduit dans le micro-organisme une ou plusieurs constructions d'acides nucléiques, contenant un acide nucléique ayant une activité de promoteur selon la revendication 7, ayant éventuellement une activité de promoteur spécifique modifiée, et, fonctionnellement liés, un ou plusieurs des acides nucléiques à transcrire.

9. Micro-organisme génétiquement modifié selon la revendication 7 ou 8, ayant un taux de transcription augmenté ou créé d' au moins un gène par comparaison au type sauvage, **caractérisé en ce que** la transcription de gènes dans le micro-organisme est régulée par des acides nucléiques ayant une activité de promoteur selon la revendication 7, les gènes étant hétérologues pour ce qui est des acides nucléiques ayant une activité de promoteur.

10. Micro-organisme génétiquement modifié selon la revendication 9, **caractérisé en ce que** la régulation de la transcription de gènes dans le micro-organisme par des acides nucléiques ayant une activité de promoteur est réalisée par le fait que
bh1) on introduit dans le génome du micro-organisme un ou plusieurs des acides nucléiques ayant une activité de promoteur selon la revendication 7, ayant éventuellement une activité de promoteur spécifique augmentée, de telle sorte que la transcription d'un ou plusieurs gènes endogènes soit effectuée sous le contrôle de l'acide nucléique introduit ayant une activité de promoteur, ou
bh2) on introduit un ou plusieurs gènes dans le génome du micro-organisme, de telle sorte que la transcription d'un ou plusieurs des gènes introduits soit effectuée sous le contrôle des acides nucléiques endogènes ayant une activité de promoteur selon la revendication 7, ayant éventuellement une activité de promoteur spécifique augmentée, ou
bh3) on introduit dans le micro-organisme une ou plusieurs constructions d'acides nucléiques, contenant un acide nucléique ayant une activité de promoteur selon la revendication 7, ayant éventuellement une activité de promoteur spécifique augmentée, et, fonctionnellement liés, un ou plusieurs des acides nucléiques à transcrire.

11. Micro-organisme génétiquement modifié selon l'une des revendications 7 à 10, **caractérisé en ce que** les gènes sont choisis dans le groupe consistant en les acides nucléiques codant pour une protéine de la voie de biosynthèse d'acides aminés protéinogènes et non protéinogènes, les acides nucléiques codant pour une protéine de la voie de biosynthèse des nucléotides et des nucléosides, les acides nucléiques codant pour une protéine de la voie de biosynthèse d'acides organiques, les acides nucléiques codant pour une protéine de la voie de biosynthèse des lipides et des acides gras, les acides nucléiques codant pour une protéine de la voie de biosynthèse des diols, les acides nucléiques codant pour une protéine de la voie de biosynthèse des hydrates de carbone, les acides nucléiques codant pour une protéine de la voie de biosynthèse des composés aromatiques, les acides nucléiques codant pour une protéine de la voie de biosynthèse des vitamines, les acides nucléiques codant pour une protéine de la voie de biosynthèse des co-facteurs, et les acides nucléiques codant pour une protéine de la biosynthèse des enzymes, les gènes pouvant éventuellement contenir des éléments régulateurs supplémentaires.

12. Micro-organisme génétiquement modifié selon la revendication 11, **caractérisé en ce que** les protéines de la voie de biosynthèse des acides aminés sont choisies dans le groupe consistant en l'aspartatekinase, l'aspartate-semi-aldéhyde-déshydrogénase, la diaminopimélate-déshydrogénase, la diaminopimélate-décarboxylase, la dihydrodipicolinate-synthétase, la dihydrodipicolinate-réductase, la glycérolaldéhyde-3-phosphate-déshydrogénase, la 3-phosphoglycérate-kinase, la pyruvate-carboxylase, la triosophosphate-isomé-rase, le régulateur transcriptionnel LuxR, le régulateur transcriptionnel LysR!, le régulateur transcriptionnel LysR2, la malate-quinone-oxyréductase, la glucose-6-phosphate-déshydrogénase, la 6-phosphogluconate-déshydrogénase, la transkétolase, la transaldolase, l'homosérine-0-acétyltransférase, la cystahionine-gamma-synthase, la cystahionine-bêta-lyase, la sérine-hydroxyméthyltransférase, l'O-acétylhomosérine-sulfhydrylase, la méthylène-tétrahydrofolate-réductase, la phosphosérine-aminotransférase, la phosphosérine-phosphatase, la sérine-acétyl-transférase, l'homosérine-déshydrogénase, l'homosérine-kinase, la thréonine-synthase, l'exportateuar-porteur de thréonine, la thréonine-déshydratase, la pyruvate-oxydase, l'exportateur de lysine, la biotine-ligase, la cystéine-synthase I, la cystéine-synthase II, la méthionine-synthase coenzyme B12-dépendante, la méthionine-synthase coenzyme B12-indépendante, la sous-unité 1 et 2 de la sulfate-adényltransférase, la phosphoadénosine phosphosulfate réductase, la ferrédoxine-sulfite-réductase, la ferrédoxine NADP réductase, la 3-phosphoglycérate déshydrogénase, le régulateur RXA00655, le régulateur RXN2910, l'arginyl-ARNt-synthétase, la phosphoénolpyruvate-carboxylase, la protéine d'efflux de la thréonine, la sérine hydroxyméthyltransférase, la fructose-1,6-biphosphatase, la protéine de réduction des sulfates RXA077, la protéine de réduction des sulfates RXA248,
la protéine de réduction des sulfates RXA247, la protéine OpcA, la 1-phosphofructokinase et la 6-phosphofructokinase.


### SEQUENCE LISTING

<110> BASF Aktiengesellschaft
<120> PEF-TS-Expressionseinheiten
<130> 20040509
<160> 20
<170> PatentIn version 3.1
<210> 1
   <211> 178
   <212> DNA
   <213> Corynebacterium glutamicum
<220>
   <221> Promotor
   <222> (1)..(178)
   <223>
<400> 1
   cccccacgac aatggaactt tgacttttaa aatttcatcg ccgtgggggc tttttgggca      60
   gccagcccgc cgtgtcgcaa cgtaatcgac tgaatacctg tacgatcact ttttagacgg     120
   gcgggtaggg ctactgtgcc ctaacctaag cttgtaaagc attaattatc catacata       178
<210> 2
   <211> 195
   <212> DNA
   <213> Corynebacterium glutamicum
<220>
   <221> misc_feature
   <222> (1)..(195)
   <223> Expressionseinheit
<400> 2
   cccccacgac aatggaactt tgacttttaa aatttcatcg ccgtgggggc tttttgggca      60
   gccagcccgc cgtgtcgcaa cgtaatcgac tgaatacctg tacgatcact ttttagacgg     120
   gcgggtaggg ctactgtgcc ctaacctaag cttgtaaagc attaattatc catacataag     180
   gaggatcgcc ccgta                                                      195
<210> 3
   <211> 1365
   <212> DNA
   <213> Corynebacterium glutamicum
<220>
   <221> CDS
   <222> (1)..(1365)
   <223>
<400> 3
   atg aat gat gag aat att caa agc tcc aac tat cag cca ttc ccg agt       48
   Met Asn Asp Glu Asn Ile Gln Ser Ser Asn Tyr Gln Pro Phe Pro Ser
   1               5                   10                  15
   ttt gac gat tgg aaa cag atc gag gtg tcg ctc tta gat gtc atc gaa       96
   Phe Asp Asp Trp Lys Gln Ile Glu Val Ser Leu Leu Asp Val Ile Glu
               20                  25                  30
   tcc tca cgc cat ttt tct gat ttg aaa gat agc act gat cgt tct gcg      144
   Ser Ser Arg His Phe Ser Asp Leu Lys Asp Ser Thr Asp Arg Ser Ala
           35                  40                  45
   tta gat gct gcg cta gag aga gca aaa aga gct gcc gca gtt gat acc      192
   Leu Asp Ala Ala Leu Glu Arg Ala Lys Arg Ala Ala Ala Val Asp Thr
       50                  55                  60
   aat gcc ata gaa gga atc ttc caa act gat cgc ggt ttt acc cat aca      240
   Asn Ala Ile Glu Gly Ile Phe Gln Thr Asp Arg Gly Phe Thr His Thr
   65                  70                  75                  80
   gtt gca acg cag gta ggg gct tgg gag caa caa atg gcg atg aaa ggc      288
   Val Ala Thr Gln Val Gly Ala Trp Glu Gln Gln Met Ala Met Lys Gly
                   85                  90                  95
   aaa cat gtt aag cct gcg ttt gac gat act cta gaa ggc ttt gag tat      336
   Lys His Val Lys Pro Ala Phe Asp Asp Thr Leu Glu Gly Phe Glu Tyr
               100                 105                 110
   gtt ctc gat gca gta act ggt aga act cca atc tct cag caa tgg att      384
   Val Leu Asp Ala Val Thr Gly Arg Thr Pro Ile Ser Gln Gln Trp Ile
           115                 120                 125
   aga aat ttg cac gcc gtc att ctg cgg agc caa gaa agc cac gag gtt      432
   Arg Asn Leu His Ala Val Ile Leu Arg Ser Gln Glu Ser His Glu Val
       130                 135                 140
   ttt aca gcc gtt gga gtc caa aat cag gcg ctt cag aaa ggc gag tat      480
   Phe Thr Ala Val Gly Val Gln Asn Gln Ala Leu Gln Lys Gly Glu Tyr
   145                 150                 155                 160
   aaa act cag cca aat agt cca cag cgc tca gat gga tct gta cat gca      528
   Lys Thr Gln Pro Asn Ser Pro Gln Arg Ser Asp Gly Ser Val His Ala
                   165                 170                 175
   tac gcc cca gtt gaa gat act cct gct gaa atg gct aga ttt att tca      576
   Tyr Ala Pro Val Glu Asp Thr Pro Ala Glu Met Ala Arg Phe Ile Ser
               180                 185                 190
   gaa ctt gaa tct aag gaa ttc tta gca gcc gag aag gtt att caa gct      624
   Glu Leu Glu Ser Lys Glu Phe Leu Ala Ala Glu Lys Val Ile Gln Ala
           195                 200                 205
   gcc tat gcc cac tat gct ttc gta tgt att cat cct ttt gca gat ggg      672
   Ala Tyr Ala His Tyr Ala Phe Val Cys Ile His Pro Phe Ala Asp Gly
       210                 215                 220
   aat gga cga gtt gca cga gcc ttg gct agt gtt ttt cta tac aaa gat      720
   Asn Gly Arg Val Ala Arg Ala Leu Ala Ser Val Phe Leu Tyr Lys Asp
   225                 230                 235                     240
   cct ggt gtc cct ctc gta atc tac caa gat caa cgc aga gat tac atc      768
   Pro Gly Val Pro Leu Val Ile Tyr Gln Asp Gln Arg Arg Asp Tyr Ile
                   245                 250                 255
   cat gct cta gaa gca gcg gac aag aat aac ccg ctc ctg ctg att aga      816
   His Ala Leu Glu Ala Ala Asp Lys Asn Asn Pro Leu Leu Leu Ile Arg
               260                 265                 270
   ttc ttt gct gaa cga gtg acc gat act att aac tct att atc gtt gat      864
   Phe Phe Ala Glu Arg Val Thr Asp Thr Ile Asn Ser Ile Ile Val Asp
           275                 280                 285
   ctc act acc ccg atc gcg ggt aaa tct ggt tcg gct aag ctt tcg gat      912
   Leu Thr Thr Pro Ile Ala Gly Lys Ser Gly Ser Ala Lys Leu Ser Asp
       290                 295                 300
   gcg cta cgc ccc act cgc gta tta cca gaa tta cat gat gct gca cat      960
   Ala Leu Arg Pro Thr Arg Val Leu Pro Glu Leu His Asp Ala Ala His
   305                 310                 315                     320
   agg ctc caa gaa agt tta ttt aca gaa atc cga tct cga ttg gat gaa     1008
   Arg Leu Gln Glu Ser Leu Phe Thr Glu Ile Arg Ser Arg Leu Asp Glu
                   325                 330                 335
   gaa gga aaa agg aat ggg ttg gag ttt cta ctt caa cgg att ttt atc     1056
   Glu Gly Lys Arg Asn Gly Leu Glu Phe Leu Leu Gln Arg Ile Phe Ile
               340                 345                 350
   ggt tcc cca ttc aat ctg cca gag ggc tat aac gct ttc cct gat agc     1104
   Gly Ser Pro Phe Asn Leu Pro Glu Gly Tyr Asn Ala Phe Pro Asp Ser
           355                 360                 365
   tat tgt ctg acc tta gct ttc aat agc aac tct cca aaa caa atc ttc     1152
   Tyr Cys Leu Thr Leu Ala Phe Asn Ser Asn Ser Pro Lys Gln Ile Phe
       370                 375                 380
   cac ccg cta tcc ata gta ata gca gct cga gat ggg aaa aga gcg agc     1200
   His Pro Leu Ser Ile Val Ile Ala Ala Arg Asp Gly Lys Arg Ala Ser
   385                 390                 395                     400
   agc gac ctc gtg gca gct act tct att gga tac aac ttt cac gct tac     1248
   Ser Asp Leu Val Ala Ala Thr Ser Ile Gly Tyr Asn Phe His Ala Tyr
                   405                 410                 415
   gga cgt gaa gtc gag cct gtt gtt act gaa agc ttt cga gaa cgt gtg     1296
   Gly Arg Glu Val Glu Pro Val Val Thr Glu Ser Phe Arg Glu Arg Val
               420                 425                 430
   aaa att tac gcc gac ggg att gta gat cac ttc tta acc gaa ctg gct     1344
   Lys Ile Tyr Ala Asp Gly Ile Val Asp His Phe Leu Thr Glu Leu Ala
           435                 440                 445
   aaa aag ttt caa cag aat taa                                         1365
   Lys Lys Phe Gln Gln Asn
       450
<210> 4
   <211> 454
   <212> PRT
   <213> Corynebacterium glutamicum
<400> 4
   Met Asn Asp Glu Asn Ile Gln Ser Ser Asn Tyr Gln Pro Phe Pro Ser
   1               5                   10                  15
   Phe Asp Asp Trp Lys Gln Ile Glu Val Ser Leu Leu Asp Val Ile Glu
               20                  25                  30
   Ser Ser Arg His Phe Ser Asp Leu Lys Asp Ser Thr Asp Arg Ser Ala
           35                  40                  45
   Leu Asp Ala Ala Leu Glu Arg Ala Lys Arg Ala Ala Ala Val Asp Thr
       50                  55                  60
   Asn Ala Ile Glu Gly Ile Phe Gln Thr Asp Arg Gly Phe Thr His Thr
   65                  70                  75                  80
   Val Ala Thr Gln Val Gly Ala Trp Glu Gln Gln Met Ala Met Lys Gly
                   85                  90                  95
   Lys His Val Lys Pro Ala Phe Asp Asp Thr Leu Glu Gly Phe Glu Tyr
               100                 105                 110
   Val Leu Asp Ala Val Thr Gly Arg Thr Pro Ile Ser Gln Gln Trp Ile
           115                 120                 125
   Arg Asn Leu His Ala Val Ile Leu Arg Ser Gln Glu Ser His Glu Val
       130                 135                 140
   Phe Thr Ala Val Gly Val Gln Asn Gln Ala Leu Gln Lys Gly Glu Tyr
   145                 150                 155                 160
   Lys Thr Gln Pro Asn Ser Pro Gln Arg Ser Asp Gly Ser Val His Ala
                   165                 170                 175
   Tyr Ala Pro Val Glu Asp Thr Pro Ala Glu Met Ala Arg Phe Ile Ser
               180                 185                 190
   Glu Leu Glu Ser Lys Glu Phe Leu Ala Ala Glu Lys Val Ile Gln Ala
           195                 200                 205
   Ala Tyr Ala His Tyr Ala Phe Val Cys Ile His Pro Phe Ala Asp Gly
       210                 215                 220
   Asn Gly Arg Val Ala Arg Ala Leu Ala Ser Val Phe Leu Tyr Lys Asp
   225                 230                 235                 240
   Pro Gly Val Pro Leu Val Ile Tyr Gln Asp Gln Arg Arg Asp Tyr Ile
                   245                 250                 255
   His Ala Leu Glu Ala Ala Asp Lys Asn Asn Pro Leu Leu Leu Ile Arg
               260                 265                 270
   Phe Phe Ala Glu Arg Val Thr Asp Thr Ile Asn Ser Ile Ile Val Asp
           275                 280                 285
   Leu Thr Thr Pro Ile Ala Gly Lys Ser Gly Ser Ala Lys Leu Ser Asp
   290 295 300
   Ala Leu Arg Pro Thr Arg Val Leu Pro Glu Leu His Asp Ala Ala His
   305 310 315 320
   Arg Leu Gln Glu Ser Leu Phe Thr Glu Ile Arg Ser Arg Leu Asp Glu
   325 330 335
   Glu Gly Lys Arg Asn Gly Leu Glu Phe Leu Leu Gln Arg Ile Phe Ile
   340 345 350
   Gly Ser Pro Phe Asn Leu Pro Glu Gly Tyr Asn Ala Phe Pro Asp Ser
   355 360 365
   Tyr Cys Leu Thr Leu Ala Phe Asn Ser Asn Ser Pro Lys Gln Ile Phe
   370 375 380
   His Pro Leu Ser Ile Val Ile Ala Ala Arg Asp Gly Lys Arg Ala Ser
   385 390 395 400
   Ser Asp Leu Val Ala Ala Thr Ser Ile Gly Tyr Asn Phe His Ala Tyr
   405 410 415
   Gly Arg Glu Val Glu Pro Val Val Thr Glu Ser Phe Arg Glu Arg Val
   420 425 430
   Lys Ile Tyr Ala Asp Gly Ile Val Asp His Phe Leu Thr Glu Leu Ala
   435 440 445
   Lys Lys Phe Gln Gln Asn
   450
<210> 5
   <211> 52
   <212> DNA
   <213> Künstliche Sequenz
<220>
   <221> Primer
   <222> (1)..(52)
   <223> Primer
<400> 5
   cccgggatcc gctagcggcg cgccggccgg cccggtgtga aataccgcac ag 52
<210> 6
   <211> 53
   <212> DNA
   <213> Künstliche Sequenz
<220>
   <221> Primer
   <222> (1)..(53)
   <223> Primer
<400> 6
   tctagactcg agcggccgcg gccggccttt aaattgaaga cgaaagggcc tcg 53
<210> 7
   <211> 47
   <212> DNA
   <213> Künstliche Sequenz
<220>
   <221> Primer
   <222> (1)..(47)
   <223> Primer
<400> 7
   gagatctaga cccggggatc cgctagcggg ctgctaaagg aagcgga 47
<210> 8
   <211> 38
   <212> DNA
   <213> Künstliche Sequenz
<220>
   <221> Primer
   <222> (1)..(38)
   <223> Primer
<400> 8
   gagaggcgcg ccgctagcgt gggcgaagaa ctccagca 38
<210> 9
   <211> 34
   <212> DNA
   <213> Künstliche Sequenz
<220>
   <221> Primer
   <222> (1)..(34)
   <223> Primer
<400> 9
   gagagggcgg ccgcgcaaag tcccgcttcg tgaa 34
<210> 10
   <211> 34
   <212> DNA
   <213> Künstliche sequenz
<220>
   <221> Primer
   <222> (1)..(34)
   <223> Primer
<400> 10
   gagagggcgg ccgctcaagt cggtcaagcc acgc 34
<210> 11
   <211> 140
   <212> DNA
   <213> Künstliche Sequenz
<220>
   <221> Primer
   <222> (1)..(140)
   <223> Primer
<400> 11
   tcgaatttaa atctcgagag gcctgacgtc gggcccggta ccacgcgtca tatgactagt     60
   tcggacctag ggatatcgtc gacatcgatg ctcttctgcg ttaattaaca attgggatcc    120
   tctagacccg ggatttaaat                                                140
<210> 12
   <211> 140
   <212> DNA
   <213> Künstliche Sequenz
<220>
   <221> Primer
   <222> (1)..(140)
   <223> Primer
<400> 12
   gatcatttaa atcccgggtc tagaggatcc caattgttaa ttaacgcaga agagcatcga     60
   tgtcgacgat atccctaggt ccgaactagt catatgacgc gtggtaccgg gcccgacgtc    120
   aggcctctcg agatttaaat                                                140
<210> 13
   <211> 5091
   <212> DNA
   <213> Künstliche Sequenz
<220>
   <221> misc_feature
   <222> (15091)..()
   <223> Plasmid
<400> 13
   tcgatttaaa tctcgagagg cctgacgtcg ggcccggtac cacgcgtcat atgactagtt 60
   cggacctagg gatatcgtcg acatcgatgc tcttctgcgt taattaacaa ttgggatcct 120
   ctagacccgg gatttaaatc gctagcgggc tgctaaagga agcggaacac gtagaaagcc 180
   agtccgcaga aacggtgctg accccggatg aatgtcagct actgggctat ctggacaagg 240
   gaaaacgcaa gcgcaaagag aaagcaggta gcttgcagtg ggcttacatg gcgatagcta 300
   gactgggcgg ttttatggac agcaagcgaa ccggaattgc cagctggggc gccctctggt 360
   aaggttggga agccctgcaa agtaaactgg atggctttct tgccgccaag gatctgatgg 420
   cgcaggggat caagatctga tcaagagaca ggatgaggat cgtttcgcat gattgaacaa 480
   gatggattgc acgcaggttc tccggccgct tgggtggaga ggctattcgg ctatgactgg 540
   gcacaacaga caatcggctg ctctgatgcc gccgtgttcc ggctgtcagc gcaggggcgc 600
   ccggttcttt ttgtcaagac cgacctgtcc ggtgccctga atgaactgca ggacgaggca 660
   gcgcggctat cgtggctggc cacgacgggc gttccttgcg cagctgtgct cgacgttgtc 720
   actgaagcgg gaagggactg gctgctattg ggcgaagtgc cggggcagga tctcctgtca 780
   tctcaccttg ctcctgccga gaaagtatcc atcatggctg atgcaatgcg gcggctgcat 840
   acgcttgatc cggctacctg cccattcgac caccaagcga aacatcgcat cgagcgagca 900
   cgtactcgga tggaagccgg tcttgtcgat caggatgatc tggacgaaga gcatcagggg 960
   ctcgcgccag ccgaactgtt cgccaggctc aaggcgcgca tgcccgacgg cgaggatctc 1020
   gtcgtgaccc atggcgatgc ctgcttgccg aatatcatgg tggaaaatgg ccgcttttct 1080
   ggattcatcg actgtggccg gctgggtgtg gcggaccgct atcaggacat agcgttggct 1140
   acccgtgata ttgctgaaga gcttggcggc gaatgggctg accgcttcct cgtgctttac 1200
   ggtatcgccg ctcccgattc gcagcgcatc gccttctatc gccttcttga cgagttcttc 1260
   tgagcgggac tctggggttc gaaatgaccg accaagcgac gcccaacctg ccatcacgag 1320
   atttcgattc caccgccgcc ttctatgaaa ggttgggctt cggaatcgtt ttccgggacg 1380
   ccggctggat gatcctccag cgcggggatc tcatgctgga gttcttcgcc cacgctagcg 1440
   gcgcgccggc cggcccggtg tgaaataccg cacagatgcg taaggagaaa ataccgcatc 1500
   aggcgctctt ccgcttcctc gctcactgac tcgctgcgct cggtcgttcg gctgcggcga 1560
   gcggtatcag ctcactcaaa ggcggtaata cggttatcca cagaatcagg ggataacgca 1620
   ggaaagaaca tgtgagcaaa aggccagcaa aaggccagga accgtaaaaa ggccgcgttg 1680
   ctggcgtttt tccataggct ccgcccccct gacgagcatc acaaaaatcg acgctcaagt 1740
   cagaggtggc gaaacccgac aggactataa agataccagg cgtttccccc tggaagctcc 1800
   ctcgtgcgct ctcctgttcc gaccctgccg cttaccggat acctgtccgc ctttctccct 1860
   tcgggaagcg tggcgctttc tcatagctca cgctgtaggt atctcagttc ggtgtaggtc 1920
   gttcgctcca agctgggctg tgtgcacgaa ccccccgttc agcccgaccg ctgcgcctta 1980
   tccggtaact atcgtcttga gtccaacccg gtaagacacg acttatcgcc actggcagca 2040
   gccactggta acaggattag cagagcgagg tatgtaggcg gtgctacaga gttcttgaag 2100
   tggtggccta actacggcta cactagaagg acagtatttg gtatctgcgc tctgctgaag 2160
   ccagttacct tcggaaaaag agttggtagc tcttgatccg gcaaacaaac caccgctggt 2220
   agcggtggtt tttttgtttg caagcagcag attacgcgca gaaaaaaagg atctcaagaa 2280
   gatcctttga tcttttctac ggggtctgac gctcagtgga acgaaaactc acgttaaggg 2340
   attttggtca tgagattatc aaaaaggatc ttcacctaga tccttttaaa ggccggccgc 2400
   ggccgcgcaa agtcccgctt cgtgaaaatt ttcgtgccgc gtgattttcc gccaaaaact 2460
   ttaacgaacg ttcgttataa tggtgtcatg accttcacga cgaagtacta aaattggccc 2520
   gaatcatcag ctatggatct ctctgatgtc gcgctggagt ccgacgcgct cgatgctgcc 2580
   gtcgatttaa aaacggtgat cggatttttc cgagctctcg atacgacgga cgcgccagca 2640
   tcacgagact gggccagtgc cgcgagcgac ctagaaactc tcgtggcgga tcttgaggag 2700
   ctggctgacg agctgcgtgc tcggccagcg ccaggaggac gcacagtagt ggaggatgca 2760
   atcagttgcg cctactgcgg tggcctgatt cctccccggc ctgacccgcg aggacggcgc 2820
   gcaaaatatt gctcagatgc gtgtcgtgcc gcagccagcc gcgagcgcgc caacaaacgc 2880
   cacgccgagg agctggaggc ggctaggtcg caaatggcgc tggaagtgcg tcccccgagc 2940
   gaaattttgg ccatggtcgt cacagagctg gaagcggcag cgagaattat cgcgatcgtg 3000
   gcggtgcccg caggcatgac aaacatcgta aatgccgcgt ttcgtgtgcc gtggccgccc 3060
   aggacgtgtc agcgccgcca ccacctgcac cgaatcggca gcagcgtcgc gcgtcgaaaa 3120
   agcgcacagg cggcaagaag cgataagctg cacgaatacc tgaaaaatgt tgaacgcccc 3180
   gtgagcggta actcacaggg cgtcggctaa cccccagtcc aaacctggga gaaagcgctc 3240
   aaaaatgact ctagcggatt cacgagacat tgacacaccg gcctggaaat tttccgctga 3300
   tctgttcgac acccatcccg agctcgcgct gcgatcacgt ggctggacga gcgaagaccg 3360
   ccgcgaattc ctcgctcacc tgggcagaga aaatttccag ggcagcaaga cccgcgactt 3420
   cgccagcgct tggatcaaag acccggacac ggagaaacac agccgaagtt ataccgagtt 3480
   ggttcaaaat cgcttgcccg gtgccagtat gttgctctga cgcacgcgca gcacgcagcc 3540
   gtgcttgtcc tggacattga tgtgccgagc caccaggccg gcgggaaaat cgagcacgta 3600
   aaccccgagg tctacgcgat tttggagcgc tgggcacgcc tggaaaaagc gccagcttgg 3660
   atcggcgtga atccactgag cgggaaatgc cagctcatct ggctcattga tccggtgtat 3720
   gccgcagcag gcatgagcag cccgaatatg cgcctgctgg ctgcaacgac cgaggaaatg 3780
   acccgcgttt tcggcgctga ccaggctttt tcacataggc tgagccgtgg ccactgcact 3840
   ctccgacgat cccagccgta ccgctggcat gcccagcaca atcgcgtgga tcgcctagct 3900
   gatcttatgg aggttgctcg catgatctca ggcacagaaa aacctaaaaa acgctatgag 3960
   caggagtttt ctagcggacg ggcacgtatc gaagcggcaa gaaaagccac tgcggaagca 4020
   aaagcacttg ccacgcttga agcaagcctg ccgagcgccg ctgaagcgtc tggagagctg 4080
   atcgacggcg tccgtgtcct ctggactgct ccagggcgtg ccgcccgtga tgagacggct 4140
   tttcgccacg ctttgactgt gggataccag ttaaaagcgg ctggtgagcg cctaaaagac 4200
   accaagggtc atcgagccta cgagcgtgcc tacaccgtcg ctcaggcggt cggaggaggc 4260
   cgtgagcctg atctgccgcc ggactgtgac cgccagacgg attggccgcg acgtgtgcgc 4320
   ggctacgtcg ctaaaggcca gccagtcgtc cctgctcgtc agacagagac gcagagccag 4380
   ccgaggcgaa aagctctggc cactatggga agacgtggcg gtaaaaaggc cgcagaacgc 4440
   tggaaagacc caaacagtga gtacgcccga gcacagcgag aaaaactagc taagtccagt 4500
   caacgacaag ctaggaaagc taaaggaaat cgcttgacca ttgcaggttg gtttatgact 4560
   gttgagggag agactggctc gtggccgaca atcaatgaag ctatgtctga atttagcgtg 4620
   tcacgtcaga ccgtgaatag agcacttaag gtctgcgggc attgaacttc cacgaggacg 4680
   ccgaaagctt cccagtaaat gtgccatctc gtaggcagaa aacggttccc ccgtagggtc 4740
   tctctcttgg cctcctttct aggtcgggct gattgctctt gaagctctct aggggggctc 4800
   acaccatagg cagataacgt tccccaccgg ctcgcctcgt aagcgcacaa ggactgctcc 4860
   caaagatctt caaagccact gccgcgactg ccttcgcgaa gccttgcccc gcggaaattt 4920
   cctccaccga gttcgtgcac acccctatgc caagcttctt tcaccctaaa ttcgagagat 4980
   tggattctta ccgtggaaat tcttcgcaaa aatcgtcccc tgatcgccct tgcgacgttg 5040
   gcgtcggtgc cgctggttgc gcttggcttg accgacttga tcagcggccg c 5091
<210> 14
   <211> 32
   <212> DNA
   <213> Künstliche Sequenz
<220>
   <221> Primer
   <222> (1)..(32)
   <223> Primer
<400> 14
   gtgtgtcgac ttagatgtag aactcgatgt ag 32
<210> 15
   <211> 17
   <212> DNA
   <213> Künstliche Sequenz
<220>
   <221> Primer
   <222> (1)..(17)
   <223> Primer
<400> 15
   atgcccaccc tcgcgcc 17
<210> 16
   <211> 28
   <212> DNA
   <213> Künstliche Sequenz
<220>
   <221> Primer
   <222> (1)..(28)
   <223> Primer
<400> 16
   gagaggatcc cccccacgac aatggaac 28
<210> 17
   <211> 44
   <212> DNA
   <213> Künstliche Sequenz
<220>
   <221> Primer
   <222> (1)..(44)
   <223> Primer
<400> 17 cctgaaggcg cgagggtggg cattacgggg cgatcctcct tatg 44
<210> 18
   <211> 6389
   <212> DNA
   <213> Künstliche Sequenz
<220>
   <221> misc_feature
   <222> (1)..(6389)
   <223> Plasmid
<400> 18
   tcgatttaaa tctcgagagg cctgacgtcg ggcccggtac cacgcgtcat atgactagtt 60
   cggacctagg gatatcgtcg acttagatgt agaactcgat gtaggtcgaa gggttgtctt 120
   cgtctgggga gatgaggctg aagaagttcc tcacgatgcg atccatttgg cggctttcgg 180
   tgaggaaagc atcgtggccg acaggggata cgatttttgc cattgccagt agatttccca 240
   ggtttctgga gaggtgttct tgctggtggt aggggtacaa aatatcggta tctacgcctg 300
   cgacaaggac tggaactttg atggattcga gtgccttgtt gaggcctccg cggtcgcgac 360
   caatgtcgtg gcggttgagg gcgtcggtga gcaagacgta ggagccggcg tcgaaacgct 420
   gtactagctt gtctgcttgg tagtccaagt aggattccac ggcgaagcgc tggtcgggct 480
   tgcggtaggg accgagtggg ttttcgttct tttgggcttt ggtgccgaag cgttcgtcga 540
   tttctagttc gccacggtag gtgaggtggg cgatgcgtcg ggcggcgccg agtccggtgg 600
   ctgggttgca gccggattcg tagtagttgc cttcgtgcca gtggtggtcg ttttcaatcg 660
   ccttaatttg ggcggattga atgccgattt gccaggcgct ggcgcgtgca gaaactgcaa 720
   gaacagcagc tgcgccaaca gtttctgggt acattgcggc ccactctagg gtgcgggcac 780
   cacccatgga accaccaagt actgcggcga ccgtggtgat gccgagtgcg tcgaggaatt 840
   gtttttcggc gtttacctga tcacgaatgg acgtggcggg gaagcgatta ccccagaaat 900
   ttccatctgg atgcatggag ccaggtccgg tggaaccgtt gcaaccaccg atgacgttgg 960
   tacagatcac gcagtaaata tcagtgttga tggctttgcc gggaccgagc aagtcagccc 1020
   accaatcggc tgcgttggaa tctccagtga gggcgtgttc gatgagaacg acattgctgc 1080
   gtccttcttt atctacgcgg tattcacccc agcggtgata ggcgatttca gcgtttgtaa 1140
   tgattgctcc ggcttcggtg gagacatcac cgatcgcttg gatttcaagt tgacctgaag 1200
   gcgcgagggt gggcattacg gggcgatcct ccttatgtat ggataattaa tgctttacaa 1260
   gcttaggtta gggcacagta gccctacccg cccgtctaaa aagtgatcgt acaggtattc 1320
   agtcgattac gttgcgacac ggcgggctgg ctgcccaaaa agcccccacg gcgatgaaat 1380
   tttaaaagtc aaagttccat tgtcgtgggg gggatcctct agacccggga tttaaatcgc 1440
   tagcgggctg ctaaaggaag cggaacacgt agaaagccag tccgcagaaa cggtgctgac 1500
   cccggatgaa tgtcagctac tgggctatct ggacaaggga aaacgcaagc gcaaagagaa 1560
   agcaggtagc ttgcagtggg cttacatggc gatagctaga ctgggcggtt ttatggacag 1620
   caagcgaacc ggaattgcca gctggggcgc cctctggtaa ggttgggaag ccctgcaaag 1680
   taaactggat ggctttcttg ccgccaagga tctgatggcg caggggatca agatctgatc 1740
   aagagacagg atgaggatcg tttcgcatga ttgaacaaga tggattgcac gcaggttctc 1800
   cggccgcttg ggtggagagg ctattcggct atgactgggc acaacagaca atcggctgct 1860
   ctgatgccgc cgtgttccgg ctgtcagcgc aggggcgccc ggttcttttt gtcaagaccg 1920
   acctgtccgg tgccctgaat gaactgcagg acgaggcagc gcggctatcg tggctggcca 1980
   cgacgggcgt tccttgcgca gctgtgctcg acgttgtcac tgaagcggga agggactggc 2040
   tgctattggg cgaagtgccg gggcaggatc tcctgtcatc tcaccttgct cctgccgaga 2100
   aagtatccat catggctgat gcaatgcggc ggctgcatac gcttgatccg gctacctgcc 2160
   cattcgacca ccaagcgaaa catcgcatcg agcgagcacg tactcggatg gaagccggtc 2220
   ttgtcgatca ggatgatctg gacgaagagc atcaggggct cgcgccagcc gaactgttcg 2280
   ccaggctcaa ggcgcgcatg cccgacggcg aggatctcgt cgtgacccat ggcgatgcct 2340
   gcttgccgaa tatcatggtg gaaaatggcc gcttttctgg attcatcgac tgtggccggc 2400
   tgggtgtggc ggaccgctat caggacatag cgttggctac ccgtgatatt gctgaagagc 2460
   ttggcggcga atgggctgac cgcttcctcg tgctttacgg tatcgccgct cccgattcgc 2520
   agcgcatcgc cttctatcgc cttcttgacg agttcttctg agcgggactc tggggttcga 2580
   aatgaccgac caagcgacgc ccaacctgcc atcacgagat ttcgattcca ccgccgcctt 2640
   ctatgaaagg ttgggcttcg gaatcgtttt ccgggacgcc ggctggatga tcctccagcg 2700
   cggggatctc atgctggagt tcttcgccca cgctagcggc gcgccggccg gcccggtgtg 2760
   aaataccgca cagatgcgta aggagaaaat accgcatcag gcgctcttcc gcttcctcgc 2820
   tcactgactc gctgcgctcg gtcgttcggc tgcggcgagc ggtatcagct cactcaaagg 2880
   cggtaatacg gttatccaca gaatcagggg ataacgcagg aaagaacatg tgagcaaaag 2940
   gccagcaaaa ggccaggaac cgtaaaaagg ccgcgttgct ggcgtttttc cataggctcc 3000
   gcccccctga cgagcatcac aaaaatcgac gctcaagtca gaggtggcga aacccgacag 3060
   gactataaag ataccaggcg tttccccctg gaagctccct cgtgcgctct cctgttccga 3120
   ccctgccgct taccggatac ctgtccgcct ttctcccttc gggaagcgtg gcgctttctc 3180
   atagctcacg ctgtaggtat ctcagttcgg tgtaggtcgt tcgctccaag ctgggctgtg 3240
   tgcacgaacc ccccgttcag cccgaccgct gcgccttatc cggtaactat cgtcttgagt 3300
   ccaacccggt aagacacgac ttatcgccac tggcagcagc cactggtaac aggattagca 3360
   gagcgaggta tgtaggcggt gctacagagt tcttgaagtg gtggcctaac tacggctaca 3420
   ctagaaggac agtatttggt atctgcgctc tgctgaagcc agttaccttc ggaaaaagag 3480
   ttggtagctc ttgatccggc aaacaaacca ccgctggtag cggtggtttt tttgtttgca 3540
   agcagcagat tacgcgcaga aaaaaaggat ctcaagaaga tcctttgatc ttttctacgg 3600
   ggtctgacgc tcagtggaac gaaaactcac gttaagggat tttggtcatg agattatcaa 3660
   aaaggatctt cacctagatc cttttaaagg ccggccgcgg ccgcgcaaag tcccgcttcg 3720
   tgaaaatttt cgtgccgcgt gattttccgc caaaaacttt aacgaacgtt cgttataatg 3780
   gtgtcatgac cttcacgacg aagtactaaa attggcccga atcatcagct atggatctct 3840
   ctgatgtcgc gctggagtcc gacgcgctcg atgctgccgt cgatttaaaa acggtgatcg 3900
   gatttttccg agctctcgat acgacggacg cgccagcatc acgagactgg gccagtgccg 3960
   cgagcgacct agaaactctc gtggcggatc ttgaggagct ggctgacgag ctgcgtgctc 4020
   ggccagcgcc aggaggacgc acagtagtgg aggatgcaat cagttgcgcc tactgcggtg 4080
   gcctgattcc tccccggcct gacccgcgag gacggcgcgc aaaatattgc tcagatgcgt 4140
   gtcgtgccgc agccagccgc gagcgcgcca acaaacgcca cgccgaggag ctggaggcgg 4200
   ctaggtcgca aatggcgctg gaagtgcgtc ccccgagcga aattttggcc atggtcgtca 4260
   cagagctgga agcggcagcg agaattatcg cgatcgtggc ggtgcccgca ggcatgacaa 4320
   acatcgtaaa tgccgcgttt cgtgtgccgt ggccgcccag gacgtgtcag cgccgccacc 4380
   acctgcaccg aatcggcagc agcgtcgcgc gtcgaaaaag cgcacaggcg gcaagaagcg 4440
   ataagctgca cgaatacctg aaaaatgttg aacgccccgt gagcggtaac tcacagggcg 4500
   tcggctaacc cccagtccaa acctgggaga aagcgctcaa aaatgactct agcggattca 4560
   cgagacattg acacaccggc ctggaaattt tccgctgatc tgttcgacac ccatcccgag 4620
   ctcgcgctgc gatcacgtgg ctggacgagc gaagaccgcc gcgaattcct cgctcacctg 4680
   ggcagagaaa atttccaggg cagcaagacc cgcgacttcg ccagcgcttg gatcaaagac 4740
   ccggacacgg agaaacacag ccgaagttat accgagttgg ttcaaaatcg cttgcccggt 4800
   gccagtatgt tgctctgacg cacgcgcagc acgcagccgt gcttgtcctg gacattgatg 4860
   tgccgagcca ccaggccggc gggaaaatcg agcacgtaaa ccccgaggtc tacgcgattt 4920
   tggagcgctg ggcacgcctg gaaaaagcgc cagcttggat cggcgtgaat ccactgagcg 4980
   ggaaatgcca gctcatctgg ctcattgatc cggtgtatgc cgcagcaggc atgagcagcc 5040
   cgaatatgcg cctgctggct gcaacgaccg aggaaatgac ccgcgttttc ggcgctgacc 5100
   aggctttttc acataggctg agccgtggcc actgcactct ccgacgatcc cagccgtacc 5160
   gctggcatgc ccagcacaat cgcgtggatc gcctagctga tcttatggag gttgctcgca 5220
   tgatctcagg cacagaaaaa cctaaaaaac gctatgagca ggagttttct agcggacggg 5280
   cacgtatcga agcggcaaga aaagccactg cggaagcaaa agcacttgcc acgcttgaag 5340
   caagcctgcc gagcgccgct gaagcgtctg gagagctgat cgacggcgtc cgtgtcctct 5400
   ggactgctcc agggcgtgcc gcccgtgatg agacggcttt tcgccacgct ttgactgtgg 5460
   gataccagtt aaaagcggct ggtgagcgcc taaaagacac caagggtcat cgagcctacg 5520
   agcgtgccta caccgtcgct caggcggtcg gaggaggccg tgagcctgat ctgccgccgg 5580
   actgtgaccg ccagacggat tggccgcgac gtgtgcgcgg ctacgtcgct aaaggccagc 5640
   cagtcgtccc tgctcgtcag acagagacgc agagccagcc gaggcgaaaa gctctggcca 5700
   ctatgggaag acgtggcggt aaaaaggccg cagaacgctg gaaagaccca aacagtgagt 5760
   acgcccgagc acagcgagaa aaactagcta agtccagtca acgacaagct aggaaagcta 5820
   aaggaaatcg cttgaccatt gcaggttggt ttatgactgt tgagggagag actggctcgt 5880
   ggccgacaat caatgaagct atgtctgaat ttagcgtgtc acgtcagacc gtgaatagag 5940
   cacttaaggt ctgcgggcat tgaacttcca cgaggacgcc gaaagcttcc cagtaaatgt 6000
   gccatctcgt aggcagaaaa cggttccccc gtagggtctc tctcttggcc tcctttctag 6060
   gtcgggctga ttgctcttga agctctctag gggggctcac accataggca gataacgttc 6120
   cccaccggct cgcctcgtaa gcgcacaagg actgctccca aagatcttca aagccactgc 6180
   cgcgactgcc ttcgcgaagc cttgccccgc ggaaatttcc tccaccgagt tcgtgcacac 6240
   ccctatgcca agcttctttc accctaaatt cgagagattg gattcttacc gtggaaattc 6300
   ttcgcaaaaa tcgtcccctg atcgcccttg cgacgttggc gtcggtgccg ctggttgcgc 6360
   ttggcttgac cgacttgatc agcggccgc 6389
<210> 19
   <211> 1005
   <212> DNA
   <213> Corynebacterium glutamicum
<220>
   <221> CDS
   <222> (1)..(1005)
   <223>
<400> 19
   atg aac cta aag aac ccc gaa acg cca gac cgt aac ctt gct atg gag      48
   Met Asn Leu Lys Asn Pro Glu Thr Pro Asp Arg Asn Leu Ala Met Glu
   1               5                   10                  15
   ctg gtg cga gtt acg gaa gca gct gca ctg gct tct gga cgt tgg gtt      96
   Leu Val Arg Val Thr Glu Ala Ala Ala Leu Ala Ser Gly Arg Trp Val
               20                  25                  30
   gga cgt ggc atg aag aat gaa ggc gac ggt gcc gct gtt gac gcc atg     144
   Gly Arg Gly Met Lys Asn Glu Gly Asp Gly Ala Ala Val Asp Ala Met
           35                  40                  45
   cgc cag ctc atc aac tca gtg acc atg aag ggc gtc gtt gtt atc ggc     192
   Arg Gln Leu Ile Asn Ser Val Thr Met Lys Gly Val Val Val Ile Gly
       50                  55                  60
   gag ggc gaa aaa gac gaa gct cca atg ctg tac aac ggc gaa gag gtc     240
   Glu Gly Glu Lys Asp Glu Ala Pro Met Leu Tyr Asn Gly Glu Glu Val
   65                  70                  75                  80
   gga acc ggc ttt gga cct gag gtt gat atc gca gtt gac cca gtt gac     288
   Gly Thr Gly Phe Gly Pro Glu Val Asp Ile Ala Val Asp Pro Val Asp
                   85                  90                  95
   ggc acc acc ctg atg gct gag ggt cgc ccc aac gca att tcc att ctc     336
   Gly Thr Thr Leu Met Ala Glu Gly Arg Pro Asn Ala Ile Ser Ile Leu
               100                 105                 110
   gca gct gca gag cgt ggc acc atg tac gat cca tcc tcc gtc ttc tac     384
   Ala Ala Ala Glu Arg Gly Thr Met Tyr Asp Pro Ser Ser Val Phe Tyr
           115                 120                 125
   atg aag aag atc gcc gtg gga cct gag gcc gca ggc aag atc gac atc     432
   Met Lys Lys Ile Ala Val Gly Pro Glu Ala Ala Gly Lys Ile Asp Ile
       130                 135                 140
   gaa gct cca gtt gcc cac aac atc aac gcg gtg gca aag tcc aag gga     480
   Glu Ala Pro Val Ala His Asn Ile Asn Ala Val Ala Lys Ser Lys Gly
   145                 150                 155                 160
   atc aac cct tcc gac gtc acc gtt gtc gtg ctt gac cgt cct cgc cac     528
   Ile Asn Pro Ser Asp Val Thr Val Val Val Leu Asp Arg Pro Arg His
                   165                 170                 175
   atc gaa ctg atc gca gac att cgt cgt gca ggc gca aag gtt cgt ctc     576
   Ile Glu Leu Ile Ala Asp Ile Arg Arg Ala Gly Ala Lys Val Arg Leu
               180                 185                 190
   atc tcc gac ggc gac gtt gca ggt gca gtt gca gca gct cag gat tcc     624
   Ile Ser Asp Gly Asp Val Ala Gly Ala Val Ala Ala Ala Gln Asp Ser
           195                 200                 205
   aac tcc gtg gac atc atg atg ggc acc ggc gga acc cca gaa ggc atc      672
   Asn Ser Val Asp Ile Met Met Gly Thr Gly Gly Thr Pro Glu Gly Ile
       210                 215                 220
   atc act gcg tgc gcc atg aag tgc atg ggt ggc gaa atc cag ggc atc      720
   Ile Thr Ala Cys Ala Met Lys Cys Met Gly Gly Glu Ile Gln Gly Ile
   225                 230                 235                 240
   ctg gcc cca atg aac gat ttc gag cgc cag aag gca cac gac gct ggt      768
   Leu Ala Pro Met Asn Asp Phe Glu Arg Gln Lys Ala His Asp Ala Gly
                   245                 250                 255
   ctg gtt ctt gat cag gtt ctg cac acc aac gat ctg gtg agc tcc gac      816
   Leu Val Leu Asp Gln Val Leu His Thr Asn Asp Leu Val Ser Ser Asp
               260                 265                 270
   aac tgc tac ttc gtg gca acc ggt gtg acc aac ggt gac atg ctc cgt      864
   Asn Cys Tyr Phe Val Ala Thr Gly Val Thr Asn Gly Asp Met Leu Arg
           275                 280                 285
   ggc gtt tcc tac cgc gca aac ggc gca acc acc cgt tcc ctg gtt atg      912
   Gly Val Ser Tyr Arg Ala Asn Gly Ala Thr Thr Arg Ser Leu Val Met
       290                 295                 300
   cgc gca aag tca ggc acc atc cgc cac atc gag tct gtc cac cag ctg      960
   Arg Ala Lys Ser Gly Thr Ile Arg His Ile Glu Ser Val His Gln Leu
   305                 310                 315                 320
   tcc aag ctg cag gaa tac tcc gtg gtt gac tac acc acc gcg acc         1005
   Ser Lys Leu Gln Glu Tyr Ser Val Val Asp Tyr Thr Thr Ala Thr
                   325                 330                 335
<210> 20
   <211> 335
   <212> PRT
   <213> Corynebacterium glutamicum
<400> 20

### SEQUENCE LISTING

<110> BASF Aktiengesellschaft
<120> PEF-TS-Expressionseinheiten
<130> 20040509
<160> 20
<170> PatentIn version 3.1
<210> 1
   <211> 178
   <212> DNA
   <213> Corynebacterium glutamicum
<220>
   <221> Promotor
   <222> (1)..(178)
   <223>
<400> 1
   cccccacgac aatggaactt tgacttttaa aatttcatcg ccgtgggggc tttttgggca     60
   gccagcccgc cgtgtcgcaa cgtaatcgac tgaatacctg tacgatcact ttttagacgg    120
   gcgggtaggg ctactgtgcc ctaacctaag cttgtaaagc attaattatc catacata      178
<210> 2
   <211> 195
   <212> DNA
   <213> Corynebacterium glutamicum
<220>
   <221> misc_feature
   <222> (1)..(195)
   <223> Expressionseinheit
<400> 2
   cccccacgac aatggaactt tgacttttaa aatttcatcg ccgtgggggc tttttgggca     60
   gccagcccgc cgtgtcgcaa cgtaatcgac tgaatacctg tacgatcact ttttagacgg    120
   gcgggtaggg ctactgtgcc ctaacctaag cttgtaaagc attaattatc catacataag    180
   gaggatcgcc ccgta                                                     195
<210> 3
   <211> 1365
   <212> DNA
   <213> Corynebacterium glutamicum
<220>
   <221> CDS
   <222> (1)..(1365)
   <223>
<400> 3
   atg aat gat gag aat att caa agc tcc aac tat cag cca ttc ccg agt      48
   Met Asn Asp Glu Asn Ile Gln Ser Ser Asn Tyr Gln Pro Phe Pro Ser
   1               5                   10                  15
   ttt gac gat tgg aaa cag atc gag gtg tcg ctc tta gat gtc atc gaa      96
   Phe Asp Asp Trp Lys Gln Ile Glu Val Ser Leu Leu Asp Val Ile Glu
               20                  25                  30
   tcc tca cgc cat ttt tct gat ttg aaa gat agc act gat cgt tct gcg     144
   Ser Ser Arg His Phe Ser Asp Leu Lys Asp Ser Thr Asp Arg Ser Ala
           35                  40                  45
   tta gat gct gcg cta gag aga gca aaa aga gct gcc gca gtt gat acc     192
   Leu Asp Ala Ala Leu Glu Arg Ala Lys Arg Ala Ala Ala Val Asp Thr
       50                  55                  60
   aat gcc ata gaa gga atc ttc caa act gat cgc ggt ttt acc cat aca     240
   Asn Ala Ile Glu Gly Ile Phe Gln Thr Asp Arg Gly Phe Thr His Thr
   65                  70                  75                  80
   gtt gca acg cag gta ggg gct tgg gag caa caa atg gcg atg aaa ggc     288
   Val Ala Thr Gln Val Gly Ala Trp Glu Gln Gln Met Ala Met Lys Gly
                   85                  90                  95
   aaa cat gtt aag cct gcg ttt gac gat act cta gaa ggc ttt gag tat     336
   Lys His Val Lys Pro Ala Phe Asp Asp Thr Leu Glu Gly Phe Glu Tyr
               100                 105                 110
   gtt ctc gat gca gta act ggt aga act cca atc tct cag caa tgg att     384
   Val Leu Asp Ala Val Thr Gly Arg Thr Pro Ile Ser Gln Gln Trp Ile
           115                 120                 125
   aga aat ttg cac gcc gtc att ctg cgg agc caa gaa agc cac gag gtt     432
   Arg Asn Leu His Ala Val Ile Leu Arg Ser Gln Glu Ser His Glu Val
       130                 135                 140
   ttt aca gcc gtt gga gtc caa aat cag gcg ctt cag aaa ggc gag tat     480
   Phe Thr Ala Val Gly Val Gln Asn Gln Ala Leu Gln Lys Gly Glu Tyr
   145                 150                 155                 160
   aaa act cag cca aat agt cca cag cgc tca gat gga tct gta cat gca     528
   Lys Thr Gln Pro Asn Ser Pro Gln Arg Ser Asp Gly Ser Val His Ala
                   165                 170                 175
   tac gcc cca gtt gaa gat act cct gct gaa atg gct aga ttt att tca     576
   Tyr Ala Pro Val Glu Asp Thr Pro Ala Glu Met Ala Arg Phe Ile Ser
               180                 185                 190
   gaa ctt gaa tct aag gaa ttc tta gca gcc gag aag gtt att caa gct     624
   Glu Leu Glu Ser Lys Glu Phe Leu Ala Ala Glu Lys Val Ile Gln Ala
           195                 200                 205
   gcc tat gcc cac tat gct ttc gta tgt att cat cct ttt gca gat ggg      672
   Ala Tyr Ala His Tyr Ala Phe Val Cys Ile His Pro Phe Ala Asp Gly
       210                 215                 220
   aat gga cga gtt gca cga gcc ttg gct agt gtt ttt cta tac aaa gat      720
   Asn Gly Arg Val Ala Arg Ala Leu Ala Ser Val Phe Leu Tyr Lys Asp
   225                 230                 235                 240
   cct ggt gtc cct ctc gta atc tac caa gat caa cgc aga gat tac atc      768
   Pro Gly Val Pro Leu Val Ile Tyr Gln Asp Gln Arg Arg Asp Tyr Ile
                   245                 250                 255
   cat gct cta gaa gca gcg gac aag aat aac ccg ctc ctg ctg att aga      816
   His Ala Leu Glu Ala Ala Asp Lys Asn Asn Pro Leu Leu Leu Ile Arg
               260                 265                 270
   ttc ttt gct gaa cga gtg acc gat act att aac tct att atc gtt gat      864
   Phe Phe Ala Glu Arg Val Thr Asp Thr Ile Asn Ser Ile Ile Val Asp
           275                 280                 285
   ctc act acc ccg atc gcg ggt aaa tct ggt tcg gct aag ctt tcg gat      912
   Leu Thr Thr Pro Ile Ala Gly Lys Ser Gly Ser Ala Lys Leu Ser Asp
       290                 295                 300
   gcg cta cgc ccc act cgc gta tta cca gaa tta cat gat gct gca cat      960
   Ala Leu Arg Pro Thr Arg Val Leu Pro Glu Leu His Asp Ala Ala His
   305                 310                 315                 320
   agg ctc caa gaa agt tta ttt aca gaa atc cga tct cga ttg gat gaa     1008
   Arg Leu Gln Glu Ser Leu Phe Thr Glu Ile Arg Ser Arg Leu Asp Glu
                   325                 330                 335
   gaa gga aaa agg aat ggg ttg gag ttt cta ctt caa cgg att ttt atc     1056
   Glu Gly Lys Arg Asn Gly Leu Glu Phe Leu Leu Gln Arg Ile Phe Ile
               340                 345                 350
   ggt tcc cca ttc aat ctg cca gag ggc tat aac gct ttc cct gat agc     1104
   Gly Ser Pro Phe Asn Leu Pro Glu Gly Tyr Asn Ala Phe Pro Asp Ser
           355                 360                 365
   tat tgt ctg acc tta gct ttc aat agc aac tct cca aaa caa atc ttc     1152
   Tyr Cys Leu Thr Leu Ala Phe Asn Ser Asn Ser Pro Lys Gln Ile Phe
       370                 375                 380
   cac ccg cta tcc ata gta ata gca gct cga gat ggg aaa aga gcg agc     1200
   His Pro Leu Ser Ile Val Ile Ala Ala Arg Asp Gly Lys Arg Ala Ser
   385                 390                 395                 400
   agc gac ctc gtg gca gct act tct att gga tac aac ttt cac gct tac     1248
   Ser Asp Leu Val Ala Ala Thr Ser Ile Gly Tyr Asn Phe His Ala Tyr
                   405                 410                 415
   gga cgt gaa gtc gag cct gtt gtt act gaa agc ttt cga gaa cgt gtg     1296
   Gly Arg Glu Val Glu Pro Val Val Thr Glu Ser Phe Arg Glu Arg Val
               420                 425                 430
   aaa att tac gcc gac ggg att gta gat cac ttc tta acc gaa ctg gct     1344
   Lys Ile Tyr Ala Asp Gly Ile Val Asp His Phe Leu Thr Glu Leu Ala
           435                 440                 445
   aaa aag ttt caa cag aat taa                                         1365
   Lys Lys Phe Gln Gln Asn
       450
<210> 4
   <211> 454
   <212> PRT
   <213> Corynebacterium glutamicum
<400> 4
   Met Asn Asp Glu Asn Ile Gln Ser Ser Asn Tyr Gln Pro Phe Pro Ser
   1               5                   10                  15
   Phe Asp Asp Trp Lys Gln Ile Glu Val Ser Leu Leu Asp Val Ile Glu
               20                  25                  30
   Ser Ser Arg His Phe Ser Asp Leu Lys Asp Ser Thr Asp Arg Ser Ala
           35                  40                  45
   Leu Asp Ala Ala Leu Glu Arg Ala Lys Arg Ala Ala Ala Val Asp Thr
       50                  55                  60
   Asn Ala Ile Glu Gly Ile Phe Gln Thr Asp Arg Gly Phe Thr His Thr
   65                  70                  75                  80
   Val Ala Thr Gln Val Gly Ala Trp Glu Gln Gln Met Ala Met Lys Gly
                   85                  90                  95
   Lys His Val Lys Pro Ala Phe Asp Asp Thr Leu Glu Gly Phe Glu Tyr
               100                 105                 110
   Val Leu Asp Ala Val Thr Gly Arg Thr Pro Ile Ser Gln Gln Trp Ile
           115                 120                 125
   Arg Asn Leu His Ala Val Ile Leu Arg Ser Gln Glu Ser His Glu Val
       130                 135                 140
   Phe Thr Ala Val Gly Val Gln Asn Gln Ala Leu Gln Lys Gly Glu Tyr
   145                 150                 155                 160
   Lys Thr Gln Pro Asn Ser Pro Gln Arg Ser Asp Gly Ser Val His Ala
                   165                 170                 175
   Tyr Ala Pro Val Glu Asp Thr Pro Ala Glu Met Ala Arg Phe Ile Ser
               180                 185                 190
   Glu Leu Glu Ser Lys Glu Phe Leu Ala Ala Glu Lys Val Ile Gln Ala
           195                 200                 205
   Ala Tyr Ala His Tyr Ala Phe Val Cys Ile His Pro Phe Ala Asp Gly
       210                 215                 220
   Asn Gly Arg Val Ala Arg Ala Leu Ala Ser Val Phe Leu Tyr Lys Asp
   225                 230                 235                 240
   Pro Gly Val Pro Leu Val Ile Tyr Gln Asp Gln Arg Arg Asp Tyr Ile
                   245                 250                 255
   His Ala Leu Glu Ala Ala Asp Lys Asn Asn Pro Leu Leu Leu Ile Arg
               260                 265                 270
   Phe Phe Ala Glu Arg Val Thr Asp Thr Ile Asn Ser Ile Ile Val Asp
           275                 280                 285
   Leu Thr Thr Pro Ile Ala Gly Lys Ser Gly Ser Ala Lys Leu Ser Asp
   290 295 300
   Ala Leu Arg Pro Thr Arg Val Leu Pro Glu Leu His Asp Ala Ala His
   305 310 315 320
   Arg Leu Gln Glu Ser Leu Phe Thr Glu Ile Arg Ser Arg Leu Asp Glu
   325 330 335
   Glu Gly Lys Arg Asn Gly Leu Glu Phe Leu Leu Gln Arg Ile Phe Ile
   340 345 350
   Gly Ser Pro Phe Asn Leu Pro Glu Gly Tyr Asn Ala Phe Pro Asp Ser
   355 360 365
   Tyr Cys Leu Thr Leu Ala Phe Asn Ser Asn Ser Pro Lys Gln Ile Phe
   370 375 380
   His Pro Leu Ser Ile Val Ile Ala Ala Arg Asp Gly Lys Arg Ala Ser
   385 390 395 400
   Ser Asp Leu Val Ala Ala Thr Ser Ile Gly Tyr Asn Phe His Ala Tyr
   405 410 415
   Gly Arg Glu Val Glu Pro Val Val Thr Glu Ser Phe Arg Glu Arg Val
   420 425 430
   Lys Ile Tyr Ala Asp Gly Ile Val Asp His Phe Leu Thr Glu Leu Ala
   435 440 445
   Lys Lys Phe Gln Gln Asn
   450
<210> 5
   <211> 52
   <212> DNA
   <213> Künstliche Sequenz
<220>
   <221> Primer
   <222> (1)..(52)
   <223> Primer
<400> 5
   cccgggatcc gctagcggcg cgccggccgg cccggtgtga aataccgcac ag 52
<210> 6
   <211> 53
   <212> DNA
   <213> Künstliche Sequenz
<220>
   <221> Primer
   <222> (1)..(53)
   <223> Primer
<400> 6
   tctagactcg agcggccgcg gccggccttt aaattgaaga cgaaagggcc tcg 53
<210> 7
   <211> 47
   <212> DNA
   <213> Künstliche Sequenz
<220>
   <221> Primer
   <222> (1)..(47)
   <223> Primer
<400> 7
   gagatctaga cccggggatc cgctagcggg ctgctaaagg aagcgga 47
<210> 8
   <211> 38
   <212> DNA
   <213> Künstliche sequenz
<220>
   <221> Primer
   <222> (1)..(38)
   <223> Primer
<400> 8
   gagaggcgcg ccgctagcgt gggcgaagaa ctccagca 38
<210> 9
   <211> 34
   <212> DNA
   <213> Künstliche Sequenz
<220>
   <221> Primer
   <222> (1)..(34)
   <223> Primer
<400> 9
   gagagggcgg ccgcgcaaag tcccgcttcg tgaa 34
<210> 10
   <211> 34
   <212> DNA
   <213> Künstliche Sequenz
<220>
   <221> Primer
   <222> (1)..(34)
   <223> Primer
<400> 10
   gagagggcgg ccgctcaagt cggtcaagcc acgc 34
<210> 11
   <211> 140
   <212> DNA
   <213> Künstliche sequenz
<220>
   <221> Primer
   <222> (1)..(140)
   <223> Primer
<400> 11
   tcgaatttaa atctcgagag gcctgacgtc gggcccggta ccacgcgtca tatgactagt 60
   tcggacctag ggatatcgtc gacatcgatg ctcttctgcg ttaattaaca attgggatcc 120
   tctagacccg ggatttaaat 140
<210> 12
   <211> 140
   <212> DNA
   <213> Künstliche sequenz
<220>
   <221> Primer
   <222> (1)..(140)
   <223> Primer
<400> 12
   gatcatttaa atcccgggtc tagaggatcc caattgttaa ttaacgcaga agagcatcga 60
   tgtcgacgat atccctaggt ccgaactagt catatgacgc gtggtaccgg gcccgacgtc 120
   aggcctctcg agatttaaat 140
<210> 13
   <211> 5091
   <212> DNA
   <213> Künstliche Sequenz
<220>
   <221> misc_feature
   <222> (15091)..()
   <223> Plasmid
<400> 13
   tcgatttaaa tctcgagagg cctgacgtcg ggcccggtac cacgcgtcat atgactagtt     60
   cggacctagg gatatcgtcg acatcgatgc tcttctgcgt taattaacaa ttgggatcct    120
   ctagacccgg gatttaaatc gctagcgggc tgctaaagga agcggaacac gtagaaagcc    180
   agtccgcaga aacggtgctg accccggatg aatgtcagct actgggctat ctggacaagg    240
   gaaaacgcaa gcgcaaagag aaagcaggta gcttgcagtg ggcttacatg gcgatagcta    300
   gactgggcgg ttttatggac agcaagcgaa ccggaattgc cagctggggc gccctctggt    360
   aaggttggga agccctgcaa agtaaactgg atggctttct tgccgccaag gatctgatgg    420
   cgcaggggat caagatctga tcaagagaca ggatgaggat cgtttcgcat gattgaacaa    480
   gatggattgc acgcaggttc tccggccgct tgggtggaga ggctattcgg ctatgactgg    540
   gcacaacaga caatcggctg ctctgatgcc gccgtgttcc ggctgtcagc gcaggggcgc    600
   ccggttcttt ttgtcaagac cgacctgtcc ggtgccctga atgaactgca ggacgaggca    660
   gcgcggctat cgtggctggc cacgacgggc gttccttgcg cagctgtgct cgacgttgtc    720
   actgaagcgg gaagggactg gctgctattg ggcgaagtgc cggggcagga tctcctgtca    780
   tctcaccttg ctcctgccga gaaagtatcc atcatggctg atgcaatgcg gcggctgcat    840
   acgcttgatc cggctacctg cccattcgac caccaagcga aacatcgcat cgagcgagca    900
   cgtactcgga tggaagccgg tcttgtcgat caggatgatc tggacgaaga gcatcagggg    960
   ctcgcgccag ccgaactgtt cgccaggctc aaggcgcgca tgcccgacgg cgaggatctc   1020
   gtcgtgaccc atggcgatgc ctgcttgccg aatatcatgg tggaaaatgg ccgcttttct   1080
   ggattcatcg actgtggccg gctgggtgtg gcggaccgct atcaggacat agcgttggct   1140
   acccgtgata ttgctgaaga gcttggcggc gaatgggctg accgcttcct cgtgctttac   1200
   ggtatcgccg ctcccgattc gcagcgcatc gccttctatc gccttcttga cgagttcttc 1260
   tgagcgggac tctggggttc gaaatgaccg accaagcgac gcccaacctg ccatcacgag 1320
   atttcgattc caccgccgcc ttctatgaaa ggttgggctt cggaatcgtt ttccgggacg 1380
   ccggctggat gatcctccag cgcggggatc tcatgctgga gttcttcgcc cacgctagcg 1440
   gcgcgccggc cggcccggtg tgaaataccg cacagatgcg taaggagaaa ataccgcatc 1500
   aggcgctctt ccgcttcctc gctcactgac tcgctgcgct cggtcgttcg gctgcggcga 1560
   gcggtatcag ctcactcaaa ggcggtaata cggttatcca cagaatcagg ggataacgca 1620
   ggaaagaaca tgtgagcaaa aggccagcaa aaggccagga accgtaaaaa ggccgcgttg 1680
   ctggcgtttt tccataggct ccgcccccct gacgagcatc acaaaaatcg acgctcaagt 1740
   cagaggtggc gaaacccgac aggactataa agataccagg cgtttccccc tggaagctcc 1800
   ctcgtgcgct ctcctgttcc gaccctgccg cttaccggat acctgtccgc ctttctccct 1860
   tcgggaagcg tggcgctttc tcatagctca cgctgtaggt atctcagttc ggtgtaggtc 1920
   gttcgctcca agctgggctg tgtgcacgaa ccccccgttc agcccgaccg ctgcgcctta 1980
   tccggtaact atcgtcttga gtccaacccg gtaagacacg acttatcgcc actggcagca 2040
   gccactggta acaggattag cagagcgagg tatgtaggcg gtgctacaga gttcttgaag 2100
   tggtggccta actacggcta cactagaagg acagtatttg gtatctgcgc tctgctgaag 2160
   ccagttacct tcggaaaaag agttggtagc tcttgatccg gcaaacaaac caccgctggt 2220
   agcggtggtt tttttgtttg caagcagcag attacgcgca gaaaaaaagg atctcaagaa 2280
   gatcctttga tcttttctac ggggtctgac gctcagtgga acgaaaactc acgttaaggg 2340
   attttggtca tgagattatc aaaaaggatc ttcacctaga tccttttaaa ggccggccgc 2400
   ggccgcgcaa agtcccgctt cgtgaaaatt ttcgtgccgc gtgattttcc gccaaaaact 2460
   ttaacgaacg ttcgttataa tggtgtcatg accttcacga cgaagtacta aaattggccc 2520
   gaatcatcag ctatggatct ctctgatgtc gcgctggagt ccgacgcgct cgatgctgcc 2580
   gtcgatttaa aaacggtgat cggatttttc cgagctctcg atacgacgga cgcgccagca 2640
   tcacgagact gggccagtgc cgcgagcgac ctagaaactc tcgtggcgga tcttgaggag 2700
   ctggctgacg agctgcgtgc tcggccagcg ccaggaggac gcacagtagt ggaggatgca 2760
   atcagttgcg cctactgcgg tggcctgatt cctccccggc ctgacccgcg aggacggcgc 2820
   gcaaaatatt gctcagatgc gtgtcgtgcc gcagccagcc gcgagcgcgc caacaaacgc 2880
   cacgccgagg agctggaggc ggctaggtcg caaatggcgc tggaagtgcg tcccccgagc 2940
   gaaattttgg ccatggtcgt cacagagctg gaagcggcag cgagaattat cgcgatcgtg 3000
   gcggtgcccg caggcatgac aaacatcgta aatgccgcgt ttcgtgtgcc gtggccgccc 3060
   aggacgtgtc agcgccgcca ccacctgcac cgaatcggca gcagcgtcgc gcgtcgaaaa 3120
   agcgcacagg cggcaagaag cgataagctg cacgaatacc tgaaaaatgt tgaacgcccc 3180
   gtgagcggta actcacaggg cgtcggctaa cccccagtcc aaacctggga gaaagcgctc 3240
   aaaaatgact ctagcggatt cacgagacat tgacacaccg gcctggaaat tttccgctga 3300
   tctgttcgac acccatcccg agctcgcgct gcgatcacgt ggctggacga gcgaagaccg 3360
   ccgcgaattc ctcgctcacc tgggcagaga aaatttccag ggcagcaaga cccgcgactt 3420
   cgccagcgct tggatcaaag acccggacac ggagaaacac agccgaagtt ataccgagtt 3480
   ggttcaaaat cgcttgcccg gtgccagtat gttgctctga cgcacgcgca gcacgcagcc   3540
   gtgcttgtcc tggacattga tgtgccgagc caccaggccg gcgggaaaat cgagcacgta   3600
   aaccccgagg tctacgcgat tttggagcgc tgggcacgcc tggaaaaagc gccagcttgg   3660
   atcggcgtga atccactgag cgggaaatgc cagctcatct ggctcattga tccggtgtat   3720
   gccgcagcag gcatgagcag cccgaatatg cgcctgctgg ctgcaacgac cgaggaaatg   3780
   acccgcgttt tcggcgctga ccaggctttt tcacataggc tgagccgtgg ccactgcact   3840
   ctccgacgat cccagccgta ccgctggcat gcccagcaca atcgcgtgga tcgcctagct   3900
   gatcttatgg aggttgctcg catgatctca ggcacagaaa aacctaaaaa acgctatgag   3960
   caggagtttt ctagcggacg ggcacgtatc gaagcggcaa gaaaagccac tgcggaagca   4020
   aaagcacttg ccacgcttga agcaagcctg ccgagcgccg ctgaagcgtc tggagagctg   4080
   atcgacggcg tccgtgtcct ctggactgct ccagggcgtg ccgcccgtga tgagacggct   4140
   tttcgccacg ctttgactgt gggataccag ttaaaagcgg ctggtgagcg cctaaaagac   4200
   accaagggtc atcgagccta cgagcgtgcc tacaccgtcg ctcaggcggt cggaggaggc   4260
   cgtgagcctg atctgccgcc ggactgtgac cgccagacgg attggccgcg acgtgtgcgc   4320
   ggctacgtcg ctaaaggcca gccagtcgtc cctgctcgtc agacagagac gcagagccag   4380
   ccgaggcgaa aagctctggc cactatggga agacgtggcg gtaaaaaggc cgcagaacgc   4440
   tggaaagacc caaacagtga gtacgcccga gcacagcgag aaaaactagc taagtccagt   4500
   caacgacaag ctaggaaagc taaaggaaat cgcttgacca ttgcaggttg gtttatgact   4560
   gttgagggag agactggctc gtggccgaca atcaatgaag ctatgtctga atttagcgtg   4620
   tcacgtcaga ccgtgaatag agcacttaag gtctgcgggc attgaacttc cacgaggacg   4680
   ccgaaagctt cccagtaaat gtgccatctc gtaggcagaa aacggttccc ccgtagggtc   4740
   tctctcttgg cctcctttct aggtcgggct gattgctctt gaagctctct aggggggctc   4800
   acaccatagg cagataacgt tccccaccgg ctcgcctcgt aagcgcacaa ggactgctcc   4860
   caaagatctt caaagccact gccgcgactg ccttcgcgaa gccttgcccc gcggaaattt   4920
   cctccaccga gttcgtgcac acccctatgc caagcttctt tcaccctaaa ttcgagagat   4980
   tggattctta ccgtggaaat tcttcgcaaa aatcgtcccc tgatcgccct tgcgacgttg   5040
   gcgtcggtgc cgctggttgc gcttggcttg accgacttga tcagcggccg c              5091
<210> 14
   <211> 32
   <212> DNA
   <213> Künstliche sequenz
<220>
   <221> Primer
   <222> (1)..(32)
   <223> Primer
<400> 14
   gtgtgtcgac ttagatgtag aactcgatgt ag 32
<210> 15
   <211> 17
   <212> DNA
   <213> Künstliche Sequenz
<220>
   <221> Primer
   <222> (1)..(17)
   <223> Primer
<400> 15
   atgcccaccc tcgcgcc 17
<210> 16
   <211> 28
   <212> DNA
   <213> Künstliche Sequenz
<220>
   <221> Primer
   <222> (1)..(28)
   <223> Primer
<400> 16
   gagaggatcc cccccacgac aatggaac 28
<210> 17
   <211> 44
   <212> DNA
   <213> Künstliche Sequenz
<220>
   <221> Primer
   <222> (1)..(44)
   <223> Primer
<400> 17
   cctgaaggcg cgagggtggg cattacgggg cgatcctcct tatg 44
<210> 18
   <211> 6389
   <212> DNA
   <213> Künstliche sequenz
<220>
   <221> misc_feature
   <222> (1)..(6389)
   <223> Plasmid
<400> 18 7
<210> 19
   <211> 1005
   <212> DNA
   <213> corynebacterium glutamicum
<220>
   <221> CDS
   <222> (1)..(1005)
   <223>
<400> 19
   atg aac cta aag aac ccc gaa acg cca gac cgt aac ctt gct atg gag      48
   Met Asn Leu Lys Asn Pro Glu Thr Pro Asp Arg Asn Leu Ala Met Glu
   1               5                   10                  15
   ctg gtg cga gtt acg gaa gca gct gca ctg gct tct gga cgt tgg gtt      96
   Leu Val Arg Val Thr Glu Ala Ala Ala Leu Ala Ser Gly Arg Trp Val
               20                  25                  30
   gga cgt ggc atg aag aat gaa ggc gac ggt gcc gct gtt gac gcc atg     144
   Gly Arg Gly Met Lys Asn Glu Gly Asp Gly Ala Ala Val Asp Ala Met
           35                  40                  45
   cgc cag ctc atc aac tca gtg acc atg aag ggc gtc gtt gtt atc ggc     192
   Arg Gln Leu Ile Asn Ser Val Thr Met Lys Gly Val Val Val Ile Gly
       50                  55                  60
   gag ggc gaa aaa gac gaa gct cca atg ctg tac aac ggc gaa gag gtc     240
   Glu Gly Glu Lys Asp Glu Ala Pro Met Leu Tyr Asn Gly Glu Glu Val
   65                  70                  75                  80
   gga acc ggc ttt gga cct gag gtt gat atc gca gtt gac cca gtt gac     288
   Gly Thr Gly Phe Gly Pro Glu Val Asp Ile Ala Val Asp Pro Val Asp
                   85                  90                  95
   ggc acc acc ctg atg gct gag ggt cgc ccc aac gca att tcc att ctc     336
   Gly Thr Thr Leu Met Ala Glu Gly Arg Pro Asn Ala Ile Ser Ile Leu
               100                 105                 110
   gca gct gca gag cgt ggc acc atg tac gat cca tcc tcc gtc ttc tac     384
   Ala Ala Ala Glu Arg Gly Thr Met Tyr Asp Pro Ser Ser Val Phe Tyr
           115                 120                 125
   atg aag aag atc gcc gtg gga cct gag gcc gca ggc aag atc gac atc     432
   Met Lys Lys Ile Ala Val Gly Pro Glu Ala Ala Gly Lys Ile Asp Ile
       130                 135                 140
   gaa gct cca gtt gcc cac aac atc aac gcg gtg gca aag tcc aag gga     480
   Glu Ala Pro Val Ala His Asn Ile Asn Ala Val Ala Lys Ser Lys Gly
   145                 150                 155                 160
   atc aac cct tcc gac gtc acc gtt gtc gtg ctt gac cgt cct cgc cac     528
   Ile Asn Pro Ser Asp Val Thr Val Val Val Leu Asp Arg Pro Arg His
                   165                 170                 175
   atc gaa ctg atc gca gac att cgt cgt gca ggc gca aag gtt cgt ctc        576
   Ile Glu Leu Ile Ala Asp Ile Arg Arg Ala Gly Ala Lys Val Arg Leu
               180                 185                 190
   atc tcc gac ggc gac gtt gca ggt gca gtt gca gca gct cag gat tcc        624
   Ile Ser Asp Gly Asp Val Ala Gly Ala Val Ala Ala Ala Gln Asp Ser
           195                 200                 205
   aac tcc gtg gac atc atg atg ggc acc ggc gga acc cca gaa ggc atc        672
   Asn Ser Val Asp Ile Met Met Gly Thr Gly Gly Thr Pro Glu Gly Ile
       210                 215                 220
   atc act gcg tgc gcc atg aag tgc atg ggt ggc gaa atc cag ggc atc        720
   Ile Thr Ala Cys Ala Met Lys Cys Met Gly Gly Glu Ile Gln Gly Ile
   225                 230                 235                 240
   ctg gcc cca atg aac gat ttc gag cgc cag aag gca cac gac gct ggt        768
   Leu Ala Pro Met Asn Asp Phe Glu Arg Gln Lys Ala His Asp Ala Gly
                   245                 250                 255
   ctg gtt ctt gat cag gtt ctg cac acc aac gat ctg gtg agc tcc gac        816
   Leu Val Leu Asp Gln Val Leu His Thr Asn Asp Leu Val Ser Ser Asp
               260                 265                 270
   aac tgc tac ttc gtg gca acc ggt gtg acc aac ggt gac atg ctc cgt        864
   Asn Cys Tyr Phe Val Ala Thr Gly Val Thr Asn Gly Asp Met Leu Arg
           275                 280                 285
   ggc gtt tcc tac cgc gca aac ggc gca acc acc cgt tcc ctg gtt atg        912
   Gly Val Ser Tyr Arg Ala Asn Gly Ala Thr Thr Arg Ser Leu Val Met
       290                 295                 300
   cgc gca aag tca ggc acc atc cgc cac atc gag tct gtc cac cag ctg        960
   Arg Ala Lys Ser Gly Thr Ile Arg His Ile Glu Ser Val His Gln Leu
   305                 310                 315                 320
   tcc aag ctg cag gaa tac tcc gtg gtt gac tac acc acc gcg acc           1005
   Ser Lys Leu Gln Glu Tyr Ser Val Val Asp Tyr Thr Thr Ala Thr
                   325                 330                 335
<210> 20
   <211> 335
   <212> PRT
   <213> Corynebacterium glutamicum
<400> 20